# EUROPEAN PATENT APPLICATION

(11) **EP 4 282 448 A1**
(43) Date of publication of application: **29.11.2023**
(21) Application number: 22174810.6
(22) Date of filing: 23.05.2022
(51) Int. Cl.: A61L 27/54, A61B 5/06

(54) **POROUS EXPANDING BIOCOMPATIBLE SCAFFOLDS**

(71) Applicant: Danmarks Tekniske Universitet, 2800 Kongens Lyngby (DK)
(72) Inventor: Andresen, Thomas Lars, 2800 Kongens Lyngby (DK); Henriksen, Jonas Rosager, 2800 Kongens Lyngby (DK); Hansen, Anders Elias, 2800 Kongens Lyngby (DK); Bruun, Linda Maria, 2800 Kongens Lyngby (DK); Fuglsang-Madsen, Albert Juan, 2800 Kongens Lyngby (DK)
(74) Representative: AWA Sweden AB

(57) **Abstract**

The present invention provides a composition comprising:
- at least one gel-forming hydrophobic carbohydrate ester or an analogue thereof based on mono-, di-, and tri-saccharides, or mixtures thereof;
- at least one polymer; and
- at least one solvent being mixable with water;
wherein the composition is a ECell being a hydrophobic gel-depot having the ability to take up water by means of said at least one solvent being mixable with water and having the ability to self-expand by formation of water pores, channels, and/or cavities.

## Description

### Field of the invention

The present invention relates to an ECell composition.

### Technical Background

Carbohydrate ester depots also referred to as ECells, are composed of carbohydrate ester scaffolds, organic co-solvents, and solvents, which are designed for embedding and retaining hydrophobic substances such as contrast agents, colours, or fluorophores and to provide sustained release of hydrophobic drug molecules or ion-paired hydrophilic, hydrophobic, or amphipathic drug molecules. The ECell formulation is a liquid, and upon injection into aqueous media or tissues, the solvent of the ECell, which is mixable with water, diffuses out of the carbohydrate ester co-solvent mixture. In this process the ECell is formed as a hydrophobic depot. Standard compositions of ECell repel water and form dense water depleted depots, which is apparent by the signal void observed by MR imaging. ECells are primarily used as fiducial markers in radiotherapy, surgical procedures, and as sustained release drug depots.

However, there are many challenges that must still be overcome for widely deploying ECells for the above mentioned uses since they are intrinsically hydrophobic and have high ECell-water interfacial tension, which prevent formation of interfacial surface such as water channels and internal voids. This leads to non-expanding ECells that have limited application as tissue scaffolds, void fillers, and surgical markers with positive MRI visibility.

### Summary of the invention

There is an object of the present invention to provide biomaterial compositions for use as fiducial markers, drug eluding depots or biomimetic scaffolds that have self-expanding properties and form porous water channel networks. Further, methods for changing the surface charge, polymer grafting, adding cell signalling molecules for optimized tissue interaction of the biomaterial is presented herein. These altered material properties are reflected in changed drug release kinetics, enhanced MR image contrast, and changed wetting behaviour.

In a first aspect the present invention relates to a composition comprising:
- at least one gel-forming hydrophobic carbohydrate ester or an analogue thereof based on mono-, di-, and tri-saccharides, or mixtures thereof;
- at least one polymer; and
- at least one solvent being mixable with water;
wherein the composition is a ECell being a hydrophobic gel-depot having the ability to take up water by means of said at least one solvent being mixable with water and having the ability to self-expand by formation of water pores, channels, and/or cavities.

In relation to the above it should be noted that "gel-forming" covers such becoming semi-crystalline, crystalline or amorphous structures.

Furthermore, said at least polymer preferably is hydrophobic or amphipathic and thereby compatible with the ECell according to the present invention.

It should be noted that in the current invention, special compositions are described for Ecells that surprisingly expand upon contact with water or tissue. These ECell compositions further allow for modifying the ECell surface by use of lipids and polymers. Overall, these new ECell compositions provide a range of exciting new applications including use as tissue scaffolds, surgical void filler and fiducial marker with intrinsic positive MRI contrast.

### Short description of figures

Figure 1 shows the impact of varying the type of carbohydrate ester on the expansion property of expanding ECells.
Figure 2 shows the impact of varying the triglyceride co-solvent content on the expansion property of expanding ECells.
Figure 3 shows the impact of solvent type on the expansion property of expanding ECells, at 25 °C.
Figure 4 shows the impact of CAB content on the expansion property of expanding ECells at 25 °C.
Figure 5 shows the impact of CAB content on the expansion property of expanding ECells at 37 °C.
Figure 6 shows the impact of temperature on the expansion property of expanding ECells; 25 vs. 37 °C.
Figure 7 shows the effect of CAB length variation on the expansion property of expanding ECells at 37 °C.
Figure 8 shows the effect of CAB and GTO variation on the *in vivo* expansion property of expanding ECells.
Figure 9 shows the effect of LOP- or LOIB-based expanding ECells on its *in vivo* expansion properties.
Figure 10 shows cryo-SEM imaging of an expanding ECells formulation (1): 55:10:35; LOIB, CLA8, DMSO, collected from a mouse after 7 days of *in vivo* expansion and snap-frozen.
Figure 11 shows cryo-SEM imaging of an expanding ECells formulation (2): LOIB:CLA8:CAB(12 kDa) :DMSO (50:10:5:35), collected from a mouse after 7 days of *in vivo* expansion which was snap-frozen before imaging.
Figure 12 shows cryo-SEM imaging of an expanding ECells formulation (3): LOIB, CLA8, CAB(12 kDa):DMSO (47.5:10:7.5:35), collected from a mouse after 7 days of *in vivo* expansion which was snap-frozen before imaging.
Figure 13 shows cryo-SEM imaging of an expanding ECells formulation (4): LOIB:CLA8:CAB(12 kDa):GTO:DMSO (47.5:10:5:2.5:35), collected from a mouse after 7 days of *in vivo* expansion which was snap-frozen before imaging.
Figure 14 shows cryo-SEM imaging of an expanding ECells formulation (2): LOP:CLA8:CAB(12 kDa):DMSO (50:10:5:35), collected from a mouse after 7 days of *in vivo* expansion which was snap-frozen before imaging.
Figures 15a and 15b show *in vitro*-expanded ECells of LOIB:CAB(12 kDa):DMSO (65:5:30) with 0.025% w/w curcumin, imaged by spinning disc confocal microscopy, wherein Figure 15a shows the surface and Figure 15b the cross-section.
Figures 16a and 16b show *in vitro*-expanded ECells of LOIB:CAB(12 kDa):DMSO (65:5:30) with 0.025% curcumin, incubated for 5 days with osteoblast-differentiated C2C12 cells, imaged by spinning disc confocal microscopy.
Figure 17 shows a cross-section of *in vitro*-expanded ECell of LOIB:CAB(12 kDa):DMSO, 65:5:30, with 0.025 % w/w curcumin and incubated for 5 days with osteoblast-differentiated C2C12 cells, imaged by spinning disc confocal microscopy.
Figure 18 shows *in vitro* release of antimicrobial compounds from expanding ECells over time (in days).
Figure 19a shows the *in vitro* release of Batimastat, LMT-28 and SB-3CT from an expanding ECell formulation at 37 °C, in dPBS and Figure 19b shows the expanding of each expanding ECells over time during the course of the study.
Figure 20 shows dissolution of 5 % w/w CAB in a LOIB:DMSO ECell (vial labeled Exp001) and an example of an insoluble excipient in a ECell formulation (vial labeled Exp020).
Figure 21 shows images of ECells compositions based on different types of solvent.
Figure 22 shows images of expanding ECells comprising different types of solvent.
Figure 23 shows images of homogeneous ECell compositions comprising 2.5%w/w of various polymers, such as PPG, VitE-PEG, PCL-PEG-PCL, PCL Diol 2 kDa, PLGA Ester, PLGA-COOH short, PLGA-COOH medium, PLA Ester medium, PLA Ether medium, PEG 1kDa, PEG 1.5kDa and PEG-C18 2kDa, CAB 12 kDa, CAB 30 kDa, CAB 70 kDa, CP 70 kDa, CAP 75 kDa, bPEI 600 Da, and bPEI 1.2 kDa..
Figure 24 shows images displaying expanding ECells comprising different polymers, such as PPG, VitE-PEG, PCL-PEG-PCL, PCL Diol 2 kDa, PLGA Ester, PLGA-COOH short, PLGA-COOH medium, PLA Ester medium, and PLA Ether medium. Expansion was conducted at 37°C and images were recorded 1.5h, 2 days and 7 days post injection (pi).
Figure 25 shows images displaying expanding ECells comprising different polymers, such as PEG 1kDa, PEG 1.5kDa and PEG-C18 2kDa, CAB 12 kDa, CAB 30 kDa, CAB 70 kDa, CP 70 kDa, CAP 75 kDa, bPEI 600 Da, and bPEI 1.2 kDa. Expansion was conducted at 37°C and images were recorded 1.5h, 2 days and 7 days post injection (pi).
Figure 26 shows solubility of lipids/surfactant in ECell compositions. Transparent and homogeneous LOIB:CAB:DMSO:Surfactant 66.5:2.5:30:1 (left vials labelled A in the figure), and LOIB:GTH:DMSO:Surfactant 69:10:20:1 (right vials labelled B in the figure) ECell fomulations comprising varying surfactants.
Figure 27 shows expanding ECell compositions comprising lipids/surfactants. Expansion of LOIB:CAB:DMSO:Surfactant 66.5:2.5:30:1 (left vials labelled A.1 in the figure), and LOIB:GTH:DMSO:Surfactant 69:10:20:1 (right vials labelled B.1 in the figure) ECell fomulations, comprising varying surfactants, as a function of time (1.5 hours, 2 days and 7 days). The Reference composition was LOIB:CAB:DMSO 67.5:2.5:30 (left vial), and LOIB:GTH:DMSO 70:10:20 (right vial).
Figure 28 shows expansion of ECell compositions comprising lipids/surfactants. Expansion of LOIB:CAB:DMSO:Surfactant 66.5:2.5:30:1 (left vials labelled A.1 in the figure), and LOIB:GTH:DMSO:Surfactant 69:10:20:1 (right vials labelled B.1 in the figure) ECell fomulations comprising varying surfactants as a function of time (1.5 hours, 2 days and 7 days). The Reference composition was LOIB:CAB:DMSO 67.5:2.5:30 (left vial), and LOIB:GTH:DMSO 70:10:20 (right vial).
Figure 29 shows MRI and CT of ECell injected subcutaneously in mice. Left column; T2 weighted MRI images (axial slices) acquired 14 days after injection of ECell compositions LOIB:CLA-8:DMSO (55:10:35) (top) and LOIB:CLA-8:CAB:DMSO (47.45:10:7.5:35) (bottom). Right image serie; CT scan images (axial slices) of the LOIB:CLA-8:CAB:DMSO (47.45:10:7.5:35) composition at indicated time points after injection.

### Definitions

"Prodrug" refers to drug molecules that have been chemically modified via covalent bonds. Upon activation of the prodrug, one or more covalent bonds are broken, and the native drug is released.
"Drug analogue" refers to a modified version of a drug where new chemical bonds have been created.
"Targeting" of drugs, drug analogues or prodrugs refers to drug molecules that carry a ligand that enhances the molecules affinity for certain tissues or cells.
"Tissue" refers to an ensemble of similar cells and their extracellular matrix from the same origin that together carry out a specific function.
"Aqueous media" refers to water-based solutions, such as e.g. saline or saline solutions.
"Non-water soluble carbohydrates" refers to carbohydrates that are insoluble in water, which is defined as carbohydrates that precipitates when the concentration exceeds 0.1 M at 25 degrees Celsius.

In the context of the present invention, a "gel" is defined as a carrier matrix in which the detectable agent (contrast agent) or active pharmaceutical ingredient is dispersed and/or dissolved within.

The term "gel" as used in the present invention includes systems such as gels or amorphous glass matrices, crystalline solids, amorphous solids, which upon injection into a human or an animal increases viscosity where the composition changes from being liquid like to gel-like in its appearance.

In term "ECell" is used for gel or gel-forming compositions comprising carbohydrate esters that have features of a gel.

With the term "gel-like" or "gel-forming" compound or material, as used herein, we refer to any compound comprising some of the properties of a gel i.e. a material that exhibits limited flow when in the steady-state. By weight, gels are mostly liquid, yet they behave like solids due to a three-dimensional interaction within the liquid. It is the interactions within the fluid that gives a gel its structure (hardness) and contributes to the adhesive stick. In this way gels are a dispersion of molecules of a liquid within a solid in which the solid is the continuous phase and the liquid is the discontinuous phase providing a gel-like material with a higher viscosity than for that of a liquid.

With the term "hydrophobicity" we refer to the effect that molecule is seemingly repelled from water, that is a molecule that has a logP > 0.

The terms "drug", "medicament", "agent", or "pharmaceutical agent" as used herein include, biologically, physiologically, or pharmacologically active substances that act locally or systemically in the human or animal body.

The terms "treating", "treatment" and "therapy" as used herein refer equally to curative therapy, prophylactic or preventative therapy and ameliorating therapy. The term includes an approach for obtaining beneficial or desired physiological results, which may be established clinically. For purposes of this invention, beneficial or desired clinical results include, but are not limited to, alleviation of symptoms, diminishment of extent of disease, stabilized (i.e., not worsening) condition, delay or slowing of progression or worsening of condition/symptoms, amelioration or palliation of the condition or symptoms, and remission (whether partial or total), whether detectable or undetectable.

The term "excipient" refers to pharmaceutically inactive compounds of the formulation, e.g. the carbohydrate esters, contrast agents, fluorophores, dyes, oils or solvents of the ECell composition.

The terms "low dielectric" media refers to a media with low dielectric constant, such as organic solvents, oils and the like.

The term "surface tension" is the surface energy of the liquid in contact with a gas phase (usually air).

The term "interfacial tension" is the surface energy between any two substances. It could be liquid-liquid, liquid-solid or solid-air.

As used herein, "drug", "therapeutic", "pharmaceutical", "active agent" or "bioactive agent" refers to any compound or mixture of compounds which produces a physiological result, e.g., a beneficial or useful result, upon contact with a living organism, e.g., a mammal, such as a human. Active agents are distinguishable from other components of the delivery compositions, such as carriers, diluents, binders, colorants, etc. The active agent may be any molecule, as well as binding portion or fragment thereof, that can modulate a biological process in a living subject. In certain embodiments, the active agent may be a substance used in the diagnosis, treatment, or prevention of a disease or as a component of a medication. In some embodiments, an active agent may refer to a compound that facilitates obtaining diagnostic information about a targeted site in a body of a living organism, such as a mammal or in a human. For example, imaging agents may be classified as active agents in the present invention as they are substances that provide imaging information required for diagnosis.

"Viscosity" herein refers to the state of being thick, sticky, and semi-fluid in consistency, due to internal friction. Viscosity is a quantity expressing the magnitude of internal friction in a fluid, as measured by the force per unit area resisting uniform flow. Viscosity can be quantified in the unit centipoise (cP) and can be measured using an EMS Viscometer (EMS-1000S).

"Release rate" herein refers to the rate or speed at which a compound such as a HIP complex of an API, excipient, imaging agent and the like are released from the ECell or depot into the aqueous embedding media or tissue.

"Release profile" herein refers to the profile said progression of the accumulated release of a compound as function of time.

"Imaging agent" herein refers to agents that provides contrast or visibility in imaging technologies, such as ultrasound sonography (US), magnetic resonance imaging (MRI), x-ray, fluoroscopy or Computed tomography (CT) contrast imaging, visible colours, fluorophores including dyes visible in the Infrared (IR) and Near Infrared (NIR) region, or radionuclides for PET and SPECT imaging, or the like.

### Detailed description of the invention

Carbohydrate ester depots also referred to as ECell are homogeneous liquids comprised of carbohydrate esters of mono-, di- and tri-saccharides, co-solvents such as but not limited to mono, di- and tri-glycerides, and a solvent such as ethanol (EtOH), propylene carbonate (PC), dimethyl sulphoxide (DMSO), benzyl alcohol (BnOH) and the like. The carbohydrate esters and co-solvents are hydrophobic whereas the solvent of the ECell is mixable with water. Upon injection of ECell into aqueous media or tissues, the ECell prefers to phase separate and its solvent to mix with water, which can occur via two scenarios. Either mixing of ECell solvent and water occurs outside the ECell or alternatively inside the ECell or combinations thereof. In the first and standard scenario, the solvent diffuses to the surface of the ECell where it mixes with water. This requires long diffusion distances, creates a compact ECell with no water pockets or channels, and the ECell does not expand in volume but rather collapses slightly due to solvent loss. In the second and new scenario, water flows into the ECell matrix by formation of channels, pores, and voids, in which the ECell solvent and water are mixed. In this process, the ECell volume and interfacial area are increased up to more than 500%. The formation of pores and void inside ECell creates shorter distances that the solvent needs to diffuse before mixing with water but requires formation of a much larger water-ECell interface and displacement of ECell material. Creation of interfacial area and displacement of ECell material is associated with energy cost that may be alleviated by lowering the interfacial tension and shear viscosity of the ECell. Polymers and lipids of the ECell composition can affect both shear viscosity and interfacial tension and are thus of key importance for controlling the expansive features of ECell.

With reference to above, it should be noted that some solvents induce expansion of the Ecell by influx of water. The polymer function is to stabilize the Ecell from collapse and to stabilize internal structures.

The current disclosure describes ECell compositions comprising carbohydrates ester, solvents, and polymers that surprisingly enable influx of water into the ECell upon contact with water or tissues and thereby expansion of the ECell matrix. Expansion of ECell may be obtained for a minimal composition only comprising specific combinations of carbohydrate esters and solvents. Addition of specific polymers stabilize the ECell structure on macro and microscopic length-scales and minimizes collapse of the ECell. Lipids may further affect the interfacial tension and regulate the structure of ECell. The expansion of ECell thus depends on the specific carbohydrate ester, the specific polymer, and choice of solvent.

Based on the above it should be noted that according to one embodiment of the present invention, the present invention provides a composition comprising:
- at least one gel-forming hydrophobic carbohydrate ester or an analogue thereof based on mono-, di-, and tri-saccharides, or mixtures thereof;
   and
- at least one solvent being mixable with water;
wherein the composition is a ECell being a hydrophobic gel-depot having the ability to take up water by means of said at least one solvent being mixable with water and having the ability to self-expand by formation of water pores, channels, and/or cavities.

As notable, in this case, the composition may exclude one or more polymers.

### ECell forming component

**ECell comprising components:** The ECell solution, is the solution of the composition before injection of the ECell solution into human or animal tissues or aqueous media, after which it forms the ECell as a gel or gel-like depot within the tissue or aqueous media. The ECell solution comprises organic solvents, oils (co-solvent), gel-forming carbohydrates esters, pharmaceutical agents (APIs), Imaging agents, polymers and is a homogeneous fluid with viscosities typically, but not limited to, in the range 50-5000cP. Upon administration of the ECell solution into tissues, the solution gets into contact with aqueous fluids, which causes a phase separation to occur. In this process, the organic solvent of the ECell solution diffuses into the aqueous phase (interstitial fluids), and the oil (co-solvent) and gel-forming carbohydrate esters forms a high viscosity fluid, solidify or precipitate or a combination thereof forming a hydrophobic depot (the ECell) at the site of injection. This phase separation can occur via two scenarios. Either mixing of ECell solvent and water occurs outside the ECell or alternatively inside the ECell or combinations thereof. In the first scenario, the solvent diffuses to the surface of the ECell where it mixes with water. In this process, the ECell volume remains constant or decreases slightly. In the second scenario, water flows into the ECell by formation of channels, pores, and voids, in which the ECell solvent and water are mixed. This formation of channels and uptake of water causes the ECell to expand in volume and expand its surface area. The expansive property of Ecell is governed by the solvents and formation and preservation of internal structures such as water channels and voids are supported by polymers. The viscous fluid, solid, precipitate or combinations thereof formed by this process is referred to as a gel or gel depot or the ECell.

**Solvents of the ECell solution:** Solvents of the ECell solution are soluble or mixable in hydrophobic substances such as the oil (co-solvent) and gel-forming carbohydrates, as well as in hydrophilic substances such as water. This partial hydrophilic / hydrophobic property of the solvents drives the non-solvent induced phase separation since the solvents of the ECell solution readily diffuses out of the ECell solution or ECell when exposed to an aqueous environment. The solvent further has a role in the expansion of ECell as some solvent induce expansion and others do not.

The chemical composition of the solvent (dispersion medium) should not be particularly limited, and examples include biocompatible organic solvents such as ethanol, ethyl lactate, propylene carbonate, glycofurol, N-methylpyrrolidone, 2-pyrrolidone, propylene glycol, acetone, methyl acetate, ethyl acetate, methyl ethyl ketone, benzyl alcohol, triacetin, dimethylformamide, dimethylsulfoxide, tetrahydrofuran, caprolactam, decylmethylsulfoxide, such as but not limited to N-methyl-2-pyrrolidone, glycofurol, polyethylene glycol (PEG), benzyl benzoate, triglycerides, acetone, benzyl alcohol, V-(betahydromethyl) lactamide, butylene glycol, caprolactam, caprolactone, corn oil, decylmethylsulfoxide, dimethyl ether, dimethyl sulfoxide, 1-dodecylazacyclo-heptan-2-one, ethanol, ethyl acetate, ethyl lactate, ethyl oleate, glycerol, glycofurol (tetraglycol), isopropyl myristate, methyl acetate, methyl ethyl ketone, esters of caprylic and/or capric acids with glycerol or alkylene glycols, oleic acid, peanut oil, polyethylene glycol, propylene carbonate, 2-pyrrolidone, sesame oil, [±]-2,2-dimethyl-1 ,3-dioxolane-4-methanol, tetrahydrofuran, diethylene glycol monoethyl ether, carbitol, triacetin, triethyl citrate, and combinations thereof; or desirably from trichlorofluoromethane, dichlorofluoromethane, tetrafluoroethane, dimethyl ether, propane, butane, and combinations thereof; or specifically from caprylic/capric triglyceride, oleic acid, 1-dodecylazacycloheptan-2-one and the like. Although the gel formulation can be stably dispersed in these solvents (dispersion media), the solvents may be further added with a saccharide derivatives of for example, triglycerides such as tri-pentanoyl glycerol, tri-octanoyl glycerol, tri-dodecanoyl glycerol, a monosaccharide such as glucose, galactose, mannose, fructose, inositol, ribose and xylose, disaccharide such as lactose, sucrose, cellobiose, trehalose and maltose, trisaccharide such as raffinose and melezitose, and polysaccharide such as α-, β-, or γ-cyclodextrin, sugar alcohol such as erythritol, xylitol, sorbitol, mannitol, and maltitol, or a polyhydric alcohol such as glycerin, diglycerin, polyglycerin, propylene glycol, polypropylene glycol, ethylene glycol, diethylene glycol, triethylene glycol, polyethylene glycol, ethylene glycol mono-alkyl ether, diethylene glycol mono-alkyl ether and 1,3-butylene glycol.

Examples of more preferable solvents are polyhydric alcohol such as glycerin, diglycerin, polyglycerin, propylene glycol, polypropylene glycol, ethylene glycol, diethylene glycol, triethylene glycol, polyethylene glycol, polyethylene glycol (PEG), benzyl benzoate, triglycerides, acetone, benzyl alcohol, ethanol, ethyl lactate, propylene carbonate and Dimethyl Sulfoxide, 1-butanol, 2-butanol, Tert-butylmethyl ether, Ethyl ether, Ethyl formate, Heptane, 3-Methyl-1-butanol, Methylisobutylketone, 2-Methylisobutylketone, 2-Methyl-I-propanol, Pentane, 1-Pentanol, 1-Propanol, 2-Propanol. Preferably, the solvents are selected from the group consisting of Ethanol (EtOH), Dimethyl sulphoxide (DMSO), Dimethyl formamide (DMF), N-methyl pyrrolidone (NMP), Propylene carbonate (PC), Benzyl alcohol (BnOH) or Butyl acetate (BuAc).

**Oils, also referred to as co-solvent, of the ECell solution:** Oils of the ECell solution are hydrophobic substances that mix poorly with aqueous media. Upon injection of a ECell solution into tissue or aqueous media, the oil (co-solvent) and gel-forming carbohydrate ester are separated from the solvent due to NIPS. During this phase separation, the oil (co-solvent) carbohydrate ester mixture forms a gel, gel depot or ECell with properties governed by the oil (co-solvent) and carbohydrate ester. The co-solvent is important for modulating and controlling the release rate of APIs from ECell.

Examples of oils (co-solvents) include, but are not limited to glycerol trivalerate, glycerol trihexanoate (GTH), glycerol trioctanoate (GTO), glycerol tridecanoate (GTD), ethyl octanoate, ethyl hexanoate, ethyl decanoate, Ethyl myristate, ethyl laurate, ethyl oleate, ethyl palmitate, ethyl myristate, corn oil, peanut oil, coconut oil, sesame oil, cinnamon oil, soybean oil, and poppyseed oil, or Lipiodol and aliphatic alkyl acyl esters.

**Gel-forming carbohydrate esters of the ECell solution:** Carbohydrate esters comprise the ECell forming constituents of the ECell solution. Upon solvent efflux caused by Non-solvent Induced Phase Separation (NIPS), the carbohydrate esters alone form viscous fluid depots, amorphous solid depots, crystal solid depots or mixtures thereof.

The gel-forming carbohydrate esters are suitable based on any of the following structures:

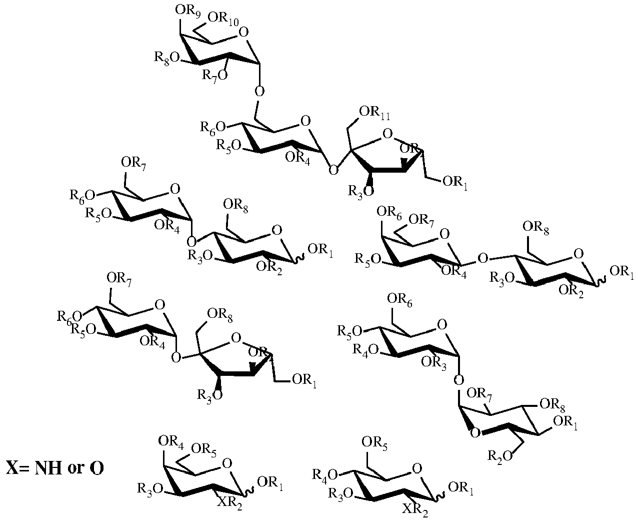

These are schematic views of chemical structures of exemplary carbohydrate esters, wherein R₁, R₂, R₃, R₄, R₅, R₆, R₇, R₈, R₉, R₁₀, and R₁₁ are selected collectively from the group consisting of hydrogen, methyl, alkanoyl, hydroxyl-substituted alkanoyl, and acyloxy-substituted alkanoyl, alkanyl, hydroxy-substituted alkanyl, acyloxy substituted alkanyl, benzoyl, and substituted benzyol; or wherein R₁, R₂, R₃, R₄, R₅, R₆, R₇, R₈, R₉, R₁₀, and R₁₁ are independently selected from the group consisting of hydrogen methyl alkanoyl, hydroxyl-substituted alkanoyl, cyloxy-substituted alkanoyl, alkanyl, hydroxysubstituted alkanyl, acyloxy substituted alkanyl, benzoyl and substituted benzoyl;or wherein all groups of R₁, R₂, R₃, R₄, R₅, R₆, R₇, R₈, R₉, R₁₀, and R₁₁ are selected collectively from the group consisting of methyl, acetyl, isobutyryl, propionyl or benzoyl; or wherein R₁, R₂, R₃, R₄, R₅, R₆, R₇, R₈, R₉, R₁₀, and R₁₁ are independently selected from the group consisting methyl, acetyl, isobutyryl, propionyl or benzoyl; and wherein both pure anomers and mixtures of α- and β- anomers of the above mentioned structural variations are claimed.

Examples of gel forming carbohydrate esters are carbohydrate ester analogues based on mono-, di-, and tri-saccharides such as but not limited to Glucose (dextrose), Fructose (levulose), Galactose, Sibose, Xylose, such as but not limited to Sucrose, Lactulose, Maltose, Trehalose, Cellobiose, Chitobiose, Isomaltise, such as but not limited to Nigerotriose, Maltotriose, Melezitose, Maltotriulose, Raffinose and Kestose.

Examples of carbohydrate esters include but are not limited to acetate, propionate, butyrate, iso-butyrate and benzoate esters and Sucrose, Lactose, Trehalose, Raffinose, and Maltose, such as Sucrose octaacetate, Sucrose octapropionate (SOP), Sucrose acetate isobutyrate (SAIB), Sucrose octaisobutyrate (SOIB), Sucrose octabenzoate (SuBen), Lactose octapropionate (LOP), lactose octaisobutyrate (LOIB), Lactose octabenzoate (LacBen), Rafinose undecapropionate (RUP), Rafinose undecaisobutyrate (RUIB), Rafinose undecabenzoate (RaBen), Trehalose octapropionate (TOP), Trehalose octaisobutyrate (TOIB), Trehalose octabenzoate (TreBen), Maltose octapropionate (MOP), Maltose octaisobutyrate (MOIB), Maltose octabenzoate (MaBen), MeLOIB (Methyl hepta-O-isobutyryl-α,β-lactoside), and the like.

The carbohydrates may be fully or partially functionalized/esterified with small organic acids such as but not limited to actetate, propanoic acid, butyrate, isobutyrate, valerate, iso-valerate, benzoic acid or mixtures thereof.

Most preferred carbohydrate esters are acetate, propionate, butyrate, isobutyrate esters and mixtures thereof of lactose, sucrose, trehalose, raffinose, and maltose such as but not limited to Lactose octapropionate (LOP), lactose octaisobutyrate (LOIB), Raffinose undecapropionate (RUP) Raffinose undecaisobutyrate (ROIB), Sucrose octapropionate (SOP), Sucrose acetate isobutyrate (SAIB), Sucrose octaisobutyrate (SOIB), Trehalose octapropionate (TOP), Trehalose octaisobutyrate (TOIB) and methoxy-LOIB (meLOIB).

**Polymers of the ECell solution:** The polymers of the ECell solution are hydrophobic substances that mix poorly or only partially with aqueous media. Depending on the hydrophobicity, the polymer may predominantly reside inside the ECell matrix or be presented at the surface of the ECell. Block co-polymers comprising both a hydrophobic and a hydrophilic domain may also be presented at the surface of the ECell matrix. Polymers may further be functionalized with Cell targeting or simulating ligand that can be presented at the interface of ECell. The polymers may stabilize the ECell matrix and provide mechanical stability of internal structures, such as water channels and voids. Examples of polymers providing support for internal structures of ECell *in vitro* and *in vivo* are presented in example 4, 6 and 12.

Examples of polymers of the ECell include but are not limited to polymers from the class polyethyleneimine (PEI), polylysine (PLL), polyarginine (PAA), Chitosans, Cellulose, Poly(2-ethyl-2-oxazoline) (ULTROXA), Diethylaminoethyl-dextran (DEAE-dextran), dendritic polyamidoamine (PAMAM) and PDMAEMA [poly(N,N-dimethylaminoethyl methacrylate], glycol (PEG), polylactic acid (PLA), poly(lactic-co-glycolic acid) (PLGA) and Poly(N-isopropylacrylamide) (PNIPAM) and the like.

Examples of polymers of the ECell include but are not limited to Poly D,L lactide-co-glycolide, PLGA-PEG-PLGA, Poly lactic acid (PLA), poly(lactic-co-glycolic acid) (PLGA), PEG, Polycaprolactone, Polycaprolactone diol, Poly(methyl methacrylate) (PMMA), PVP (polyvinyl pyrrolidone), Poly-allylamine, bPEI ( branched polyethylene imine), I-PEI (linear polyethylene imine), Poly(L-lactide) amine terminated, Trimethyl chitosan, Chitin (poly-N-acetyl glucosamine), Potassium hyaluronate (HA), Alpha tocopherol, Polypropylene glycol) monobutyl ether, PCL-PEG-PCL, Polycaprolactone diol, PEG-OMe, Carrageenan, 2-Diethylamino-etyl cellulose (DEAE cellulose), Cellulose acetate (CA), Ethyl Cellulose (EC), Methyl Cellulose (ME), Cyanoethyl cellulose, Cellulose acetate propionate (CAP), Cellulose acetate phthalate (CaPh), Cellulose acetate butyrate (CAB), Cellulose propionate (CP), Cellulose acetate propionate (CAP), 2-Hydroxyethyl cellulose, Hydroxypropyl cellulose, (Hydroxypropyl)methyl cellulose, Sodium carboxymethyl cellulose and the like.

Examples of polymers of the ECell include but are not limited to Poly D,L lactide-co-glycolide 75:25, 75-115 KDa, Ester terminated (PLGA ester). Poly D,L lactide-co-glycolide 75:25, 4-15 KDa, acid terminated (PLGA-COOH short). Poly D,L lactide-co-glycolide 75:25, 10-18 KDa, acid terminated (PLGA-COOH medium). Poly-L-lactide (PLA), 10-18 KDa, ester terminated (PLA ester medium). PLA, 18-28 KDa, ether terminated (PLA ether medium). PLA, 2 KDa. PLA, 50 KDa. Alpha -tocopherol PEG-1000 succinate (acid terminated) (VitE-PEG1K). Poly(propylene glycol) monobutyl ether (PPG), 2.5 KDa. PCL-PEG-PCL (1:1:1 KDa). Polycaprolactone diol (PCL Diol), 2 KDa. Polyethylene glycol methyl ether (PEG-OMe), 1KDa. PEG, 1.5 KDa. PEG(2000)-C18, 2 KDa. Polyethylene imine, branched (bPEI), 600 Da. bPEI, 1.2KDa. Cellulose acetate butyrate (CAB), 12KDa. CAB, 30 KDa. CAB, 70 KDa. Cellulose propionate (CP), 70 KDa. Celluose, acetate propionate (CAP), 75 KDa.

Preferred polymers are classes include but are not limited to PEI, CAB, PEG, PLA, PLGA and mixtures or block copolymers thereof.

Examples of polymers of the ECell include but are not limited to bPEI (branched polyethylene imine), I-PEI (linear polyethylene imine), Ethyl Cellulose (EC), Cyanoethyl cellulose, Cellulose acetate propionate (CAP), Cellulose acetate phthalate (CaPh), Cellulose acetate butyrate (CAB), Cellulose propionate (CP), Cellulose acetate propionate (CAP), 2-Hydroxyethyl cellulose, Hydroxypropyl cellulose, (Hydroxypropyl)methyl cellulose, Sodium carboxymethyl cellulose and the like.

**Lipids of the ECell solution:** Lipids and surfactant of the ECell composition are hydrophobic or amphipathic and form micelle, liposome or aggregate structures when in contact with water. Once ECell gets into contact with water or tissues, the lipids or surfactant may migrate to the interface and form a monolayer on the water-ECell interface. The lipids and surfactant will be oriented so that their hydrophobic domain is presented to the ECell matrix, whereas the hydrophilic or charged domain is facing water or tissue. Here the lipids and surfactants may modulate the interfacial tension and or present functional groups such as polymers and cell targeting and stimulating ligands on the ECell interface.

Examples of lipid and surfactant of the ECell include but are not limited to the class of carboxylic acids, amines, organophosphorus compounds, or organosulfur compounds functionalized with one or multiple aliphatic or aromatic C4-C22 hydrocarbon chains, or preferable functionalized with one or multiple aliphatic or aromatic C8-C16 hydrocarbon chains, or yet more preferable functionalized with one or multiple aliphatic C8-C12 hydrocarbon chains.

Examples of lipids and surfactants of the ECell include but are not limited to Acetic acid, propanoic acid, butanoic acid, hexanoic acid, octanoic acid, benzoic acid, Myristic acid, Myristoleic acid, Palmitic acid, Palmitoleic acid, Stearic acid, Oleic acid, Elaidic acid, Linoleic acid, Linolenic acid, Linolelaidic acid, Arachidonic acid, Elauricacid acid, Mycolic Acids, α-mycolic acid (C80), α-mycolic acid, methoxy cis, α-mycolic acid, keto cis, Cyclopropyl Lipids, Cis-9,10-methylenehexadecanoic acid, 1-palmitoyl-2-cis-9,10-methylenehexadecanoyl-sn-glycero-3-phosphocholine, 1-palmitoyl-2-cis-9,10-methylenehexadecanoyl -sn-glycero-3-phosphoethanolamine, (s)-12-methyltetradecanoic acid, 13-methyltetradecanoic acid, cis-9-oleicacid, cis-9-octadecenoicacid, trans-9-octadecenoicacid, (2-16)-octadecenoicacid, , 2-tridecenoic acid, 11-tridecenoic acid, 12 tridecenoicacid,2-dodecenoicacid, 5-dodecenoicacid, 6-dodecenoic acid, 7-dodecenoic acid, 9-dodecenoic, 10-dodecenoic acid, 11-dodecenoic acid, 11-eicosenoic acid, 14-eicosenoic acid, 2-undecenoic acid, 6-undecenoic acid, 9-undecenoic acid, 10 undecenoicacid, 2-decenoicacid, 3-decenoicacid, 8-decenoicacid, 9-decenoicacid, acrylic acid, A-methylacrylic acid, vinyl acetic acid, b-b-dimethylacrylic acid, beta-pentenoic acid, alylacetic acid, angelic acid, tiglic acid, 2-heptadecenoic, 9-hep-tadecenoic acid, 2-hexadecenoic acid, 9-hexadecenoicacid(cis form);2-tetradecenoicacid, 4-tetradecenoicacid, radecenoicacid, 8-tetradecenoicacid;9-tetradecenoic acid; 2-nonenoicacid, 3-nonenoicacid, 8-nonenoicacid, 2-octenoicacid, 3-octenoicacid, 7-octenoicacid, 2-hep tenoicacid, 3-heptenoicacid;4-heptenoicacid, 5-hep tenoicacid, 6-heptenoicacid, 2-hexenoicacid, 3-hexenoicacid, 4-hexenoicacid, 5-hexenoicacid,15-tetracosenoicacid;17-hexacosenicacid, 21-triacentenoic acid, 2- to 8-nonynoic acid, Octadecadienoic acid 8:10, oleic acids (i.e.,cis-9-oleicacidorcis-9-octadecenoicacid), octadecadienoicacid,(8-trans and 10-transforms), 8:11 acidsiselaidicacid(i.e.,trans-9-octadecenoicacid), octadecadienoicacid,(8-cis and 11-cis forms), 9:11 octadecadienoicacid,(9-cis and 11-cis and I-trans forms), 5:12-octadecadienoic acid, (5-cis, 5-trans, 12 trans and 12-cisforms), 9:12-octadecadienoic acid,(9- cis, 9-trans, 12-trans and 12-cis forms), 10:12 octadecadienoic acid, (10-cis, 10-trans, 12-cis and 12 trans forms), 10:13-octadecadienoic acid, (10-cis and 13-cisforms), 11:14-octadecadienoicacid,(11-cis and 14-cisforms), beta-vinylacrylic acid, sorbic acid, geranicacid, tetra-triethenoidfaty acids, triethenoidfaty acid, clupandoic acid, moroctic acid, arachidonic acid, alpha and beta parinaric acids, oleicacid, linoleicacid,licanic acid, eleostearic acid, ricinoleicacid, clupanodonicacid , palmitoleic acid, eleostearic acid, cis-11-methyl-2-dodecenoic acid, trans-2-decanoic acid (T2DA), heptylcyclopropane-1-carboxylic acid (2CP), palmitic acid, Cis-2-decanoic acid (C2DA), palmitoleic acid, Palmitelaidic acid, 7(Z),10(Z)-Hexadecadienoic acid, Linoleic acid, γ-Linolenic acid, Arachidonic acid, chenodeoxycholic acid, deoxycholic acid, formic acid or hexanoic acid, and the like. 1-Hydroxy-2-naphthoic acid (xinafoic acid), 2-Naphthalene sulfonic acid (NSA), Brilliant blue FCF, Carboxy methyl polyethylene glycol (CM-PEG), Cholesteryl hemisuccinate, Cholic acid (sodium cholate), Decanoic acid (sodium decanoate/sodium caprate), Docosahexaenoic acid, Hexadecylphosphate, Linoleic acid, N,N-Dipalmitoyl-L-lysine, Oleic acid (sodium oleate also used), Pamoic (disodium pamoate also used), acetate, cholesteryl sulfate, Sodium decanesulfonate (SDES), deoxycholate, docusate (AOT, dioctyl sulfosuccinic acid, bis-2- ethylhexylsulfosuccinate), dodecyl benzenesulfonate (SDBS), dodecyl sulfate (sodium lauryl sulfate), laurate (sodium dodecanoate), n-octadecyl sulfate (sodium stearyl sulfate), stearate (stearic acid also used), stearoyl glutamate (SSG), taurodeoxycholate (STDC), tetradecyl sulfate, tripolyphosphate, Taurocholic acid (sodium taurocholate also used), Vitamin E (a-tocopherol) succinate, Arginine-hexadecanoyl ester (AHE), Arginine-nonyl ester (ANE), Benethamine (N-benzyl-2-phenylethanamine), Dodecylamine (laurylamine), Hexadecyl trimethylammonium(cetrimonium) bromide (CTAB), Maprotiline, Na-Deoxycholyl-L-lysyl-methylester, N,N'-Dibenzyl ethylenediamine-(benzathine), N,N-Dimethyl dodecylamine (DDA), N,N-Dimethyl hexylamine, N,N-Dimethyl octadecylamine(dimethyl stearamine), Stearylamine(octadecylamine), Tetrabutyl ammonium bromide (TBAB), Tetraheptyl ammonium bromide (THA), Tetrahexyl ammonium bromide, Tetraoctyl ammonium bromide (TOAB), Tetrapentyl ammonium bromide (TPA), Triethylamine (TEA), Cholesteryl, hemisuccinate, Sulfonated Cholesterol, 25-hydroxycholesterol-3-sulfate, cholesterol 3-sulfate, Dehydroepiandrosterone sulfate and the like.

Examples of lipids and surfactants of the ECell include but are not limited to compounds with one or multiple ionizable groups belonging to the group of lipids comprising phosphatidic acid (PA), cyclic PA, lysophosphatidic acid (LPA), cyclic LPA, phosphatidylglycerol (PG), lysophosphatidylglycerol (LPG), phosphoinositides (PI), lysophosphatidylinositol (LPI), phosphatidylserine (PS), or Lysophosphatidylserine (LPS) , Phosphatidylcholine (PTC), Lysophosphatidylcholine (LPC), phospholipids and or ether lipids, or sphingolipids (SP) or sphingolysolipids (LSP), all with mixed or non-mixed, saturated and or unsaturated, aliphatic and or aromatic, C6-C22 hydrocarbon chains. Preferred examples of hydrocarbon chains are but not limited to propionyl, butyryl, hexanoyl, octanoyl, decanoyl, lauroyl, myristoyl, palmitoyl, stearoyl, arachidoyl, behenoyl, lignoceroyl and the like. Other preferred examples of hydrocarbon chains are vaccenoyl, (8Z)octadecenoyl, myristoleoyl, myristelaidoyl, palmitoleoyl, palmitelaidoyl, oleoyl, dielaidoyl and the like.

Examples of lipids and surfactants of the ECell include but are not limited to compounds with one or multiple ionizable groups belonging to the group of lipids comprising PTC, LPC, PA, LPA, PG, LPG, PI, LPI, PS, or LPS phospholipids, or SP or LSP all with mixed or non-mixed, saturated or unsaturated, sterol modified, fatty acid modified, or headgroup modified such as but not limited to PEGylated, fluorophore or chelator functionalized. Functionalized here includes but is not limited to deuteration, fluorescence, biotinylation, diphytanoylation, bromination, oxidization, diacetylenation, fluorination, and modifications with bioactive molecules.

Examples of lipids and surfactants of the ECell include but are not limited to compounds with one or multiple ionizable groups belonging to the group of multivalent cationic lipids such as but not limited to N1-[2-((1S)-1-[(3-aminopropyl)amino]-4-[di(3-amino-propyl)amino]butylcarboxamido)ethyl]-3,4-di[oleyloxy]-benzamide or N4-Cholesteryl-Spermine.

Examples of lipids and surfactants of the ECell include but are not limited to compounds with one or multiple ionizable groups belonging to the group of ionizable cationic lipids such as but not limited to DOBAQ (N-(4-carboxybenzyl)-N,N-dimethyl-2,3-bis(oleoyloxy)propan-1-aminium), 1,2-distearoyl-3-dimethylammonium-propane, 1,2-dipalmitoyl-3-dimethylammonium-propane, 1,2-dimyristoyl-3-dimethylammonium-propane, 1,2-dioleoyl-3-dimethylammonium-propane (DODAP), 1,2-dioleyloxy-3-dimethylaminopropane (DODMA), DLin-DMA, DLin-KC2-DMA, DLin-KC3-DMA, C12-200, A6, OF-02, A18-Iso5-2DC18, YSK05, 7C1, G0-C14, L319, OF-Deg-Lin, 306-O12B, 306O₁₁₀, FTT5, 9A1P9, 98N₁₂-5, 304O₁₃, cKK-E12 and the like.

Examples of lipids and surfactants of the ECell include but are not limited to compounds with one or multiple ionizable groups belonging to the group of fixed cationic lipids such as but not limited to 3β-[N-(N',N'-dimethylaminoethane)-carbamoyl]cholesterol (DC-Cholesterol), N-(2-hydroxyethyl)-N,N-dimethyl-2,3-bis(oleoyloxy)propan-1-aminium (DORI), O,O'-ditetradecanoyl-N-(α-trimethylammonioacetyl)diethanolamine, ethylphosphocholines (EPCs) of lauroyl, myristoyl, palmitoyl, stearoyl, and oleoyl. Such as Dimethyldioctadecylammonium, 1,2-dimyristoyl-3-trimethylammonium-propane, 1,2-dipalmitoyl-3-trimethylammonium-propane, 1,2-stearoyl-3-trimethylammonium-propane and 1,2-dioleoyl-3-trimethylammonium-propane. Such as 1,2-di-O-octadecenyl-3-trimethylammonium propane.

Examples of lipids and surfactants of the ECell include but are not limited to sterols such as but not limited to cholesterol, lanosterol, ergosterol and the like, C12-C18 acyl saturated and unsaturated phosphatidylcholine (PTC) or Phosphatidylethanolamine (PE) or Phosphatidylglycerol (PG) phospholipids such as 1,2-dilauroyl-sn-glycero-3-phosphocholine (DLPC), 1,2-dimyristoyl-sn-glycero-3-phosphocholine (DMPC), 1,2-dipalmitoyl-sn-glycero-3-phosphocholine (DPPC), 1,2-distearoyl-sn-glycero-3-phosphocholine (DSPC), 1,2-dioleoyl-sn-glycero-3-phosphocholine (DOPC), 1,2-dilauroyl-sn-glycero-3-phosphoethanolamine (DLPE), 1,2-dimyristoyl-sn-glycero-3-phosphoethanolamine (DMPE), 1,2-dipalmitoyl-sn-glycero-3-phosphoethanolamine (DPPE), 1,2-distearoyl-sn-glycero-3-phosphoethanolamine (DSPE), 1,2-dioleoyl-sn-glycero-3-phosphoethanolamine (DOPE), 1,2-dilauroyl-sn-glycero-3-phospho-(1'-rac-glycerol) (DLPG), 1,2-dimyristoyl-sn-glycero-3-phospho-(1'-rac-glycerol) (DMPG), 1,2-dipalmitoyl-sn-glycero-3-phospho-(1'-rac-glycerol), (DPPG), 1,2-distearoyl-sn-glycero-3-phospho-(1'-rac-glycerol) (DSPG), 1,2-dioleoyl-sn-glycero-3-phospho-(1'-rac-glycerol) (DOPG), 1-palmitoyl-2-oleoyl-glycero-3-phosphocholine (POPC),
or such as but not limited to PEGylated lipids are selected from the list of DLPE, DMPE, DPPE, DSPE, DOPE phospholipids PEGylated with either PEG350, PEG500, PEG750, PEG1k, PEG2k, PEG3k or PEG5k, sterols such as cholesterol PEGylated with either PEG350, PEG500, PEG600, PEG750, PEG1k, PEG2k, PEG3k or PEG5k or 1,2-Dimyristoyl-rac-glycero-3-methoxypolyethylene glycol-2000 (DMG-PEG2k), Distearoyl-rac-glycerol-PEG2K (DSG-PEG2k),

Examples of lipids and surfactants of the ECell include but are not limited to the cationic lipids: DC-chol, DMTAP, DPTAP, DSTAP, DOTAP, DOTMA, DOSPA, DDAB, DMDAP, DPDAP, DSDAP, DODAP, DODMA, DOBAQ, DLin-DMA, DLin-KC2-DMA, DLin-MC3-DMA, C12-200, A6, OF-02, A18-Iso5-2DC18, YSK05, 7C1, G0-C14, L319, OF-Deg-Lin, 306-O12B, 3060110, FTT5, 9A1P9, 98N12-5, 304013, cKK-E12, Spermine-chol, and MVL5

Examples of lipids and surfactants of the ECell include but are not limited to lipids and polymers functionalized with the ligands including but not limited to RGD, ceruloplasmin, prothrombin, alpha-2-HS-glycoprotein, alpha-1-antichymotrypsin,proline-rich peptides, arginine-rich peptides, lysine-rich peptides, Pep-1, L-oligomers, Calcitonin peptide(s).

Examples of lipids and surfactants of the ECell include but are not limited to a lipid or mixtures thereof from the list of Chems, DMG-PEG2K, Igepal Ca-720, PEG(20)-monooleyl ether, Monolaurin, Oleic acid, Lauric acid, PEG(10)-monolaurate, Tween 80, DSPE-PEG(2000)-RGD, DSPE-PEG(1000)-RGD, DSPE-PEG(2000), POPC, DOTAP, DC-Chol, DSPE-PEG(350Da) and the like.

**Radiographic and CT contrast agents for the ECell solution:** ECell formulations containing lipiodol as a CT contrast oil (co-solvent) or CLA-8 (α,β Lactose octa para-iodobenzoate, or alternatively named CLA8) or xSAIB (6,6'-(2,4,6-triiodophenoxy)acetoxy-isobutyric-sucrose) as CT contrast agent may be used as fiducial markers for guiding therapeutic interventions, including, but not limited to; external beam radiotherapy or surgical procedures. CT contrast in ECell may generally be used for CT, radiography or fluoroscopy guided local injection. Alternative CT contrast agent are lactose or sucrose octa ortho, meta or para iodobenzoate esters.

**Other constituents of the ECell solution:** In one embodiment a polymer may be used to work as a stabilizer between the ECell and biological surrounding and therefore, the composition may also comprise a molecule that increase ECell stability in the human or animal body, such as an amphiphilic molecule, such as an emulsifier. Therefore, in one embodiment the composition comprises poly(ethylene glycol-b-caprolactone) (PEG-PCL), sucrose acetate isobutyrate (SAIB), poly(*D,L*-lactic acid) (PLA), or poly(lactic-co-glycolic acid) (PGLA), or a combination thereof. In one embodiment of the present invention poly(*D,L*-lactic acid) (PLA) is added to the non-water-soluble carbohydrate causing a reduction of burst release of said encapsulated contents e.g., drugs, particles, contrast agents, etc. The formulation may further include other constituents, such as α-, β-, and/or γ-cyclodextrins and any derivate hereof.

The formulation may further comprise compounds or polymers, which are visible in imaging modalities other than X-ray imaging.

**Pharmaceutical agent of the ECell solution:** The gel-forming ECell composition may further comprise pharmaceutical agents including prodrugs (in short "drugs"; broadly interpreted as agents which are able to modulate the biological processes of a human or animal). These drugs, prodrugs, HIPs of APIs and APIs or the like can be formulated as a single drug or as a combination of two or more in the ECell composition.

The ECell formulation that as such has a comparable low viscosity is intended for injection in the body of a human or animal, where after the formulation becomes more viscous, i.e. it goes through a sol-gel transition (liquid to gel) transition, due to the presence of the gel-forming system. It is preferred that the viscosity of the formulation after injection in the body of a human or animal increases by at least 50 %, such as at least 80 %, such as at least 100 %, or at least 150 %, or at least 200 %, or at least 300 %, or at least 500 %, or at least 750 %, or at least 1000 %, or at least 10,000%, or that the formulation becomes essentially solid (non-viscous). The formulation is preferably adapted for injection via a thin needle used for injection into a body or surgical related procedures, such as but not limited to biopsy. The viscosity of the gel-forming formulation before injection can be any suitable viscosity such that the formulation can be parenterally administered to a patient. Exemplary formulations include, but are not limited to, those having a viscosity (prior to administration/injection) lower than 10,000 centipoise (cP), e.g. lower than 2,000 cP, such as 10 to 2,000 cP, such as 20 to 1,000 cP, such as 150 to 350 cP, such as 400 to 600 cP, such as 600 to 1,200 cP or such as 1,000 to 2,000 cP, or 10 to 600 cP, or 20 to 350 cP, at 20 °C. Alternative formulations include, but are not limited to, those having a viscosity (prior to administration/injection) lower than 10,000 centipoise (cP), e.g. lower than 2,000 cP, such as 10 to 2,000 cP, such as 20 to 1,000 cP, such as 150 to 350 cP, such as 400 to 600 cP, such as 600 to 1,200 cP or such as 1,000 to 2,000 cP, or 10 to 600 cP, or 20 to 350 cP, at 5 °C. When referred to herein, the (dynamic) viscosity is measured at the specified temperature in accordance with the method described in ASTM D7483.

### Some further embodiments of the present invention

Below some embodiments of the present invention are provided.

According to one embodiment, the gel-forming carbohydrate ester is a carbohydrate ester analogue based on glucose, fructose, galactose, sibose, xylose, sucrose, lactulose, maltose, trehalose, cellobiose, chitobiose, isomaltise, nigerotriose, maltotrios, melezitose, maltotriulose, raffinose and kestose, or a combination thereof.

According to yet another embodiment, the gel-forming carbohydrate ester is selected from sucrose acetate isobutyrate (SAIB), sucrose octapropionate (SOP), sucrose octaisobutyrate (SOIB) sucrose octabenzoate (SuBen), lactose octapropionate (LOP), lactose octaisobutyrate (LOIB), methoxy- lactose octaisobutyrate (MeOLOIB), methyl hepta-O-isobutyryl-α,β-lactoside (MeLOIB), lactose octabenzoate (LacBen), trehalose octapropionate (TOP), trehalose octaisobutyrate (TOIB), trehalose octabenzoate (TreBen), rafinose undecaisobutyrate (RUIB), and rafinose undecabenzoate (RaBen), or a combination thereof;
preferably the gel-forming carbohydrate ester is selected from LOP, LOIB, MeLOIB, SOP, SAIB, SOIB, TOP and TOIB, or a combination thereof.

Furthermore, according to one specific embodiment, the polymer is selected from polyethyleneimine (PEI), polylysine (PLL), polyarginine (PAA), Chitosan, Cellulose, Poly(2-ethyl-2-oxazoline) (ULTROXA), Diethylaminoethyl-dextran (DEAE-dextran), dendritic polyamidoamine (PAMAM) and [poly(N,N-dimethylaminoethyl methacrylate] (PDMAEMA), polylactic acid (PLA), poly(lactic-co-glycolic acid) (PLGA) and Poly(N-isopropylacrylamide) (PNIPAM), Poly D,L lactide-co-glycolide, PLGA-PEG-PLGA, Poly lactic acid (PLA), poly(lactic-co-glycolic acid) (PLGA), Polyethylene glycol (PEG), Polycaprolactone, Polycaprolactone diol, Poly(methyl methacrylate) (PMMA), polyvinyl pyrrolidone (PVP), Poly-allylamine, b-PEI (branched polyethylene imine), I-PEI (linear polyethylene imine),Poly(L-lactide) amine terminated, Trimethyl chitosan, Chitin Potassium hyaluronate (HA), Alpha tocopherol, Polypropylene glycol) monobutyl ether, PCL-PEG-PCL, Polycaprolactone diol, PEG-OMe, Carrageenan, 2-Diethylamino-etyl cellulose (DEAE cellulose), Cellulose acetate (CA), Ethyl Cellulose (EC), Methyl Cellulose (ME), Cyanoethyl cellulose, Cellulose acetate phthalate (CaPh), Cellulose acetate butyrate (CAB), Cellulose propionate (CP), Cellulose butyrate (CB), Celluose acetate propionate (CAP), 2-Hydroxyethyl cellulose, Hydroxypropyl cellulose, (Hydroxypropyl)methyl cellulose, and Sodium carboxymethyl cellulose, or a combination thereof;
preferably the polymer is selected from polyethyleneimine (PEI), Ethyl Cellulose (EC), Cyanoethyl cellulose, Cellulose acetate propionate (CAP), Cellulose acetate phthalate (CaPh), Cellulose acetate butyrate (CAB), Cellulose propionate (CP), Cellulose butyrate (CB), Celluose acetate propionate (CAP), 2-Hydroxyethyl cellulose, Hydroxypropyl cellulose, (Hydroxypropyl)methyl cellulose, and Sodium carboxymethyl cellulose), or a combination thereof;
preferably the polymer is selected from Ethyl Cellulose (EC), Cellulose acetate butyrate (CAB), Cellulose propionate (CP), Cellulose butyrate (CB), and Cellulose acetate propionate (CAP), or a combination thereof;
preferably CAB.

According to yet another embodiment, the solvent is selected from glycerol, diglycerol, polyglycerol, propylene glycol, polypropylene glycol, ethylene glycol, diethylene glycol, triethylene glycol, polyethylene glycol, benzyl benzoate, triglycerides, acetone, benzyl alcohol, ethanol, ethyl lactate, propylene carbonate and dimethyl sulfoxide, or a combination thereof; preferably the solvent is selected from ethanol, propylene carbonate, dimethyl sulphoxide, and benzyl alcohol, or a combination thereof; preferably dimethyl sulphoxide.

Also one or more co-solvents may be present. In line with this, according to one embodiment, the composition further comprises at least one co-solvent being an oil selected from of tripropionin, tributyrin, glycerol trivalerate, glycerol trihexanoate (GTH), glycerol trioctanoate (GTO), glycerol tridecanoate (GTD), glycerol tridodecanoate, ethyl hexanoate, ethyl octanoate, ethyl decanoate, ethyl laurate, ethyl myristate, ethyl palmitate, ethyl stearate, ethyl oleate, corn oil, peanut oil, coconut oil, sesame oil, cinnamon oil, soybean oil, and poppyseed oil, or a combination thereof; preferably glycerol trihexanoate (GTH), glycerol trioctanoate (GTO), glycerol tridecanoate (GTD), or Ethyl-palmitate, or a combination thereof.

According to yet another embodiment, the composition further comprises one or more lipids selected from cholesterol, lanosterol, ergosterol, DLPC, DLPE, DLPG, DMPC, DMPE, DMPG, DPPC, DPPE, DPPG, DSPC, DSPE, DSPG, DOPC, DOPE, DOPG, phospholipids (DLPE, DMPE, DPPE, DSPE, or DOPE) PEGylated with either PEG350, PEG500, PEG750, PEG1k, PEG2k, PEG3k or PEG5k; cholesterol PEGylated with either PEG350, PEG500, PEG600, PEG750, PEG1k, PEG2k, PEG3k or PEG5k; 1,2-Dimyristoyl-rac-glycero-3-methoxypolyethylene glycol-2000 (DMG-PEG2k), Distearoyl-rac-glycerol-PEG2K (DSG-PEG2k), DC-chol, DMTAP, DPTAP, DSTAP, DOTAP, DOTMA, DOSPA, DDAB, DMDAP, DPDAP, DSDAP, DODAP, DODMA, DOBAQ, DLin-DMA, DLin-KC2-DMA, DLin-MC3-DMA, C12-200, A6, OF-02, A18-Iso5-2DC18, YSK05, 7C1, G0-C14, L319, OF-Deg-Lin, 306-O12B, 3060110, FTT5, 9A1P9, 98N12-5, 304013, cKK-E12, Spermine-chol, and MVL5;
preferably one or more lipids selected from zwitterionic lipids, cationic lipids, anionic lipids, or pegylated lipids, or a combination thereof.

Furthermore, according to one embodiment, the polymers of claim 4 or the lipids of claim 7 are functionalized with groups selected from:
the ligands RGD, ceruloplasmin, prothrombin, alpha-2-HS-glycoprotein, alpha-1-antichymotrypsin,proline-rich peptides, arginine-rich peptides, lysine-rich peptides, Pep-1, L-oligomers, and Calcitonin peptide(s),
cell penetrating peptides (CPP), such as TAT, KALA;
ligands of a receptor, such as cytokines, hormones, or growth factors;
small molecules (e.g. carbohydrates like mannose or galactose or synthetic ligands), small molecule agonists, inhibitors or antagonists of receptors (e.g. RGD peptidomimetic analogues) or any such molecule. Particularly preferred are cell penetrating peptides (CPPs), which include, without being limited thereto protamine, nucleoline, spermine or spermidine, poly-L-lysine (PLL), basic polypeptides, poly-arginine, chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from Drosophila antennapedia), pAntp, plsl, etc., antimicrobial-derived CPPs e.g. Buforin-2, Bac715-24, SynB, SynB(1), pVEC, hCT-derived peptides, SAP, MAP, PpTG20, proline-rich peptides, Loligomers, arginine-rich peptides, Calcitonin-peptides, FGF, Lactoferrin, poly-L-lysine, poly-arginine, histones, VP22 derived or analog peptides, Pestivirus Erns, HSV, VP22 (Herpes simplex), MAP, KALA or protein transduction domains (PTDs, PpT620, proline-rich peptides, arginine-rich peptides, lysine-rich peptides, Pep-1, L-oligomers, Calcitonin peptide(s).

The present invention is both directed to imaging applications as well as pharmaceutical applications, such as for controlled release.

When referring to imaging applications, according to one embodiment, the composition further comprises at least one excipient selected from CT contrast agents, radiographic contrast agents, fluorophores, chromophores, MRI contrast agents, radiotracers, and contrast carrying nanoparticles, or a combination thereof. Furthermore, according to one embodiment, the CT (and radiographic) contrast agents are selected from lipiodol, α-, β-Lactose octa para-iodobenzoate (CLA-8), 6,6'-(2,4,6-triiodophenoxy)acetoxy-isobutyric-sucrose (xSAIB), lactose, sucrose octa para-iodobenzoate, and octa meta-iodobenzoate.

As said, also pharmaceutical applications are of interest according to the present invention. According to one embodiment, the composition further comprises at least one therapeutic agent selected from anticancer agents, immunotherapeutics, chemotherapeutics, antimicrobials, antibiotics, antivirals, antifungals, antiprotozoals, anthelminthics, anti-inflammatory agents, anticoagulant, antidepressant, antiepileptic, antipsychotic, sedatives, antidiabetic, cardiovascular, analgesic agents, therapeutic peptides, diagnostic peptides, agonistic receptor ligands, antagonistic receptor ligands, peptides with regulatory or enzymatic activity, hormones, peptides with special targeting, vaccines, antigens, steroids, immunotherapeutic agonists, immunotherapeutic antagonists, growth factors, cytokines, chemokines, hormones, glycoprotein hormones, antibodies, affibodies, and nanobodies, or a combination thereof.

Moreover, according to yet another embodiment, the composition comprises the
carbohydrate ester in a range of 50-90 w/w%, preferably 60-80w/w% preferably 70 w/w%, of the ECell; or
wherein the composition comprises the polymer in a range of 1-20 w/w%, preferably 2.5-15 w/w%, preferably 5 w/w%, of the ECell; or
wherein the composition comprises the co-solvent in a range of 1-20 w/w%, preferably 2.5-15 w/w%, preferably 5 w/w%, of the ECell; or
wherein the composition comprises the lipid in a range of 0.01-10 w/w%, preferably 0.1-5 w/w%, preferably 1 w/w%, of the ECell.

As said, different use areas of relevance for the present invention. According to one embodiment, there is provided a composition according to the present invention, for use in therapy, preferably for use in therapy selected from cancer therapy, such as immunotherapy, chemotherapy, cryotherapy, ablation therapy, adoptive cell transfer; adjuvant therapy for radiation therapy or cancer surgery; pre-, intra-, and post-surgical therapy for improved healing, bone loss, peritendinous adhesions, or bone non-union; infections associated with, soft tissue, bones, abscesses, phlegmons, fistulas; infections associated with, or in, catheters, drains, stents, tissue grafts, transplantations, transplanted organs, artificial organs, hearing aids, implanted devices, bone implants, metal or ceramic internal or external fixation devices, artificial organs, protheses, reconstructive surgery, plastic surgery, tissue loss, tissue defects, or pain relief; local or systemic analgesia or anaesthesia; and local or systemic immune suppression; or for use in the treatment of diseases selected from cancers, such as myelodysplastic syndrome; autoimmune disorders; rheumatoid disease; degenerative disorders; anaemia; endocrine disorders, such as endocrine gland hyposecretion, endocrine gland hypersecretion, tumours (benign or malignant) of endocrine glands, hormone replacements, hormone supplementation, hormone imbalances, hormone inactivation, adoptive cell therapies; and stem cells, such as chimeric antigen receptor cell products, or myeloid and lymphoid cell products.

The present invention also provides the composition as disclosed herein for use as:
i) fiducial markers with positive MRI contrast for guiding external beam radiotherapy (EBRT), image guided interventions, such as surgery or biopsy procedures;
ii) surgical void filler in debrided bone infections, bone trauma, tissue defects after surgical interventions, trauma, plastic surgery or reconstructive surgery;
iii) for expanding void filler or tissue spacer for minimizing normal tissue damage associated with radiation therapy, thermal ablation, or cryotherapy, such as for use as a spaceoar hydrogel;
iv) surgical spacing for sparing fragile structures in radiation therapy;
v) cell adaptive regenerative tissue scaffolds;
vi) scaffolds that supports cell engraftment and polarizations for cell therapies such as stem cell therapy;
vii) expanding drug eluding depot with tailored drug release profiles; or vii) prostate rectum spacing.

Furthermore, the present invention also provides a method comprising using a composition according to the present invention, wherein the method comprises the steps of:
- injecting the composition into water, aqueous media or tissue, wherein the composition is a ECell being a hydrophobic gel-depot is expanding by uptake of the water by means of said at least one solvent being mixable with water and having the ability to self-expand by formation of water pores, channels, and/or cavities; and
- locating the expanded ECell by means of imaging;
   or
   using the composition according to the present invention, in controlled release of at least one therapeutic agent, wherein the method comprises the steps of:
- injecting the composition into water, aqueous media or tissue, wherein the composition is a ECell being a hydrophobic gel-depot is expanding by uptake of the water by means of said at least one solvent being mixable with water and having the ability to self-expand by formation of water pores, channels, and/or cavities, and
- discharging of the at least one therapeutic agent from the ECell by controlled release.

### More embodiments of the present invention

The present disclosure provides compositions that contrary to previous ECells surprisingly take up water, and expands by formation of water pores, channels, and cavities inside the ECell. Upon injection, the ECells can expand more that 5-fold and may contain more than 80% v/v water in the final expanded stage. In this process, the interfacial area per volume is highly increased, which may affect hydrolysis and degradation of the ECell and provides shorter path for diffusion of sustained released molecules, and which may alter release kinetics of embedded compounds/APIs. Compositions of the current disclosure further allows for modified surface charge, polymer grafting and presentation of cell signal or targeting peptide sequences, which enhances the adaptation and compatibility of ECell with tissues. These new features of ECell allows for use as: i) fiducial marker with positive MRI contrast for guiding external beam radiotherapy (EBRT), image guided interventions, such as surgery and biopsy procedures, ii) surgical void filler in debrided bone infections, bone trauma, tissue defects after surgical interventions, trauma, plastic surgery, reconstructive surgery, iii) for expanding void filler or spacer for minimizing normal tissue damage associated with radiation therapy, thermal ablation, cryotherapy, v) surgical spacing for sparing fragile structures, iv) for cell adaptive regenerative tissue scaffold, iv) scaffold that supports cell engraftment and polarizations for cell therapies including stem cell therapy and as vii) expanding drug eluding depot with tailored drug release profiles.

The current disclosure provides ECells compositions with altered release profiles for a range of APIs. This has important therapeutic indication for compound classes including but not limited to small molecule therapeutics, such as anticancer agents, immunotherapeutic, chemotherapeutics, antimicrobials (antibiotics, antivirals, antifungals, antiprotozoals, and anthelminthics), anti-inflammatory agents, anticoagulant, antidepressant, antiepileptic, antipsychotic, sedatives, antidiabetic, cardiovascular, and analgesic agents, peptides such as, but not limited to, therapeutic peptides, agonistic and antagonistic receptor ligands, peptides with regulatory or enzymatic activity, hormones, peptides with special targeting activities, vaccines, antigens, therapeutic peptides, diagnostic peptides, steroids, proteins such as but not limited to immunotherapeutic agonists and antagonists, growth factors, cytokines, chemokines, hormones, glycoprotein hormones and antibodies, affibodies, peptide and nanobodies and formulation of functional ECell excipients such as but not limited to CT contrast agents, radiographic contrast agents, fluorophores, chromophores, MRI contrast agents, radiotracers, and contrast carrying nanoparticles.

The expanding ECells with optimized surface properties may be used for a range of indications. Indications include, but are not limited to: vaccination against; cancer (cancer antigens and neoantigens), infectious diseases, cancer therapy; immunotherapy, chemotherapy, adjuvant therapy for radiation therapy, cancer surgery, cryotherapy, ablation therapy, adoptive cell transfer, pre-, intra-, and post-surgical therapy for; improved healing, bone loss, peritendinous adhesions, bone non-union, infections associated with; soft tissue, bones, abscesses, phlegmons, fistulas, infections associated with, or in, catheters, drains, stents, tissue grafts, transplantations, transplanted organs, artificial organs, hearing aids, implanted devices, mechanical devices, electronic devices, bone implants (hip, knee, joint), metal and ceramic internal and external fixation devices, artificial organs, protheses, reconstructive surgery, plastic surgery, tissue loss, tissue defects, pain relief; local and systemic analgesia and anaesthesia, local or systemic immune suppression, autoimmune disorders, rheumatoid disease, degenerative disorders, anaemia, myelodysplastic syndrome, endocrine disorders; endocrine gland hyposecretion, endocrine gland hypersecretion, tumours (benign or malignant) of endocrine glands, hormone replacements, hormone supplementation, hormone imbalances, hormone inactivation, adoptive cell therapies; stem cells, chimeric antigen receptor cell products, myeloid and lymphoid cell products

APIs in ECell may provide a controlled therapeutic effect locally, loco-regionally, intracavitary or provide a systemic effect following local release. The formulation of small molecule therapeutics, peptides, proteins, glycoproteins, glycopeptides, bioactive agents, antibodies, affibodies, and nanobodies using hydrophobic ion parring is highly important for local drug release and delivery technologies using carbohydrate ester gel, oil or ECell compositions.

The ECell technology is not limited by the type of tissue, region or anatomical position that can be injected with ECell. For application in cancer therapy injected locations may include primary cancers, metastatic lesions and/or lymph nodes or lymphatic tissues to achieve cancer cell death, antigen release and immune activation. In the case of infectious disease, the injection(s) may be performed directly in or around the infected lesions or in the periphery of the region to be treated. It can further be primary solid cancers, peritumoral tissues, metastatic lymph nodes or metastatic lesions. For application in combination with surgical procedures, tissue grafts, catheters, stents, drains, devices, implants the injection(s) may be performed both in tissue and in the canals or cavities associated with these. When used in combination with cell-based therapies, the ECell may be injected both in the site (e.g., primary solid cancer, metastases, lymph node) where a specific immune activation (e.g., recruitment, engraftment, proliferation) of the cell product is to be achieved. The injection may also be injected at a site where the immune activation will take place e.g., sub cutaneous space, spleen, lymph node, intramuscular, intradermal, intraarticular, in bone and soft tissue. For supporting stem cell-based therapies the ECell may be injected adjacent to the injection of the stem cell product and/or in a tissue or organ of interest and here support engraftment, survival, proliferation, and polarization of the stem cell product. In case the ECell is intended to provide systemic controlled dosing of the bioactive(s) (e.g., hormone therapy, sustained release of anti-inflammatory drugs, immunosuppressive drugs, statins, metabolically active agents, vaccine antigens) injection may be flexible in terms of location.

ECell fiducial markers containing e.g., but not limited to radiographic contrast agents and fluorophores, may be injected as markers in cancerous tissue or lesions of interest. Such fiducial markers may aid surgeons in locating the tissue of interest using NIR fluorescence imaging, radiographic based imaging e.g., CT, x-ray, fluoroscopy forimage guided surgery. The fluorophores may further be released from the ECell, whereafter it may visualize lymphatic drainage, lymph vessels, lymphatic leakage and accumulate in draining lymph nodes.

Expanding ECell may serve as scaffolds for induction and / or guidance of tissue regeneration. Here the ECell may serve both as a basis for cell growth, polarization based on ECell composition and / or bioactive APIs released from the ECell.

Expanding ECell may be used as flexible void fillers that can be administered in any soft or bone tissue where a tissue defect or void needs to be filled for supporting healing, avoiding collapse or for cosmetical purposes. Additional use include temporary void filling during repetitive surgery. This ability of the expanding ECell to serve as a physical spacer allows its use as spacers for increasing distance, improving differentiation visually or on imaging modalities of tissues for increasing therapeutic safety, normal tissue sparring during radiation-, ablation-, and cryo-therapy or surgery or interventional procedures.

The physical properties of expanding ECells allow them to serve as physical palpatory markers that can be installed directly and using image guided technologies (e.g., MR, CT, fluoroscopy, ultrasound), endoscopic and or interventional radiology procedures. The ECell can, at the installation / injection site, provide a marker for identifying a lesion, region or section that could not be identified by visually or by physical structure. This may allow surgeons to accurately locate very small structure in soft tissue e.g., lung lesions, lung cancers, foreign bodies and secure that a precise intervention is performed with lesions identified and treated with minimal trauma.

Expanding ECell provide MR contrast and can therefore be used as MR fiducial markers. The ECell can therefore serve as a broadly applicable marker technology across multiple imaging technologies. This allows for the fusion of images between modalities to guide e.g., radiation therapy with minimal attenuation and dose perturbations. This allow for radiation planning based on radiography-based technologies while positioning and regions of interest may be based / identified based on MR characteristics.

Expanding ECells provide a unique biomaterial for controlling and optimizing drug release of incorporated therapeutic APIs. The surface properties and tissue interactions can be optimized for the specific API of interest to tailor release kinetics. The ability to enhance tissue compatibility and optimized drug release profiles is of great importance for next generation tissue regenerative scaffolds, surgical void fillers, spacers for radiotherapy and as fiducial markers in surgical guidance and radiotherapy.

### ECell Compositions

In one embodiment, an expanding ECell composition comprises a carbohydrate ester and a solvent.

In one embodiment, an expanding ECell composition comprises a carbohydrate ester, a co-solvent, and a solvent.

In one embodiment, an expanding ECell composition comprises a carbohydrate ester, an imaging agent, and a solvent.

In one embodiment, an expanding ECell composition comprises a carbohydrate ester, a polymer, and a solvent.

In one embodiment, an expanding ECell composition comprises a carbohydrate ester, a co-solvent, an imaging agent, and a solvent.

In one embodiment, an expanding ECell composition comprises a carbohydrate ester, a hydrophobic ion-pair, prodrug or native API, and a solvent.

In one embodiment, an expanding ECell composition comprises a carbohydrate ester, a co-solvent, a hydrophobic ion-pair, prodrug or native API, and a solvent.

In one embodiment, an expanding ECell composition comprises a carbohydrate ester, an Imaging agent, a hydrophobic ion-pair, prodrug or native API, and a solvent.

In one embodiment, an expanding ECell composition comprises a carbohydrate ester, an Imaging agent, a co-solvent, a hydrophobic ion-pair, prodrug or native API, and a solvent.

In one embodiment, an expanding ECell composition comprises a carbohydrate ester, a co-solvent, a polymer, an imaging agent, and a solvent.

In one embodiment, an expanding ECell composition comprises a carbohydrate ester, a polymer, a hydrophobic ion-pair, prodrug or native API, and a solvent.

In one embodiment, an expanding ECell composition comprises a carbohydrate ester, a co-solvent, a, polymer, a hydrophobic ion-pair, prodrug or native API, and a solvent.

In one embodiment, an expanding ECell composition comprises a carbohydrate ester, an Imaging agent, a polymer, a hydrophobic ion-pair, prodrug or native API, and a solvent.

In one embodiment, an expanding ECell composition comprises a carbohydrate ester, an Imaging agent, a co-solvent, a polymer, a hydrophobic ion-pair, prodrug or native API, and a solvent.

In one embodiment, carbohydrate esters of the ECell include but are not limited to Lactose octapropionate (LOP), lactose octaisobutyrate (LOIB), raffinose undecapropionate (RUP) Raffinose undecaisobutyrate (ROIB), Sucrose octapropionate (SOP), Sucrose acetate isobutyrate (SAIB), Sucrose octaisobutyrate (SOIB), Trehalose octapropionate (TOP), Trehalose octaisobutyrate (TOIB) and methoxy-LOIB (meLOIB), Lactose octabenzoate (LacBen), Sucrose octabenzoate (SuBen), Trehalose octabenzoate (TreBen), Raffenose undecabenzoate (RaBen) and the like.

In one embodiment, carbohydrate esters of the ECell include but are not limited to Lactose octapropionate (LOP), lactose octaisobutyrate (LOIB), raffinose undecapropionate (RUP) Raffinose undecaisobutyrate (ROIB), Sucrose octapropionate (SOP), Sucrose acetate isobutyrate (SAIB), Sucrose octaisobutyrate (SOIB), Trehalose octapropionate (TOP), Trehalose octaisobutyrate (TOIB) and methoxy-LOIB (meLOIB) and the like.

In one embodiment, co-solvents of the ECell include but are not limited to glycerol trivalerate, glycerol trihexanoate (GTH), glycerol trioctanoate (GTO), glycerol tridecanoate (GTD), ethyl octanoate, ethyl hexanoate, ethyl decanoate, Ethyl myristate, ethyl laurate, ethyl oleate, ethyl palmitate, ethyl myristate, corn oil, peanut oil, coconut oil, sesame oil, cinnamon oil, soybean oil, and poppyseed oil, or Lipiodol and aliphatic alkyl acyl esters and the like.

In one embodiment, solvents of the ECell include but are not limited to glycerin, diglycerin, polyglycerin, propylene glycol, polypropylene glycol, ethylene glycol, diethylene glycol, triethylene glycol, polyethylene glycol, polyethylene glycol (PEG), benzyl benzoate, triglycerides, acetone, benzyl alcohol, ethanol, ethyl lactate, propylene carbonate and Dimethyl Sulfoxide, 1-butanol, 2-butanol, Tert-butylmethyl ether, Ethyl ether, Ethyl formate, Heptane, 3-Methyl-1-butanol, Methylisobutylketone, 2-Methylisobutylketone, 2-Methyl-I-propanol, Pentane, 1-Pentanol, 1-Propanol, 2-Propanol and the like.

In one embodiment, polymers of the ECell include but are not limited to polymers from the class polyethyleneimine (PEI), polylysine (PLL), polyarginine (PAA), Chitosans, Cellulose, Poly(2-ethyl-2-oxazoline) (ULTROXA), Diethylaminoethyl-dextran (DEAE-dextran), dendritic polyamidoamine (PAMAM) and PDMAEMA [poly(N,N-dimethylaminoethyl methacrylate], glycol (PEG), polylactic acid (PLA), poly(lactic-co-glycolic acid) (PLGA) and Poly(N-isopropylacrylamide) (PNIPAM) and mixtures thereof.

In one embodiment, polymers of the ECell include but are not limited to Poly D,L lactide-co-glycolide 75:25, 75-115 KDa, Ester terminated (PLGA ester). Poly D,L lactide-co-glycolide 75:25, 4-15 KDa, acid terminated (PLGA-COOH short). Poly D,L lactide-co-glycolide 75:25, 10-18 KDa, acid terminated (PLGA-COOH medium). Poly-L-lactide (PLA), 10-18 KDa, ester terminated (PLA ester medium). PLA, 18-28 KDa, ether terminated (PLA ether medium). PLA, 2 KDa. PLA, 50 KDa. Alpha -tocopherol PEG-1000 succinate (acid terminated) (VitE-PEG1K). Polypropylene glycol) monobutyl ether (PPG), 2.5 KDa. PCL-PEG-PCL (1:1:1 KDa). Polycaprolactone diol (PCL Diol), 2 KDa. Polyethylene glycol methyl ether (PEG-OMe), 1KDa. PEG, 1.5 KDa. PEG(2000)-C18, 2 KDa. Polyethylene imine, branched (bPEI), 600 Da. bPEI, 1.2KDa. Cellulose acetate butyrate (CAB), 12KDa. CAB, 30 KDa. CAB, 70 KDa. Cellulose propionate (CP), 70 KDa. Celluose, acetate propionate (CAP), 75 KDa and mixtures thereof

Preferred polymers are classes include but are not limited to PEI, CAB, CB, CAP, PEG, PLA, PLGA and mixtures or block copolymers thereof.

In one embodiment, lipids and surfactant of the ECell include but are not limited to compounds with one or multiple ionizable groups belonging to the group of lipids comprising PTC, LPC, PA, LPA, PG, LPG, PI, LPI, PS, or LPS phospholipids, or SP or LSP all with mixed or non-mixed, saturated or unsaturated, sterol modified, fatty acid modified, or headgroup modified such as but not limited to PEGylated, fluorophore or chelator functionalized. Functionalized here includes but is not limited to deuteration, fluorescence, biotinylation, diphytanoylation, bromination, oxidization, diacetylenation, fluorination, and modifications with bioactive molecules.

or such as but not limited to PEGylated lipids are selected from the list of DLPE, DMPE, DPPE, DSPE, DOPE phospholipids PEGylated with either PEG350, PEG500, PEG750, PEG1k, PEG2k, PEG3k or PEG5k, sterols such as cholesterol PEGylated with either PEG350, PEG500, PEG600, PEG750, PEG1k, PEG2k, PEG3k or PEG5k or 1,2-Dimyristoyl-rac-glycero-3-methoxypolyethylene glycol-2000 (DMG-PEG2k), Distearoyl-rac-glycerol-PEG2K (DSG-PEG2k),

or such as Chems, DMG-PEG2K, Igepal Ca-720, PEG(20)-monooleyl ether, Monolaurin, Oleic acid, Lauric acid, PEG(10)-monolaurate, Tween 80, DSPE-PEG(2000)-RGD, DSPE-PEG(1000)-RGD, DSPE-PEG(2000), POPC, DOTAP, DC-Chol, DSPE-PEG(350Da) and the like.

In one embodiment, imaging agents of the ECell are selected from but not limited to CT imaging agent, radiographic imaging agent, fluorophores, chromophores, MRI imaging agents, radiotracers, or contrast carrying nanoparticles or any combination of these.

In one embodiment, imaging agents of the ECell are selected from radiographic contrast agents such as, but not limited to xSAIB, CLA-8, lipiodol.

In one embodiment, imaging agents of the ECell are selected from fluorophores, chromophores and coloured agents such as, but not limited to methylene blue (MB), indocyanine green (ICG), FD&D Blue (No. 1, No.2), D&C Blue No.4, FD&C Green No. 3, D&C Green (No. 5, No.6, No. 8), D&C Orange (No. 4, No.5, No.10, No. 11), FD&C Red (No. 3, No. 4), D&C Red No.6, D&C Red (No. 7, No. 17, No. 21, No. 22, No. 27, No. 28, No. 28, No. 30, No. 31, No. 33, No. 34, No. 36, No. 39), FD&C Red No.40, D&C Violet No.2, FD&C Yellow (No. 5, No.6), D&C Yellow (No. 7, No.8, No.10, No. 11), Carbazole violet, C.I. Vat Orange 1, Poly(hydroxyethyl methacrylate) -dye copolymers(3): one or more of (1) Reactive Black 5, Reactive Blue 21, reactive orange 78, Reactive yellow 15, reactive blue (No. 19, No. 4), C.I. reactive red 11, C.I. Reactive Yellow No. 86, C.I. Reactive blue 163, C.I. reactive red 180, D&C Blue No.9, D&C Green No.5, [Phthalocyaninato(2-)] copper, FD&C Blue No.2, D&C Blue No.6, D&C Green No.6, D&C Red No. 17, D&C Violet No.2, and D&C Yellow No.10.

In one embodiment, imaging agents of the ECell are selected from magnetic resonance contrast such as, but not limited to gadopentetic acid, gadolinium, gadoteridol, gadoxetic acid, superparamagnetic iron oxide and ferristene. gadoteric acid, gadopentetate, gadobenate, gadoxentate, magnevist, multihance, dotarem, and eovist.

### Preferred ECell compositions

In a preferred embodiment, an expandable ECell composition comprise the carbohydrate esters selected from but no limited to LOP, LOIB, meLOIB, SOP, SAIB, SOIB, SUPA, or mixtures thereof and a solvent.

In a preferred embodiment, an expandable ECell composition comprise a carbohydrate ester and a solvent selected from but no limited to DMSO, NMP, DMF, or mixtures thereof.

In a preferred embodiment, an expandable ECell composition comprise 50-90% carbohydrate ester and 10-50% solvent, such as 60-80% carbohydrate ester and 20-40% solvent, such as 70% carbohydrate ester and 30% solvent.

In a preferred embodiment, an expandable ECell composition comprise 50-90% carbohydrate ester, 0.5-15% polymer and 10-50% solvent, such as 60-80% carbohydrate ester, 1-10% polymer and 20-40% solvent, such as 65% carbohydrate ester, 5% polymer and 30% solvent.

In one embodiment, the polymer of the ECell composition governs the porosity if the water-channel network formed inside the expanded ECell.

In one embodiment, increased polymer content of the ECell composition results in reduced diameter of the formed water-channel network inside the expanded ECell.

In a preferred embodiment, an expandable ECell composition comprise LOIB:DMSO (60:40), LOIB:DMSO (70:30) or LOIB:DMSO (80:20).

In an embodiment, an expandable ECell composition comprise a carbohydrate ester, a solvent and polymers selected from but no limited to PEI, CAB, CB, CAP, PEG, PLA, PLGA.

In an embodiment, an expandable ECell composition comprise a carbohydrate ester, a solvent and 0.5-15% polymer, such as 1-10% polymer such as 2.5-7.5% polymer selected from but no limited to PEI, CAB, CB, CAP, PEG, PLA, PLGA.

In a preferred embodiment, an expandable ECell composition comprise LOIB:CAB:DMSO (55:5:40), LOIB:CAB:DMSO (65:5:30), LOIB:CAB: DMSO (75:5:20)

In a preferred embodiment, an expandable ECell composition comprise LOIB:CAB:DMSO (50:10:40), LOIB:CAB:DMSO (60:10:30), LOIB:CAB: DMSO (70:10:20)

In an embodiment, an expandable ECell comprise 0.01-10% lipid, such as 0.1-5% lipid, such as 1-2.5% lipid.

In one embodiment of the disclosure, expanding ECell comprises a carbohydrate ester and a solvent, such as but not limited to LOP, LOIB, MeLOIB, SAIB, SOIB, TOIB and DMSO

In one embodiment of the disclosure, expanding ECell comprises a carbohydrate ester, 1-20 w/w% polymer, such as 2.5-15 w/w% polymer such as 5 w/w% polymer and a solvent. The polymer may be 1 w/w%, 2 w/w%, 3 w/w%, 4 w/w%, 5 w/w%, 6 w/w%, 7 w/w%, 8 w/w%, 9, w/w%, or 10 w/w% of the ECell.

In one embodiment of the disclosure, expanding ECell comprises a carbohydrate ester, a polymer, a co-solvent and a solvent, such as but not limited to LOP, LOIB, MeLOIB, SAIB, SOIB, TOIB, a polymer such as but not limited to EC, CAP, CAB, CP, CB, a co-solvent such as but not limited to GTH, GTO, GTD, Ethyl-palmitate and a solvent such as but not limited to DMSO, NMP or DMF.

In one embodiment of the disclosure, expanding ECell comprises a carbohydrate ester, a polymer, and 1-20 w/w% co-solvent, such as 2.5-15 w/w% co-solvent such as 5 w/w% co-solvent and a solvent.

In one embodiment of the disclosure, expanding ECell comprises a carbohydrate ester, a polymer, a CT contrast agent, and a solvent, such as but not limited to LOP, LOIB, MeLOIB, SAIB, SOIB, TOIB, a polymer such as but not limited to EC, CAP, CAB, CP, CB, a CT contrast agent such as but not limited to xSAIB, CLA-8 and a solvent such as but not limited to DMSO.

In one embodiment of the disclosure, expanding ECell comprises a carbohydrate ester, a polymer, 1-40 w/w% CT contrast agent, such as 2.5-20 w/w% CT contrast agent such as 10w/w% CT contrast agent, and a solvent.

In one embodiment of the disclosure, expanding ECell comprises a carbohydrate ester, such as but not limited to LOP, LOIB, MeLOIB, SAIB, SOIB, TOIB, a lipid such as but not limited to cholesteryl hemisuccinate (CHEMS), 3β-[N-(N',N'-dimethylamino-ethane)-carbamoyl]cholesterol (DC-Chol), 1,2-distearoyl-sn-glycero-3-phosphoethanol-amine-N-[methoxy(polyethylene glycol)-2000] (DSPE-PEG2k), 1,2-dioleoyl-3-trimethylammonium-propane (DOTAP), DOPG, and a solvent such as but not limited to DMSO.

In one embodiment of the disclosure, expanding ECell comprises a carbohydrate ester, a polymer, 0.01-10 w/w% lipid, such as 0.1-5 w/w% lipid such as 1 w/w% lipid, and a solvent.

In one embodiment of the disclosure, expanding ECell comprises a carbohydrate ester, a functional lipid or polymer and a solvent, such as but not limited to LOP, LOIB, MeLOIB, SAIB, SOIB, TOIB, a lipid such as but not limited to DSPE-RGD, DSPE-PEG-RGD, DSPE-DOTA(Gd), DSPE-PEG-DOTA(Gd), RGD or DOTA(Gd) conjugated to PLA, PLGA, CAB and the like, and a solvent such as but not limited to DMSO.

In one embodiment of the disclosure, expanding ECell comprising any combination of a carbohydrate ester, a lipid, a polymer, a co-solvent, a CT contrast agent, a functional lipid or polymer, and a solvent.

### Physicochemical features of ECell

In one embodiment, a composition of ECell has reduced interfacial tension with water, which promotes formation of interface and ECell expansion.

In one embodiment, a composition of ECell comprising lipids and or polymers has reduced interfacial tension with water, which promotes formation of interface and ECell expansion.

In one embodiment, a composition of ECell has reduced shear viscosity, which promotes ECell expansion.

In one embodiment, a composition of ECell comprising additional solvents and or co-solvents has reduced shear viscosity, which promotes ECell expansion.

In one embodiment, a composition of ECell has improved mechanical integrity, which promotes ECell expansion and minimizes ECell collapse.

In one embodiment of the disclosure, a composition of ECell has altered surface charge, which reduces or increases surface pH.

In one embodiment, a composition of ECell comprising lipids and or polymers has altered surface charge, which reduces or increases surface pH.

In one embodiment of the disclosure, a composition of ECell forms microstructures upon expansion in water or tissue.

In one embodiment, a composition of ECell forms microstructures upon expansion in water or tissue, where the porosity and pore sizes are governed by ECell composition.

In one embodiment, a composition of ECell expands and forms a rigid and palpable depot.

In one embodiment, a composition of ECell expands and forms a depot comprising water pores and voids.

In one embodiment, a composition of ECell expands 100-1000% in volume, such as 50-500% in volume, such as 5-250% in volume, such as 100% in volume.

In one embodiment, a composition of ECell expands 100-10000% in interfacial area, such as 50-5000% in interfacial area, such as 5-2500% in volume, such as 500% in interfacial area.

In one embodiment of the disclosure, a ECell composition is injected into water or tissue, upon which water of interstitial fluids enters the ECell matrix and it expands in volume, whereafter it retains its volume for 1-10 days, or 2-5 days such as 3-4 days.

In one embodiment of the disclosure, a ECell composition is injected into water or tissue, upon which water of interstitial fluids enters the ECell matrix and it expands in volume, whereafter it collapses to its native volume.

In one embodiment, expanding ECell comprises a lipid that partitions onto the ECell interface which modifies the surface properties of ECell.

In one embodiment, expanding ECell comprises a polymer that is that partitions onto the ECell interface which modifies the surface of ECell

In one embodiment, ECell comprises a polymer or lipid that is presented on the ECell interface, that enhances interaction with tissues.

### ECell Function

In one embodiment of the disclosure, an expanding ECell take up water which provide enhanced MRI contrast.

In one embodiment of the disclosure, an expanding ECell take up water and form internal micro and macro structures in which water provide MRI contrast.

In one embodiment of the disclosure, an expanding ECell comprising lipidated and or polymer conjugated MRI contrast agents, such as but not limited to GdDOTA and Gd DTPA, take up water and form internal micro and macro structures with MRI contrast agent present at the ECell surfaces that provide enhanced MRI contrast.

In one embodiment of the disclosure, an expanding ECell comprising surface active polymer and or lipids has a modified surface that enhances wetting and interaction of the ECell with tissues to allow them to serve as both optimized surfaces supporting cell growth and also as scaffold that supports and direct cell ingrowth.

In one embodiment of the disclosure, an expanding ECell comprising cell stimulating polymer and or lipids has a modified surface that enhances interaction with Cells and with tissues.

In one embodiment the ECell comprises a fluorophore for injection or administration in a human or animal to allow for identification by fluorescence imaging.

In on embodiment the HIP formulated in ECell comprises a NIR fluorophore as example, but not limited to ICG, for NIR fluorescence imaging.

In one embodiment the HIP formulated in ECell comprises a NIR fluorophore for injection or administration in a tissue identified by diagnostic imaging e.g., CT, MR, PET, SPECT, ultrasound, radiography for subsequent identification by NIR fluorescence imaging.

In one embodiment the ECell composition is injected or administered using image guidance, including both not limited to CT-, ultrasound-, MR-, PET-, SPECT-, fluoroscopy-, endoscopy-guided imaging.

In one embodiment the injected or administered tissue comprises a cancer lesion e.g., primary malignant or benign tumor, a metastasis, a lymph node, a surgical bed for identification by NIR imaging in connection with treatment e.g., surgery.

In one embodiment the injected or administered site contains an object of interest, e.g., foreign body, polyp, tissue defect for identification by NIR imaging in connection with treatment e.g., surgery, including NIR guided and / or robotic surgery.

In one embodiment the injected or administered site is of interest for localization contains an object or tissue that is of interest to localize during therapeutic procedures, non-limiting examples include fragile tissue, tissue margins, nerves and vessels and ducts.

In one embodiment the injected or administered to form positional waypoints that can guide dissections and navigation during NIR guided surgery. In one embodiment multiple fluorescent markers are placed in the optimal path from the skin surface to e.g., a deeply located lymph, foreign body, cancerous lesion or vascular defect to guide and minimize trauma and improve accuracy during NIR guided surgery.

In one embodiment the injected tissue is a lung lesions of interest for accurate localization by NIR imaging or palpation during surgical procedures e.g., during thoracotomy, robotic surgery and video assisted thoracic surgery (VATS).

In one embodiment the injected or administered lesions is a primary, recurrent, metastatic cancerous lesion or a lymph node, metastatic lymph node or a surgical bed after cancer surgery.

In one embodiment, the ECell contains NIR fluorophores such as but not limited to ICG or MB, that upon injection or administration in primary, recurrent, metastatic cancerous lesion or a lymph node, metastatic lymph node or a surgical bed after cancer surgery release fluorophores that travel to draining lymph node e.g., sentinel lymph nodes.

In one embodiment the fluorophore releasing ECell composition is used to identify lymphatic disease, dysregulation, lymphangiogenesis, traumatic lesions in connection with therapy, including but not limited to surgery, diagnostic, or interventional radiological procedures.

### Space filling by ECell

In one embodiment the expanded ECell is injected into tissues to increase the distance between two or more tissues of interest. In one embodiment the expanded ECell is injected into the area between a tissue intended to be irradiated by internal or external radiation therapy. In one embodiment the expanded ECell is injected into the tissue space between the prostate and rectum to increase possible margins for radiation therapy and reduce radiation doses to the rectum.

In one embodiment, the CT contrast agent of ECell, such as but not limited to xSAIB, CLA-8 or lipiodol, attenuates the beam from internal or external beam radiotherapy and reduces the dose to the rectum or other tissues.

In one embodiment a volume of 0.1-100 mL ECell composition is injected into the tissue between the prostate and rectum.

### ECell degradation by hydrolysis

In one embodiment of the disclosure, an ECell composition expands in water or tissue upon which the hydrolytic degradation of the ECell excipients is accelerated by the enlarged ECell-Water interface.

In one embodiment of the disclosure, a ECell composition comprising anionic charged lipids or polymers expands in water or tissue upon which the hydrolytic degradation of the ECell excipients is catalysed by the reduced surface pH.

In one embodiment of the disclosure, a ECell composition comprising cationic charged lipids or polymers expands in water or tissue upon which the hydrolytic degradation of the ECell excipients is catalysed by the increased surface pH.

### Release of APIs from ECell

The gel forming eCell composition may further comprise pharmaceutical agents including prodrugs (in short "drugs"; broadly interpreted as agents which are able to modulate the biological processes of a human or animal). These drugs can be formulated as a single drug or as a combination of two or more in the ECell composition.

In one embodiment combinations APIs are formulated in a ECell composition.

In one embodiment combinations APIs and imaging agents are formulated in a ECell composition.

In one embodiment the suitable therapeutic agents can also be, without limitation, immunosuppressive agents, immune activating agents, immune modulating agents, cytokines, cytotoxic agents, nucleolytic compounds, radioactive isotopes, receptors, and pro-drug activating enzymes. The therapeutic agents of the present invention may be naturally secreted or produced by synthetic or recombinant methods, or any combination thereof.

A wide spectrum of therapeutic agents may be incorporated in the ECell composition and released at controlled rates for local, locoregional, and systemic therapeutic activity. Non-limiting examples of such therapeutic agents include antineoplastic agents, anti-infective agents, antimicrobials, antibiotics, local anaesthetics, anti-allergics, anti-anemics, beta-adrenergic blockers, calcium channel antagonists, anti-hypertensive agents, glucagon like peptides, insulins, antidepressants, angiogenic, anti-angiogenic, growth factors, anticonvulsants, antibacterial, anti-fungal, anti-viral, anti-rheumatics, anthelminithics, anti-parasitic agents, corticosteroids, hormones, hormone antagonists, immunomodulators, neurotransmitter antagonists, anti-diabetic agents, statins, lipid-lowering medications that reduce illness and mortality in those who are at high risk of cardiovascular disease (e.g., HMG-CoA reductase inhibitors), anti-epileptics, anti-haemorrhagic, anti-hypertonics, antiglaucoma agents, immunomodulatory cytokines, sedatives, chemokines, vitamins, toxins, narcotics, plant derived agents, wound repair agents, tissue repair agents, thermal therapy agents, and combinations thereof prepared and formulated in ECell compositions. The ECell composition may be injected, smeared, administered or installed in both soft tissues and bone, non-limiting examples includes sub-cutaneous, intramuscular, intradermal, intranodal, intraosseous, in organs, intratumoral, in infected tissues, in tissue defects, in scars, in wounds, smearing in surgical sites and surgical beds, in and around athroplastics, in and around implants, peritendinous, peri- and intraarticular, on mucosal and serosal surfaces, in wounds and on wound surfaces, in abscesses, phlegmons and body cavities e.g., intraperitoneal, intrathoracic, intravesical, intrauterine, intranasal, in sinuses, in the inner, middles and outer ear and on the skin and body surface.

In one embodiment, the active pharmaceutical ingredient is an innate immune activating compound which is a ligand for intracellular proteins and/or receptors; or a ligand for cell surface proteins and/or receptors.

The compounds can include the following drug as single therapeutic agents or as combinations of; immune activating compounds, including; Toll-like-receptor (TLR) family; TLR1, TLR2 TLR3, TLR4, TLR5, TLR6, TLR7, TLR8, TLR9, TLR10, TLR11, TLR12, TLR13. Examples of TLR agonists includes; polyinosinic:polycytidylic acid (poly I:C), Polyadenylic-polyuridylic acid (poly A:U), poly I:C-poly-L-lysine (poly-ICLC), poly-ICR, 3p'dsRNA, 3p'dsDNA, 2p'dsRNA, 2p'dsDNA, p'dsRNA, p'dsDNA, dsRNA, dsDNA, ssDNA, ssRNA, Imiquimod (R837), Resiquimod (R848), TMX-1 0 , TMX- 201, TMX-202, DSR6434, Gardiquimod, R850, R851, 852A, Isatoribine, S-2761 0 , 3M- 002 (CL075), 3M-003, 3M-005, 3M-006, 3M-007, 3M-012 , 3M-13 , 3M-031 ,3M-854, CL075, CL097, CL264, IC-31 , Loxoribine and other imidazoquinolines, ssPolyU, ANA975, SM360320, SM3244405, RWJ 21757 , R 1354, single stranded or double stranded RNA, ORN 02 (5'- UUAUUAUUAUUAUUAUUAUU-3'), ORN 06 5'-UUGUUGUUGUUGUUGUUGUU-3', CpG-ODN DSLIM, AVE 0675, CpG B oligodeoxynucleotide, 1018, AZD 1419, ODN 1982, CpG B ODN 2006, IMO 2125, CpG A ODN 221 6 , CpG A ODN 2336, CpG 2395, CpG ODN 7909, CpG 10101, CpG ODN AVE0675, CpG ODN HYB2093, CpG ODN HYB2055, CpG-ODN IMO-21 25, CpG C ODN M362, Tolamba (Amb a 1 ragweed allergen with covalently linked CpG B class ODN 10 18), Heplisav, 10 181SS, IM02055, IRS954, (flagellin, muramyl dipeptide, saponins such as QS21 , Leishmania elongation factor, SB-AS4, threonyl-muramyl dipeptide, L 18-MDP, mifamurtid, A83-01 , A4476, GW788383, LY364947, R26871 2, RepSox, SB431542, SB505124, SB525334, SD208, FAK inhibitor 14, PF431 396, PF573228, Y 11 and OM- 174, nickel and the like, but not limited to those.

Inhibitors of Toll-like-receptors (TLR) include AT791, E6446, COV08-0064, COV08-0055 and COV08-0064 and the like, but not limited to those.

STING agonists compounds include ADU-S100, C11, Cridanimod, MK-1454, PO-424, H-151, C-176, diABZI compound 3 (2138299-34-8 and 2138299-33-7), diABZI compound2 (2138300-40-8), 3'3'-cGAMP, 2'3'-cGAMP, ChX0306710, ML RR-S2 CDA ammonium salt, ML RR-S2 CDA, sodium Cridanimod, G10 and the like, but not limited to those.

STING inhibitors, compounds include H-151, C-176, C-178, STING inhibitor 18, Astin C and the like, but not limited to those. RIG-1-like receptor agonists, examples include KIN1148, KIN131A, KIN126X, KIN150X, KIN1000, KIN1408, SLR14, MK4621, RGT100, KIN1400 and the like, but not limited to those.

In yet another embodiment the active pharmaceutical ingredient is an immune activating/modulating drug which is a ligand for intracellular proteins and/or receptors; or a ligand for cell surface proteins and/or receptors. The drug may serve as a single therapeutic API or serve as part of a combination of APIs ECell composition.

The compounds include Tumor necrosis factor alpha (TNF-a) agonists, TNF-a receptor blocking molecules, Interferon (IFN) agonists, examples include; RO8191, RO8181 and the like, but not limited to those. T-Cell Immunoreceptor with Ig and ITIM Domains (TIGIT) agonists.

In yet another embodiment the active pharmaceutical ingredient is a transcription factor modulator, post translational enzyme modulator, immune cell polarizing and immune modulating drug which is a ligand for intracellular proteins and/or receptors; or a ligand for cell surface proteins and/or receptors. The drug may serve as a single therapeutic API or serve as part of a combination of APIs ECell composition.

The compounds include, as single APIs or in combination: including transforming growth factor beta signaling inhibitors (TGF-β inhibitors) and transforming growth factor beta receptor inhibitors (TGF-β receptor inhibitors) and including ALK signaling inhibitors and Smad singnaling inhibitors; RepSox, Galunisertib (LY2157299), LY550410, LY580276, TEW-7197, SB 505124, SB 431542, A 83-01, SD 208, LY 364947, SB 525334, SB 505124, D 4476, GW 788388, R 268712, IN 1130, SM 16, A 77-01, SB 431542, LY 364947, R268712, ITD 1, SIS3, LY2109761, LY 3200882, Pirfenidone, LDN-193189, LDN-193189 HCL, K02288, LDN-214117, TEW-7179, DMH1, LDN-212854, ML347, sotirimod, Kartogenin, Hespertin, Alantolacton, Suramin sodium, BML-275 dihydrochloride, BML-275, ALK2-IN-1, Vactosertib, LSKL, Inhibitor of Thrombospondin (TSP-1), SJ000291942, K02288, LDN-212854, ML347, SM 16, TGFβRI-IN-1 and the like, but not limited to those. The compounds further include as single APIs or in combination: including tyrosine phosphatase SHP2 inhibitors; SHP099, PC61275 and the like, but not limited to those. The compounds further include, as single APIs or in combination: including Glycogen Synthase Kinase 3 (GSK-3) inhibitors; SB415286, Lithium, Valproic acid, lodotubercidin, Naproxen, Cromolyn, Famotidin, Curcumin, Olanzapine, Pyrimidine derivater, ARA014418, CHIR 99021, CHIR 99021 trihydrochloride, SB 216763, BIO, Kenpaullone, , 10Z-Hymenialdisine, SB 415286, Indirubin, Indirubin-3prime-monoxime-5-sulphonic Acid, Indirubin-3'-oxime, 5-lodo-indirubin-3prime-monoxime, Indirubin-5-sulfonic acid sodium salt, NSC 693868, TWS 119, TWS 119 ditrifluoroacetate, TCS 2002, MeBIO, BIO, BIO-acetoxime, Bisindolylmaleimide I, Bisindolylmaleimide I hydrochloride, 3F8, TCS 21311, TCS2002, TC-G 24, A 1070722, Lithium Carbonate, TDZD 8, AlsterPaullone, CHIR 99021, CHIR 99021 trihydrochloride, CHIR 98014, tideglusib, AZD2858, AZD1080, LY2090314, 2-D08, IM-12, 1-Azakenpaullone, Bikinin, L807mts, Staurosporine, KT5720, GSK-3 inhibitor IX, Ro 31-8220, CID 755673, GSK-3 Inhibitor XVI, 10Z-Hymenialdisine, GSK-3beta Inhibitor VI, Manzamine A, GSK-3 Inhibitor X, GSK-3 Inhibitor XV, GSK-3beta Inhibitor I, GSK-3beta Inhibitor VII, GSK-3beta Inhibitor II, GSK-3beta Inhibitor VIII, Hymenidin, Bisindolylmaleimide X hydrochloride, 3F8, Isogranulatimide, CR8, L779,450, Aloisine A, GSK-3beta Inhibitor XI, Ro-31-8220, Ro 31-8220 methanesulfonate, Enzastaurin, PHA 767491 hydrochloride, AR-AO 14418-d3, Indole-3-acetamide, Hymenialdisine Analogue 1, CP21R7, Necrosulfonamide, IM12, Leucettine, LY-2090314, Tideglusib and the like, but not limited to those.

The compounds include, as single APIs or in combination: including signal transducer and activator of transcription (STAT) inhibitors; Stattic, Cucurbitacin I, Niclosamide, NSC 74859, SD 1008, Cryptotanshinone, Napabucasin, Galiellalactone, S3I-201, Nifuroxazide, SH-4-54, AS1517499, Artesunate, BP-1-102, SH5-07, STA-21, HJC0152, APTSTAT3-9R, C188-9- HO-3867, RSVA 405, and the like, but not limited to those.

The compounds include, as single APIs or in combination: including Wnt/β-catenin signaling targeting therapy; WAY-316606, IWP, IWP-L6, LGK974, WNT-C59, ETC-159, Ant1.4Br/Ant 1.4CI, (hetero)arylpyrimidines, Niclosamide, apicularen, bafilomycin, XAV939, IWR, G007-LK, G244-LM, IQ1, pyrvinium, QS11, NSC668036, SB-216763, CHIR99021, BIO(6-bromoindirubin-3'-oxime), LY2090314, DCA, 2-amino-4-[3,4-(methylenedioxy)benzyl-amino]-6-(3-methoxyphenyl)pyrimidine, 2,4-diamino-quinazoline, Quercetin, ICG-001, PKF115-584, BC2059, Shizokaol D, 3289-8625, J01-017a, Derricin, Derricidin, Carnosic acid, Windorphen, TMEM88, KY-02061, KY-02327, BMD4702, DK-520, Sulindac, ICG-001, PNU-74654, E7449 and the like, but not limited to those.

The compounds include, as single APIs or in combination: including Phosphoinositide 3-kinases (PI3Ks) inhibitors; Wortmannin, LY294002, PX-866, XL-147, SF1126, GDC0941, PI-103, NCPBEZ235, XL765, GSK2126458, PKI-587, MK2206, PF-04691502, IC187114, CAL-101, Rapamycin, Torin1, AZD-8055, OSI-027, GDC-0032, NVP-BKM120, ZSTK474, BAY 80-6946, BYL719, GDC0326, SAR260301, GDC0980, GNE-317, GNE-477, PF-0491502, PKI-179, PKI-587, INK-1117, CH5132799, AZD8186, IPI-145 Buparlisib, Idelasilib, IPI-549, Pictillisib and the like, but not limited to those.

The compounds include, as single APIs or in combination: including tyrosine kinase receptor inibitors, including but not limited to c-KIT (SCFR), PDGFR, FGFR, VEGFR, and FA; Axitinib, Dasatinib, TKI-258, ST1571, AMG-706, GW786034 HCL, Sunitinib malate, AB1010, PTK787, XL184, BMS-907351, AV-951, OSI-930, MP-470, Ki8751, Telatinib, Pazopanib, TKI-258, CHIR-258, Thyrpostin AG 1296, PKC-412, ISCK03, AP 24534, KRN633, SU6668, Sorafinib, ABT-869, Divitinib, Pazopanib, 4,4prime-Bis(4-aminophenoxy)biphenyl, ISCK03, Tandutinib, SU1652, AGL 2043, PLX9486, BLU-285, AZD2932, PLX3397, MGCD516 and the like, but not limited to those.

The compounds include as single APIs or in combination: including tyrosine kinase receptor agonists, including but not limited to c-KIT (SCFR), PDGFR, FGFR, VEGFR, and FA; DRM/gremlin, and the like, but not limited to those.

The compounds include as single APIs or in combination: including Bruton's Tyrosine Kinase (BTK) and Interleukin-2-Inducible Kinase (ITK) Inhibitors: Ibrutinib, Acalabrutinib and the like, but not limited to those.

The compounds include, as single APIs or in combination: including Hedgehog pathway modulators, including Smoothened (Smo), Sonic hedgehog pro-tein (Shh), and Gli1 inhibitors and agonists: SAG, compound 10c, Mercaptobenzoimidazole, cyclopamine, HhAntag, IPI926, GDC-0449, Cur61414, GANT61, IPI-269609 BMS-833923, PF-04449913, HPI1, HPI2, HPI3, HPI4, JK-184, NMDA298-1, robotnikinin, Purmorphamine, 22(S)-hydroxycholesterol, 20(S)-hydroxycholesterol, GANT58, GANT61 and the like, but not limited to those.

The compounds include as single APIs or in combination: including Mammalian target of rapamycin (mTOR) inhibitors; Temsirolimus, Everolimus, Ridaforolimus (AP23573 and MK-8669), Dactolisib, Omipalisib, Niclosamide and the like, but not limited to those.

The compounds include as single APIs or in combination: including C-Myc inhibitors; JQ1, I-BET151, 10058-F4, 10074-G5, 7594-0035, KJ Pyr9, ML327, Mycro3, IZCZ-3, KSI-3716, 403811-55-2, Apto-253 and the like, but not limited to those.

The compounds include, as single APIs or in combination: including c-Met inhibitors; PHA665752, INC280, SU 11274, AMG208, Golvatinib, PF 02341066, LY 2801653, ARQ 197, PF 04217903, Fortinib, Crizotinib, PHA-665752, SAR125844, Pulsatilla saponin D, SGX-523, BMS-777607, JNJ-38877605, MGCD-265, INCB28060, BMS-794833, BMS-754807, AMG-208, MK-208, MK-2461, E7050, AMG-458, NVP-BVU972, EMD 1214063, AMG-337, LY12801653, S49076, Norcantharidin, NPS-1034, AZD6094 and the like, but not limited to those.

The compounds include, as single APIs or in combination: including BRAF inhibitors; BMS-9086662, LGX818, PLX3603, RAF265, RO5185426, GSK2118436, PLX4032, Sorafenib, PLX-4720, GDC-0879, AZ304, PLX-8394, LXH254, Dabrafenib mesylate, RAF265, AZ 628, NVP-BHG712, SB590885, ZM 336372, GW5074, TAK-632, CEP-32496, LGX818, BAW2881, CCT196969, RAF709, BGB-283, PLX7904, LY3009120, RO5126766, MLN2480, Regorafenib and the like, but not limited to those.

The compounds include as single APIs or in combination: including MEK inhibitors; BIX02188, PD0325901, U0126-ETOH, GSK1120212, AZD6244, PD0325901, CI-1040, PD98059, AS-703026, TAK-733, AZD8330, MEK162, PD318088, Honokoil, SL-327, RDEA119, Myricetin, BI-847325, GDC-0973, GDC-0623, APS-2-79 and the like, but not limited to those.

In yet another embodiment the active pharmaceutical ingredient is an epigenetic modulating drug, which is a ligand for intracellular proteins and/or receptors; or a ligand for cell surface proteins and/or receptors. The drug may serve as a single therapeutic API or serve as part of a combination of APIs ECell composition.

The APIs include, as single agents or in combination: including DNA methyltransferase inhibitors (DNMTi), histone deacetylase inhibitors (HDACi), histone methyltransferases inhibitors (HMTi), histone acetyltranferases inhibitors (HATi), histone demethylases inhibitors (HDMi), proteins binding to methylated and acetylated histones inhibitors (PAHi and PMHi); decitabine, azacitidine, EGCG, zebularine, hydralazine, procainamide, Vorinostat, givinostat, panobinostat, TSA, belinostat, entinostat, CG-1521, romidepsin, ITF-A, ITF-B, valproic acid, OSU-HDAC-44, HC-toxin, magnesium valproate, plitidepsin, tasquinimod, sodium butyrate, mocetinostat, carbamazepine, SB939, CHR-2845, CHR-3996, JNJ-26481585, sodium phenylbutyrate, pivanex, resveratrol, abexinostat, resminostat, dacinostat, droxinostat, Pargyline, clorgyline, bizine, GSK2879552, GSK-J4, KDM5-C70, JIB-04, tranylcypromine, EPZ-6438, GSK126, CPI360, DZNep, GSK343, EI1, BIX-01294, UNC0638, EPZ004777, UNC0224, JQ1, CPI203, RVX-208, I-BET151, I-BET762, i-BET-726, UNC669, UNC1215 and the like, but not limited to those.

In yet another embodiment the API is a lymphocyte activating and modulating drugs which is a ligand for intracellular proteins and/or receptors; or a ligand for cell surface proteins and/or receptors. The drug may serve as a single therapeutic API or serve as part of a combination of APIs ECell composition. The compounds include, as single APIs or in combination: Programmed death-ligand 1 and 2 (PD-L1 and PD-L2) inhibitors, programmed cell death protein 1 (PD1) inihibitors and PD-L1/PD-L1 checkpoint inhibitors; BMS-8, BMS-37, BMS-57, BMS-71, BMS-105, BMS-202, BMS-230, BMS-242, BMS-1001, BMS-1166, BMS-1165, BMS-2007, BMS-1016, BMS-40210, BMS-8, CA-170, CA-327, SB415286, INCB086550, INCMGA00012, CX072, CCX4503, MAX-10129, vorinostat, panobinostat, azacitidine, decitabine, entitostat, JQ1, I-BET151, GSK503, WO2015/034820, WO2015/033301 and the like, but not limited to those.

The compounds include as single APIs or in combination: including Anti-cytotoxic T-lymphocyte-associated protein-4 (anti-CTLA-4) inhibitors/modulators; Compounds "8 and 9", ACY- 241 and the like, but not limited to those.

The compounds include as single APIs or in combination: including T-cell immunoglobulin and mucin domain-3 (TIM-3) inhibitors; TSR-022, Sym023, ATIK2a, CA-327 and the like, but not limited to those.

The compounds include as single APIs or in combination: including Lymphocyte activating gene 3 (LAG3) inhibitors; IMP32, BMS986016 and the like, but not limited to those.

The compounds include as single APIs or in combination: including OX40 (CD124) activators/modulators, examples include DB36, DB71, DB15, CVN, MGCD0103, SNDX-275 and the like, but not limited to those. Tumor necrosis factor receptor super family (TNFRSF) agonist (e.g. OX40 (CD124) agonists, CD40 agonists, CD27 agonists, 4-1BB (CD137) agonists, Glucocorticoid-induced tumor necrosis factor receptor-related protein (GITR, CD357) agonists, inducible T-cell costimulator (ICOS) agonists and tumor necrosis factor related apoptosis-inducing ligand (TRAIL, CD253, TNFSF10) receptor agonist, examples include; Acrp30-CD40L, DB36, DB71, DB15, CVN, MGCD0103, SNDX-275, dulanermin, and the like, but not limited to those.

In yet another embodiment the APIs is an immunemodulating and/or inflammation modifying enzyme inhibitor or activator, a cellular receptor of metabolites, which is a ligand for intracellular proteins and/or receptors; or a ligand for cell surface proteins and/or receptors. The drug may serve as a single therapeutic API or serve as part of a combination of APIs ECell composition. The compounds include as single agents or in combination: including Indoleamine 2,3-dioxygenase-1 (IDO1) inhibitors; methyl-tryptophan, D-1MT, L-1MT, tryptophan, epacadostat, GDC-0919, Indoximod, EOS-200271, NLG919, BMS-986205 and the like, but not limited to those.

The compounds include as single APIs or in combination: including Arginase inhibitors; INCB001158 and the like, but not limited to those.

The compounds include as single APIs or in combination: including Adenosine receptor inhibitors; caffeine, AZD4635, Vipedenant, Preladenant, CPI-444 and the like, but not limited to those.

The compounds include, as single APIs or in combination: including cyclooxygenase (COX) 1 and/or 2 inhibitors; Cyclooxygenase (COX) 1 and/or 2 inhibitors; celecoxib, rofecoxib, DuP-697, valdecoxib, etoricoxib, lumiracoxib, indomethacin, 6-methoxy-α-methyl-2-naphthylacetic acid, meclofenamic acid, diclofenac, flufenamic acid, niflumic acid, mefenamic acid, sulindac, tolmetin, suprofen , ketorolac, flurbiprofen, ibuprofen, aceloferac, alcofenac, amfenac, benoxaprofen, bromfenac, carprofen, clidanac, diflunisal, Efenamine, Etodol, fenbufen, fenclofenac, fenclorac, fenoprofen, piroxicam, fleclozic, indoprofen, isofezolac, ketoprofen, loxoprofen, meclofenamate, naproxen, Organoxin , pirprofen, pranoprofen, tolfenamic acid, zaltoprofen, zomepirac and the like, but not limited to those.

The compounds include as single agents or in combination hypoxia inducible factor 1 (HIF-1) inhibitors and hypoxia inducible factor 2 (HIF-2) inhibitors; Chemotomin, Chrysin, Dimethyl-bisphenol, Echinomycin, PX 12, YC-1, Vitexin and the like, but not limited to those.

In yet another embodiment the APIs is a chemokine receptor signal and chemokine receptor modifying drug which is a ligand for intracellular proteins and/or receptors; or a ligand for cell surface proteins and/or receptors. The drug may serve as a single therapeutic API or serve as part of a combination of APIs ECell composition. The compounds include, as single APIs or in combination: chemokine receptor signaling modifiers; AZD5069, SX-682, AMD3100, X4P-001, PF-4136309, Maraviroc, LMT-28, madindoline-5 (MDL-5), MDL-16 and MDL-101, SPD-304, C87 ((E)-4-(2-(4-chloro-3-nitrophenyl)), tamatinib fodium (R788), ZINC09609430, ZINC49467549, ZINC13113075, ZINC39907639, ZINC25251930, ZINC02968981, ZINC09544246, ZINC58047088, ZINC72021182, ZINC08704414, ZINC05462670, ZINC35681945, ZINC23553920, ZINC05328058, and ZINC17206695 and the like, but not limited to those.

In yet another embodiment the APIs is a cell cycle checkpoint inhibitor, activator or modulator which is a ligand for intracellular proteins and/or receptors; or a ligand for cell surface proteins and/or receptors. The drug may serve as a single therapeutic API or serve as part of a combination of APIs ECell composition. The compounds include as single APIs or in combination Checkpoint kinase 1 and/or 2 (CHK1/2) inhibitors; AZD7762, LY 2603618, CCT 241533, NSC 109555, PD 407824, PF 477736, SB 218078, UCN-01, CHIR-124, SAR-020106, CCT244747, SCH900776, V158411, TCS 2312, Hymenialdisine, ABI, NSC1095555, PV1019, VRX0466617, CCT241533, Aminopyridine 7 and the like, but not limited to those.

The compounds further include as single APIs or in combination Ataxia telangiectasia mutated (ATM) inhibitors; Wortmannin, Caffeine, KU55933, KU55403, KU60019, CP-466722, CGK733, NVP-BEZ235, Torin-2, Methoxyquinazoline 1, Fluoroquinoline 2, SJ573017 and the like, but not limited to those.

The compounds further include as single APIs or in combination WEE1 inhibitors; PD0166285, PD407824, WEE1 inhibitor II, MK1775, Pyrimidopyrimidinone 8 and the like, but not limited to those.

The compounds include as single APIs or in combination Ataxia telangiectasia and Rad3-related (ATR) inhibitors; Schisandrin B, ETP-46464, NU6027, VE-821, VE-822, AZ20, AZD6738 and the like, but not limited to those.

In yet another embodiment the API is a cell death inducing chemotherapeutic and immunogenic cell death inducing chemotherapeutics which is a ligand for intracellular proteins and/or receptors; or a ligand for cell surface proteins and/or receptors. The drug may serve as a single therapeutic API or serve as part of a combination of APIs ECell composition. The compounds include as single APIs or in combination irinotecan hydrochloride, nogitecan hydrochloride, exatecan, RFS-2000, lurtotecan, BNP-1350, Bay-383441, PNU-166148, IDEC-132, BN-80915, DB-38, DB-81, DB-90, DB-91, CKD-620, T-0128, ST-1480, ST-1481, DRF-1042 and DE-310, taxane derivatives such as docetaxel hydrate, IND-5109, BMS-184476, BMS-188797, T-3782, TAX-1011, SB-RA-31012, SBT-1514 and DJ-927, ifosfamide, nimustine hydrochloride, carboquone, cyclophosphamide, dacarbazine, thiotepa, busulfan, melphalan, ranimustine, estramustine phosphate sodium, 6-mercaptopurine riboside, enocitabine, gemcitabine hydrochloride, carmofur, cytarabine, cytarabine ocphosphate, tegafur, doxifluridine, hydroxycarbamide, fluorouracil, methotrexate, mercaptopurine, fludarabine phosphate, actinomycin D, aclarubicin hydrochloride, idarubicin hydrochloride, epirubicin hydrochloride, daunorubicin hydrochloride, pirarubicin hydrochloride, bleomycin hydrochloride, zinostatin stimalamer, neocarzinostatin, mytomycin C, bleomycin sulfate, peplomycin sulfate, vinorelbine tartrate, vincristine sulfate, vindesine sulfate, vinblastine sulfate, amrubicin hydrochloride, gefitinib, exemestan, capecitabine, TNP-470, TAK-165, KW-2401, KW-2170, KW-2871, KT-5555, KT-8391, TZT-1027, S-3304, CS-682, YM-511, YM-598, TAT-59, TAS-101, TAS-102, TA-106, FK-228, FK-317, E7070, E7389, KRN-700, KRN-5500, J-107088, HMN-214, SM-11355, ZD-0473, magnesium 5,10,15,20-tetrakis(4-sulphophenyl)-porphine dodecahydrate, PYROA protein (Emericella nidulans), photosan III, lomefloxacin, cyamemazine, tiaprofenic acid, doxorubicin, mitomycin, paclitaxel, nitrogen mustards, etoposide, camptothecin, 5-fluorouracil, nicotinamide, metronidazole, doxorubicine, Lomeguatrib, Temozolomide, tamoxifen, bleomycin, 5-fluorouracil, cyclophosphamide, methotrexate, gemcitabine, oxaliplatin, cisplatin, carboplatin, camptothecin, CPT-11 (SN-38), Etanidazole, Nimorazole, Mitomycin C, Tirapazamine, procaine, lidocaine, chlorpromazine, Fluordeoxyuridine, bromodeoxyuridine, iododeoxyuridine, hydroxyurea, fludarabine, Texaphyrins (motexafin gadolinium), N-ethylmalemide, paclitaxel, docetaxel, irinotecan, Mechtorethamine, Cyclophosphamide, Ifosfamide, Melphalan, Chlorambucil, Procarbazine (N-methylhydrazine, MIH), Busulfan, Camustine (BCNU), Streptozocin (streptozotocin), Bendamustine, Dacarbazine (DTIC; dimethyttriazenol midazole carboxamide), Temozolomide, Cisplatin, carboplatin, oxaliplatin, Methotrexate (Amethopterin), Pemetrexed, Fluorouracil (5-fluorouracil; 5-FU), capecitabine, Cytarabine (cytosine arabinoside), Gemcitabine, 5-aza-cytidine, Deoxy-5-aza-cytidine, Mercaptoptirine (6-mercaptopurine; 6-MP), Pentostatin (2'-deoxycoformycin), camptothecin, SN-38 (CPT-11), Rudarabine, Clofarabine, Nelarabine, Tirapazamine, Vinblastine, Vinorelbine, Vincristine, Paclitaxel, docetaxel, Etoposide, Teniposide, Topotecan, Irinotecan, Dactinomycin, (actinomycin D). Daunorubicin (daunomycin, rubidomycin), Doxorubicin, Yondelis, Mitoxantrone, Bleomycin, Mitomycin C, L-Asparaginase, Mitotane (o.pDDD) Prednisone, Hydroxyprogesterone caproate, medroxyprogesterone acetate, megestrol acetate, Dietyhlstilbestrol, ethinyl estradiol, Tamoxifen, toremifene, Anastrozole, Gefitinib, letrozole, exemestane, Testosterone propionate, fluoxymesterone, Flutamide, casodex, Leuprolide. Hydroxyurea, Tretinoin, arsenic trioxide, Vorinostat, Imatinib, Dasatinib, nilotinib, Gefrtinib, ertoinib, Sorafenib, Sunitinib, Lapatinib, Bortezomib, Thalidomide, Lenaiidomide, Temsiroiimus, Everolimus, and the like, but not limited to those.

In yet another embodiment the API is an osteoinductive or osteogenic drug which is a ligand for intracellular proteins and/or receptors; or a ligand for cell surface proteins and/or receptors. The drug may serve as a single therapeutic API or serve as part of a combination of APIs ECell composition.

The compounds include as single APIs or in combination: including tiludronate, alendronate, pamidronate, risedronate, ibandronate, zoledronic acid, etidronate, BPH-675, BPH-715, bone morphogenic protein (BMP e.g. BMP-2, BMP-7 and osteogenic protein 1 (OP-1)) and the like, but not limited to those.

In yet another embodiment the API is a 3-hydroxy-3-methylglutaryl coenzyme A (HMG-CoA) reductase suppressants (statin) which is a ligand for intracellular proteins and/or receptors; or a ligand for cell surface proteins and/or receptors. The drug may serve as a single therapeutic API or serve as part of a combination of APIs ECell composition. The compounds include as single APIs or in combination compactin, simvastin and the like, but not limited to those.

In yet another embodiment the active pharmaceutical ingredient is an inducer of osteogenic commitment of stem cells, including BMP, ERK, WNT, AMPK signalling pathways modulators which is a ligand for intracellular proteins and/or receptors; or a ligand for cell surface proteins and/or receptors. The drug may serve as a single therapeutic API or serve as part of a combination of APIs ECell composition. The compounds include, as single APIs or in combination: including Purmorphamine, Mevinolin, Resveratrol, Icariin, Metformin and the like, but not limited to those.

In yet another embodiment the active pharmaceutical ingredient is an inducer of chondrogenic commitment of stem cells modulators which is a ligand for intracellular proteins and/or receptors; or a ligand for cell surface proteins and/or receptors. The drug may serve as a single therapeutic API or serve as part of a combination of APIs ECell composition. The compounds include as single APIs or in combination: including Kartogenin, TD-198.946, Prostaglandin E2 and the like, but not limited to those.

In yet another embodiment the active pharmaceutical ingredient is an inhibitor of matrix metalloproteinases (MMPs) which is a ligand for intracellular proteins and/or receptors; or a ligand for cell surface proteins and/or receptors. The drug may serve as a single therapeutic API or serve as part of a combination of APIs ECell composition. The compounds include as single APIs or in combination: including ab142180, ab145190, ab141579, Actinonin, SB-3CT, A4336, Marimastat, TAPI-0, TAPI-1, TAPI-2, Luteolin, Collagenase Inhibitor I, GM 6001, PD166793, Ro 32-3555, CP 471474, UK 356618, NNGH, ND-322, ND-336, RXP470.1 and the like, but not limited to those.

In yet another embodiment the API is an interleukin (IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36 and IL-37) modulator which is a ligand for intracellular proteins and/or receptors; or a ligand for cell surface proteins and/or receptors. The drug may serve as a single therapeutic API or serve as part of a combination of APIs ECell composition The compounds include, as single APIs or in combination: including LMT-28, rapamycin, FK506, A-552, tofacitinib, GSK650394, LTV-1, apilimod, HG-9-91-01, GNE-7915, GSK-J4, I-BET762, SR1001, digoxin, VX-765, ONX0914, SP4206, PD225002, SB265610, PD0210293, PD0220245, NSC201631, NSC61610, LMT-28, rilonacept, anakinra, and NSC80734 and the like, but not limited to those.

In yet another embodiment the composition could further comprise a formulation where one or more of the active pharmaceutical ingredients induces an anti-bacterial effect, or an anti-infectious effect in a human or animal body. The drug may serve as a single therapeutic API or serve as part of a combination of APIs ECell composition. The compounds include, as single APIs or in combination: Aminoglycosides, Amikacin, Gentamicin, Kanamycin, Neomycin, Netilmicin, Tobramycin, Paromomycin, Streptomycin, Spectinomycin(Bs), Ansamycins, Geldanamycin, Herbimycin, Rifaximin, Carbacephem, Loracarbef, Carbapenems, Ertapenem, Doripenem, Meropenem, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefdinir, Cefditoren, Cefotaxime, Cefpodoxime, Ceftibuten, Ceftizoxime, Moxalactam, Ceftriaxone, Cefepime, Ceftaroline fosamil, Ceftobiprole, Glycopeptides, Teicoplanin, Vancomycin, Telavancin, Dalbavancin, Oritavancin, Lincosamides(Bs), Clindamycin, Lincomycin, Lipopeptide, Daptomycin, Macrolides(Bs), Azithromycin, Clarithromycin, Erythromycin, Roxithromycin, Telithromycin, Spiramycin, Fidaxomicin, Monobactams, Aztreonam, Nitrofurans, Furazolidone, Nitrofurantoin(Bs), Oxazolidinones(Bs), Linezolid, Posizolid, Radezolid, Torezolid, Penicillins, Amoxicillin, Ampicillin, Azlocillin, Dicloxacillin, Flucloxacillin, Mezlocillin, Methicillin, Nafcillin, Oxacillin, Penicillin G, Penicillin V, Piperacillin, Temocillin, Ticarcillin, Amoxicillin/- clavulanate, Ampicillin/sulbactam, Piperacillin/tazobactam, Ticarcillin/clavulanate, Polypeptides, Bacitracin, Colistin, Polymyxin B, Polymyxin E, Ciprofloxacin, Enoxacin, Gatifloxacin, Gemifloxacin, Levofloxacin, Lomefloxacin, Enrofloxacin, Moxifloxacin, Nadifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide (archaic), Sulfasalazine, Sulfisoxazole, Sulfonamidochrysoidine (archaic), Tetracyclines(Bs), Demeclocycline, Doxycycline, Metacycline, Minocycline, Oxytetracycline,Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/Dalfopristin, Thiamphenicol, Tigecycline(Bs), Tinidazole, Trimethoprim(Bs), Vancomycin, Doxycyline, Ceftobiprole[, Ceftaroline, Dalbavancin, Fusidic acid, Mupirocin, Omadacycline, Oritavancin, Tedizolid, Telavancin, Tigecycline, Ceftolozane/tazobactam, etambutol, isoniazid, pyrazinamide, aciclovir, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, and the like, but not limited to those.

In yet another embodiment, the API is an immuno suppressive compound comprising a steroid selected from the group consisting of 21-Acetoxyprefnenolone, Aalclometasone, Algestone, Amicinonide, Beclomethasone, Betamethasone, Betamethasone dipropionate, Betamethasone hemisuccinate, Budesonide, Chloroprednisone, Clobetasol, Blovetasone, Clocortolone, Cloprednol, Corticosterone, Cortisone, Cortivazol, Deflazacort, Desonide, Desoximethasone, dexamethason, Dexamethasone palmitate, Dexamethasone phosphate, Diflorasone, Diflucortolone, Difluprednate, Enoxolone, Fluazacort, Flucloronide, Flumethasone, Flunisolide, Fluocinolone Acetonide, Fludrocortisone, Fluocinonide, Fluocortin Butyl, Fluocortolone, Fluorometholone, Fluperolone, Fluprednidine, Fluprednisolone, Flurandrenolide, Formocortal, Halcinonide, Glucocorticoids, Halomethasone, Halopredone, Hydrocortamate, Hydrocortisone, Limethasone, Mazipredone, Medrysone, Meprednisone, Methyolprednisolone, Methyolprednisolone hemisuccinate, Mometasone Furoate, Paramethasone, Prednicarbate, Prednisolone, Prednisolone palmitate, Prednisolone phosphate, Prednisone, Prednival, Prednylidene, Tixocortal, and Triamcinolone, azathioprine, ciclosporine, 6-mercaptopurine, mycophenolate and the like, but not limited to those.

In one embodiment of the disclosure, an expanded ECell have altered drug release kinetics.

In one embodiment of the disclosure, an expanded ECell, containing interconnected such as percolated water channels and voids, have altered drug release kinetics.

In one embodiment of the disclosure, an expanded ECell, containing interconnected such as percolated water channels and voids provide a shorter path of diffusion for embedded APIs or particles, and have altered release kinetics of the payload.

The drug(s), active agent(s) or bioactive agent(s) formulated in the ECell composition of the present invention may be used for treating, monitoring, preventing and/or diagnosing several diseases and conditions (e.g., cancer, inflammation, hormonal diseases and deficiency, metabolic disorders and diseases, regenerative disease, chronic wounds and inflammation, autoimmune disease, or infections). Certain embodiments can involve delivery of the same, multiple, or possibly different therapeutic agents to a site or region affected by a disease or condition. In some embodiments, the APIs in the ECell technology of the present invention may be particularly useful for oncological applications, such as for the treatment, monitoring, prevention and/or diagnosis of a cancerous condition (e.g., malignant tumor). In such embodiments, the APIs in the ECell of the present invention may be used for delivering an active agent (e.g., a therapeutic and/or a diagnostic agent) to a site affected with cancer (e.g., a tumor). The ECell may injected intratumorally, adjacent to malignant tumors, in tissues harbouring the malignant cells (e.g., bone marrow or lymph nodes) in lymph nodes including metastatic nodes, on non-cancerous tissue, including intramuscular and subcutaneous. Non-limiting examples of cancerous conditions that may be treated, monitored, prevented and/or diagnosed include, without limitation, leukaemia, lymphoma, skin cancers (including melanomas, basal cell carcinomas, and squamous cell carcinomas), epithelial carcinomas of the head and neck, lung cancers (including squamous or epidermoid carcinoma, small cell carcinoma, adenocarcinoma, and large cell carcinoma), breast cancer, gastrointestinal tract cancers, malignant tumors of the thyroid, sarcomas of the bone and soft tissue, ovarian cancer, carcinoma of the fallopian tube, uterine cancer, cervical cancer, prostatic carcinoma, testicular cancer, bladder cancer, renal cell carcinoma, pancreatic cancer, and hepatocellular cancer. In some embodiments, the present invention provides a method for treating a subject with a cancer characterized by solid cancers/tumors.

In one embodiment the APIs in the ECell can be used in conjunction or concurrently with other known methods of disease treatment, including, but not limited to, chemotherapy, radiotherapy, thermal ablation, surgery, electroporation, laser therapy, oncolytic viruses, adoptive cell therapies, chimeric antigen receptor cell therapy, stem cell therapies, xenogenic, allogenic and autogenic transplants and radiotherapy.

In one embodiment the APIs in the ECell can be used to stimulate immunological recognition, immune activation, and immune mediated rejection of cancer in a human or animal. The ECell delivered combination of APIs may support innate and adaptive anticancer immune activation and/or immune cell recruitment, agility, polarization, engraftment, and proliferation.

In one embodiment the API in the ECell compositions may be one or more anticancer agents, including but not limited to cancer chemotherapy agents. Examples of suitable cancer chemotherapy agents include, without limitation: nitrogen mustards, nitrosorueas, ethyleneimine, alkane sulfonates, tetrazine, platinum compounds, pyrimidine analogs, purine analogs, nucleoside analogs, epigenetic modifying agents, nucleic acid synthesis inhibitiors, antimetabolites, folate analogs, anthracyclines, TPI-1, NSC-87877, taxanes, vinca alkaloids, and topoisomerase inhibitors, hormonal agents, alkylating agents, such as cyclosphosphamide; alkyl sulfonates; aziridines; ethylenimines and methylamelamines; anti-metabolites; nucleic acid synthesis inhibitors, pyrimidine analogs; anti-adrenals; folic acid replenisher; retinoic acid; and pharmaceutically acceptable salts, acids or derivatives of any of the above. Additional APIs in the ECell compositions may include, without limitation, anti-hormonal agents that act to regulate or inhibit hormone action on tumors. Non-limiting examples of such anti-hormonal agents include anti-estrogens, including for example Tamoxifen and Toremifene; anti-androgens, such as Leuprolide and pharmaceutically acceptable salts, acids, or derivatives of any of the above.

In one embodiment APIs that are affected by classical multidrug resistance can have utility as APIs in the ECell composition. Such drugs include, without limitation: vinca alkaloids (e.g., vinblastine), the antimetabolite and pyrimidine analogues (e.g., 5FU, gemcitabine) the anthracyclines (e.g., doxorubicin) and RNA transcription inhibitors.

In one embodiment immunotherapeutic APIs such as, but are not limited to, innate immune stimulating drugs (e.g. toll like receptor (TLR) agonists, RIG-1-like receptor agonists and Stimulator of Interferon Receptor (STING) agonists, Nucleotide-binding oligomerization domain-like (NOD-like) receptor agonists), immune activation pathway inhibitors and activators (e.g. Tumor necrosis factor alpha (TNF-a), TNF-a receptor blocking molecules, OX40 (CD124) agonists, TNFSFR agonists (e.g., CD40 agonists), SHP-1 inhibitors, SHP-2 inhibitors, SHP1/2 inhibitors, NSC87877, TPI-1, NSC-87877 SHP-1 agonists, T-Cell Immunoreceptor With Ig and ITIM Domains (TIGIT) agonists), transcription factor modulators, immune cell polarizing and immune modulating drugs (e.g. transforming growth factor beta inhibitors (TGF-βi), T box-containing protein expressed in T cells stimulators, glycogen synthase kinase 3 inhibitors, signal transducer and activator of transcription (STAT) inhibitors, Wnt/β-catenin signalling targeting therapy, Phosphoinositide 3-kinases (PI3Ks) inhibitors, c-KIT inhibitors, mammalian target of rapamycin (mTOR) inhibitors, C-Myc inhibitors, MET inhibitors, BRAF inhibitors, MEK inhibitors), lymphocyte activating and modulating therapeutics (e.g. programmed death-ligand 1 (PD-L1) inhibitors, programmed death-ligand 2 (PD-L2) inhibitors, programmed cell death protein 1 (PD-1) inhibitors, anti-cytotoxic T-lymphocyte-associated protein-4 (CTLA-4) inhibitors, KIR, NOX2, T-cell immunoglobulin and mucin domain-3 (TIM-3) inhibitors, lymphocyte activating gene 3 (LAG3) inhibitors, Tyrosine phosphatase SHP2 inhibitors), , immune metabolism and inflammatory programming therapeutics (e.g. indoleamine 2,3-dioxygenase-1 (IDO1) inhibitors, arginase (Arg) inhibitors, Adenosine A2A Receptor (A2AR) antagonists, hypoxia inducible factor 1 (HIF-1) inhibitors, hypoxia inducible factor 2 (HIF-2) inhibitors, V-domain Ig suppressor of T cell activation (VISTA) inhibitors, cyclooxygenase (COX) 1 and/or 2 inhibitors), IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36 and IL-37) inhibitors and activators, immunosuppressive agents (e.g. synthetic glycocorticoids, ciclosporine, Azathioprine, ketoconazole), acute phase protein inhibiting drugs (e.g. inhibitors of: C-reactive protein, Serum amyloid P,Serum amyloid A, Complement factors, Mannan-binding lectin, Fibrinogen, prothrombin, Plasminogen activator inhibitor-1 (PAI-1), tissue Plasminogen Activator (tPA), Alpha 2-macroglobulin, Ferritin , Ceruloplasmin, Haptoglobin, (Alpha-1-acid glycoprotein, AGP), Alpha 1-antitrypsin, and Alpha 1 antichymotrypsin), drugs modulating extracellular matrix composition (e.g. matrix metalloproteinases (MMP) including (MMP1, MMP2, MMP3, MMP7, MMP8, MMP9, MMP10, MMP11, MMP12, MMP13, MMP14, MMP15, MMP16, MMP17, MMP18, MMP19, MMP20, MMP21, MMP23A, MMP23B, MMP24, MMP25, MMP26, MMP27, MMP28), Osteoconductive and osteoinductive agents (e.g. osteoprotegerin agonists and/or Glycogen synthase kinase 3b inhibitors, transforming growth factor beta-1-3), and cell death inducing chemotherapeutics are prepared and formulated in a ECell composition.

In one embodiment an immune checkpoint inhibitor, including but not limited to inhibitors of PD-1, PD-L1, CTLA-4, LAG3, and TIM-3 is formulated in a ECell composition. In one embodiment an immune checkpoint inhibitor, including but not limited to inhibitors of PD-1, PD-L1, CTLA-4, LAG3, and TIM-3 is co-formulated with a TLR7/8 agonist and / or TGFb inhibitor in a ECell composition. In one embodiment an immune checkpoint inhibitor, including but not limited to inhibitors of PD-1, PD-L1, CTLA-4, LAG3, and TIM-3 is co-formulated with R848 and / or RepSox in a ECell composition. In one embodiment an immune checkpoint inhibitor, including but not limited to inhibitors of PD-1, PD-L1, CTLA-4, LAG3, and TIM-3 is co-formulated with R848 and / or galunisertib in a ECell composition.

In one embodiment a kinase or phosphatase inhibitor, including but not limited to inhibitors of SHP1, SHP2 and SHP1/2 is formulated in a ECell composition. In one embodiment a kinase or phosphatase inhibitor, including but not limited to inhibitors of SHP1, SHP2 and SHP1/2 is co-formulated with a TLR7/8 agonist and / or TGFb inhibitor in a ECell composition. In one embodiment a kinase or phosphatase inhibitor, including but not limited to inhibitors of SHP1, SHP2 and SHP1/2 is co-formulated with R848 and / or RepSox in a ECell composition. In one embodiment a kinase or phosphatase inhibitor, including but not limited to inhibitors of SHP1, SHP2 and SHP1/2 is co-formulated with R848 and / or galunisertib in a ECell composition.

In one embodiment an immunomodulator, including but not limited to inhibitors of beta-catenin, IDO, Arg1, A2AR, GSK3 and agonists for FLT3L, OX40L is formulated in a ECell composition. In one embodiment an immunomodulator, including but not limited to inhibitors of beta-catenin, IDO, Arg1, A2AR, GSK3 and agonists for FLT3L, OX40L is co-formulated with a TLR7/8 agonist and / or TGFb inhibitor in a ECell composition. In one embodiment an immunomodulator, including but not limited to inhibitors of beta-catenin, IDO, Arg1, A2AR, GSK3 and agonists for FLT3L, OX40L, CD40 is co-formulated with R848 and / or RepSox in a ECell composition. In one embodiment an immunomodulator, including but not limited to inhibitors of beta-catenin, IDO, Arg1, A2AR, GSK3 and agonists for FLT3L, OX40L is co-formulated with R848 and / or galunisertib in a ECell composition.

In additional embodiments, antibody-based therapeutics including monoclonal antibodies as well as derivatives or analogues thereof, including variable domain (Fv) fragments, single chain Fv (scFv) fragments, Fab' fragments, F(ab')2 fragments, single domain antibodies; antibody fragments, humanized antibodies, and antibody fragments; multivalent versions of the foregoing is formulated in a ECell compositions antibodies. Non-limiting examples include atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade.

In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, sarilumab, tocilizumab, siltuximab, and remicade is formulated in a ECell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is co-formulated with a TLR7/8 agonist and / or TGFb inhibitor in a ECell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is co-formulated with R848 and / or RepSox in a ECell composition. In one embodiment an antibody-based therapeutic, including but not limited to atezolizumab, Ipilimumab, atezolizumab, avelumab, durvalumab, pembrolizumab, nivolumab, cemiplimab, spartalizumab, herceptin, erbitux, avastin, rituxan, simulect, dostarlimab, enbrel, adalimumab, and remicade is co-formulated with R848 and / or galunisertib in a ECell composition.

In one embodiment the ECell composition may contain disease-associated antigens, such as one or more cancer associated antigen, one or more cancer associated neo-antigens, one or more pathogen-associated antigen or one or more degenerative-disorder-associated antigen. The term "disease-associated antigens" can relate to proteins produced in disease associated cells that have an abnormal structure and/or an abnormal expression pattern compared to non-disease associated cells. Abnormal proteins are also produced by cells infected with oncoviruses, e.g. EBV and HPV.

In one embodiment the Ecell composition contain one or more disease-associated antigenic peptides that are co-formulated to also release one or several immune activating agents such this invention provides a highly immunogenic vaccine based on the co-delivery of vaccine peptides and immune activating agents form the ECell composition capable of presenting both an antigen and adjuvant. The combined delivery of adjuvants for improved antigen presentation represents a promising strategy for therapeutic vaccines to elicit an innate immune response by exploiting the major properties of the two components: (1) the strong adjuvanticity provided by immune activating drugs in the ECell composition; and (2) the specific adaptive immune response against antigen(s) in the ECell composition and associated with the targeted disease. For example, antigen may represent any disease-associated antigen, such as one or more cancer associated antigen for cancer therapy, one or more pathogenic antigen for treatment of infection, or any other antigen or combination of antigens associated with other diseases, for immune-compromised conditions and/or where strong potentiation of immunity is needed (e.g., in the elderly). In addition, the invention provides a strong immune response important for vaccines such as those against cancer, hepatitis, flu, malaria, and HIV. The invention is also useful for any therapy where the presentation of a combination of antigens to the immune system of a patient may be beneficial.

In one embodiment single or multiple vaccine peptides and glycopeptides are incorporated in the ECell such as, but not limited to, cancer cell antigens; carcinoembryonal antigen, alpha-1-fetoprotein, isoferritin, and fetal sulphoglycoprotein, cc2-H- ferroprotein and γ-fetoprotein, p53, ART-4, BAGE, beta-catenin/m, Bcr-abL CAMEL, CAP-1 , CASP-8, CDC27/m, CD 4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1 , G250, GAGE, GnT-V, Gapl OO, HAGE, HER-2/neu, HPV-E7, HPV-E6, HAST-2, hTERT (or hTRT), LAGE, LDLR/FUT, MAGE- A, preferably MAGE-A1 , MAGE-A2, MAGE- A3, MAGE-A4, MAGE- A5, MAGE-A6, MAGE-A7, MAGE-A8, MAGE-A9, MAGE-A10, MAGE-A1 1 , or MAGE- A12, MAGE-B, MAGE-C, MART- 1 /Melan-A, MC1 R, Myosin/m, MUC1 , MUM-1 , -2, -3, NA88-A, NF1 , NY-ESO-1 , NY-BR-1 , pl 90 minor BCR-abL, Pm I/RARa, PRAME, proteinase 3, PSA, PSM, RAGE, RUI or RU2, SAGE, SART-1 or SART-3, SCGB3A2, SCP 1 , SCP2, SCP3, SSX, SURVrVIN, TEL/AMLI , TPI/m, TRP-1 , TRP-2, TRP-2/1 NT2, TPTE and WT, preferably WT-1 and antigens for peptide and glycopeptide vaccines for infectious diseases including, but not limited to, circumsporozoite protein (CSP), apical membrane antigen 1 (AMA-1), UK39, IC41 (IPEP 83, 84, 87, 89, and 1426), gp41, gp120, gp100, and antigens for vaccines against neurodegenerative, storage and autoimmune diseases, including but not limited to, Tau, Aβ(1-42).

The vaccine peptides may be prepared and formulated in ECell compositions and delivered as single peptides or multiple disease or cancer associated antigenic peptides.

In one embodiment personalized peptide(s) for neo-antigen vaccines, synthesized based on patient tissue samples analyses, sequencing and cancer and/or neoantigen identification, are formulated in a ECell composition. The peptides, or a combination of these may be formulated as single or multipeptide combinations in the ECell and may include immune activating adjuvants in the ECell composition, examples of adjuvants include, but are not limited to Poly(I:C), Poly ICLC, STING agonists, MDA5, TLR agonists (e.g., TLR3, TLR4, TLR7, TLR8, and TLR9), RIG-1 agonists, interleukins, including but not limited to IL-1, IL-2, IL-15, IL-15 sushi, IL-6, IL-12 single chain IL-12p35, IL-12p40, IL-12p70, IL-12, cytokines and chemokines, including but not limited to CCR7, CCL20, CCR7, CXCL9, CXCL10, CXCL11, FLT3L, OX40L, TNFs and IFNs, immune modulators, including but not limited to inhibitors of beta-catenin, IDO, Arg1, A2AR, GSK3, adjuvants including but not limited to 1018 ISS, aluminium salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, JuvImmune, LipoVac, MF59, monophosphoryl lipid A, Montanide IMS 1312 VG, Montanide ISA 206 VG, Montanide ISA 50 V2, Montanide ISA 51 VG, OK-432, OM-174, OM-197-MP-EC, ISA-TLR2 agonist, ONTAK, PepTel@ vector system, PLG microparticles, resiquimod, SRL172, virosomes and other virus-like particles, YF-17D, VEGF trap, R848, beta-glucan, Pam3Cys, Pam3CSK4, acrylic or methacrylic polymers, copolymers of maleic anhydride, and QS21 stimulon. a co-stimulatory ligand, a TNF ligand, an Ig superfamily ligand, CD28, CD80, CD86, ICOS, CD40L, OX40, CD27, GITR, CD30, DR3, CD69, or 4-1BB. In one embodiment a single or combination vaccine epitopes are co-delivered with an TLR agonist and a TGFb inhibitor. In one embodiment a single or combination of vaccine epitopes are co-delivered with an TLR7/8 agonist and an immune checkpoint inhibitor, including but not limited to inhibitors of PD-1, PD-L1, CTLA-4, LAG3, and TIM-3.

In one embodiment peptides or proteins as single or multipeptide vaccine combinations may be co-formulated with an TLR7/8 agonist and a TGFb inhibitor in a ECell composition.

In one embodiment peptides or proteins as single or multipeptide vaccine combinations may be co-formulated with an TLR7/8 agonist and an immune checkpoint inhibitor, including but not limited to inhibitors of PD-1, PD-L1, CTLA-4, and TIM-3 in a ECell composition.

In one embodiment peptides or proteins as single or multipeptide vaccine combinations may be co-formulated with R848 and RepSox in a ECell composition

In one embodiment peptides or proteins as single or multipeptide combinations may be co-formulated with a R848 agonist and galunisertib in a ECell composition.

In one embodiment therapeutic agents that acts as agonist or antagonists for cytokines, chemokines, and growth factors are formulated in an ECell composition. Non-limiting examples of such cytokines are lymphokines, monokines, chemokines, leukotrins and hormones. Examples include agents that are active agonists or antagonist for such as but not limited to growth factors including vascular endothelial growth factor (VEGFs), hepatic growth factor; fibroblast growth factor (FGFs); integrin; thrombopoietin (TPO); nerve growth factors such as NGF-β; platelet growth factor; transforming growth factors (TGFs, BMPs); insulin-like growth factor-I and -II (IGFs); erythropoietin (EPO); osteoinductive factors; interferons, such as interferon-a, -β and -γ; colony stimulating factors (CSFs), such as macrophage-CSF (M-CSF), granulocyte-macrophage-CSF (GM-CSF), granulocyte-CSF (GCSF) and the like; interleukins (ILs); tumor necrosis factors, such as TNF-α or TNF-β; and other polypeptide factors, including LIF and kit ligand (KL). As used herein, the term cytokine includes proteins from natural sources or from recombinant sources (e.g., from T-cell cultures and biologically active equivalents of the native sequence cytokines). In one embodiment of the invention single or multiple growth factors are formulated in an ECell composition in combination with a single or combination of multiple antibiotics for the treatment of diseases, including but not limited to non-healing bone fractures, vascular defects, degenerative diseases, chronic infected wounds, diabetic ulcers.

In one embodiment of the invention, hormones or therapeutic synthetic hormone agonist or antagonists are formulated in an ECell composition. Non-limiting examples of hormones for and therapeutically active agents that are active agonists or antagonist for hormones are peptide hormones and peptide hormone analogues: human growth hormone; parathyroid hormone (PTH); thyroxine; insulin; proinsulin; relaxin; prorelaxin; glycoprotein hormones; prolactin; placental lactogen; tumor necrosis factor-a and -β; mullerian-inhibiting substance; gonadotropin-associated peptide; inhibin; activin; adrenocorticotropic hormone (ACTH), amylin, angiotensin, atrial natriuretic peptide (ANP), calcitonin, cholecystokinin (CCK), gastrin, ghrelin, glucagon, follicle-stimulating hormone (FSH), insulin, Glucagon-like peptide-1 (GLP-1), leptin, luteinizing hormone (LH), melanocyte-stimulating hormone (MSH), oxytocin, parathyroid hormone (PTH), prolactin, renin, somatostatin, thyroid-stimulating hormone (TSH) thyrotropin-releasing hormone (TRH), vasopressin (arginine vasopressin (AVP) or anti-diuretic hormone (ADH)), vasoactive intestinal peptide (VIP), and steroid hormones and analogues: Glucocorticoids: alclometasone, prednisone, dexamethasone, triamcinolone, cortisone, Mineralocorticoid: fludrocortisone Vitamin D: dihydrotachysterol, Androgens: oxandrolone, oxabolone, nandrolone (also known as anabolic-androgenic steroids or simply anabolic steroids), Oestrogens: diethylstilbestrol (DES) and ethinyl estradiol (EE), Progestins: norethisterone, medroxyprogesterone acetate, hydroxyprogesterone caproate, and steroid hormone antagonists and analogues: cyproterone acetate, mifepristone, and gestrinone.

In one embodiment of the invention, therapeutic peptides, glycopeptides, glycoproteins, lipidated peptides, radionuclide-peptide conjugates and drug-peptide conjugates are formulated in an ECell composition. Non-limiting examples include, CXCR4 receptor antagonist LY-2510924, Romiplostim, DOTATATE, DOTATOC, Zoptarelin, NA-1 (HIV-TAT peptide), Peginesatide, silengitide, Liraglutide, GLP-1 receptor agonists derived from the exendin-4 structures, Dopastatin (BIM-23A760), etelcalcetide, Mibenratide, Balixafortide, Etelcalcetide, Abaloparatide, Plecanatide, Virginiamycin M1, Dalfopristin, Quinupristin, Ramoplanin, Telavancin, Dalbavancin, Etelcalcetide, dulaglutide, Plecanatide, Larazotide, carfilzomib, albiglutide receptor agonist romiplostim (AMG531), abaloparatide, Pegfilgrastim, nerinetide, Sermorelin, Anakinra, Gramicidin D, Cetrorelix, Abarelix, Romidepsin, Spirapril, Lanreotide, leuprotide, lanreotide, linaclotide, afamelanotide, derivatives of the endogenous peptides apelin, adrenomedullin, and neuromedin U, elcatonin, Thymalfasin, eledoisin, enalapril, bivalirudin, cemadotin, exenatide, pexiganan, ziconotide, chlorotoxin 1-135 conjugate, Elisidepsin, dalazatide, SOR-C13, corticotropin, lypressin, vasopressin, Eledoisin, somatostatin, calcitonin, ornipressin, desmopressin, terlipressin, ambamustine, tetracosactide, elcatonin, felypressin, saralasin, cargutocin, buserelin, thiazolidine, ligdunine, leuprorelin, thymopentin, triptorelin, Sprifermin, Corticorelin, Leptin, goserelin, lisinopril, romurtide, glucagon, deslorelin, Plitidepsin, gonadorelin, ghrelin, motilin, conjugates of somatostatin-like peptides, macimorelin, nafarelin, histrelin, lanreotide, semaglutide, Tigapotide, Aclerastide, Albusomatropin, Anamorelin, G17DT, Insulin peglispro, Lenomorelin, Selepressin, Somapacitan, Cibinetide, Taspoglutide, Thymosin beta-4, Tirzepatide, Ularitide, Vapreotide, Lenograstim, Vosoritide, Zoptarelin doxorubicin, Angiotensin 1-7, Bombesin, Cenderitide, Deslorelin, Gastric inhibitory polypeptide, MK-3207, Olcegepant, Zilucoplan, Pancreatic Polypeptide, Peptide YY (3-36), Pirnabine, Somatoprim, Somatropin pegol, Thyrotropin, TT-232, BPI-3016, NBI-6024, NN344, TERT572Y, NP-12, PL120131, carperitide, Teverelix, glatiramer, eptifibatide, cetrorelix, ganirelix, P-15, atosiban, taltirelin, aviptadil, nesiritide, ovokinin, novokinin, bivalirudin, carbetocin, teriparatide, abarelix, enfuvirtide, ziconotide, abaloparatide, Ramucirumab, pazopanib, CBO-P11, Nesfatin-1, Polaprezinc, pramlintide, exenatide, romiplostim, Avexitide, Calcitonin gene-related peptide, Parathyroid Hormone-Related Protein 1-36, Peginterferon alfa-2a, degarelix, icatibant, Tiplimotide, mifamurtide, FOL-005, liraglutide, tesamorelin, peginesatide, Beinaglutide, lucinactant, pasireotide, teduglutide, linaclotide, lixisenatide, pazopanib, cabozantinib, Elobixibat, Rapastinel, lenvatinib, axitinib, vandetanib, Cediranib, Ipafricept, axitinib, Recombinant CD40-ligand, Aspartyl-alanyl-diketopiperazine, cabozantinib, albiglutide, dulaglutide, etelcalcetide, Plecanatide, bortezomib, ixazomib, carfilzomib, Forigerimod, sunitinib, vemurafenib, dabrafenib, encorafenib, trametinib, binimetinib, sorafenib, pazopanib, Setmelanotide, Plecanatide, Menotropins, Davunetide, Oprozomib, Pegdinetanib, Nagrestipen, Etelcalcetide, Macimorelin, Etelcalcetide, Murepavadin, Edratide, Pegcetacoplan, Erythropoietin, Pasireotide, PepGen P-15, Bremelanotide, nintedanib, Afamelanotide, Angiotensin II, Cyclosporin A, Eptifibatide, Difelikalin, idelalisib, Pegbelfermin, Voclosporin, Desmopressin, Pegvisomant, Zoptarelin Doxorubicin, (AN-152, AEZS-108), ANG1005, G202 (Mipsagargin), BIM-23A760, ⁶⁸Ga gozetotide, Lu177-dotatate, [111In-DTPA-D- Phe1]-octreotide, Zoptarelin Doxorubicin, AN-152, AEZS-108, GRN1005, Paclitaxel poliglumex CT2103, EP-100, BIM-23A760, Becaplermin,

CGC 1072, KAI-1455 KAI-1678, KAI-9803, PTH(1-84), XG-102, DTS-108, DTS-201, BT-1718, 177Lu- PSMA-617, GLP-1-estrogen conjugate, GLP-1-estradiol conjugate, Pentetreotide, Glucagon-T3 Conjugate, RGD-doxorubicin conjugate, and EETI-2.5Z-Val-Ala-PABC-gemcitabine.

In one specific embodiment of the invention a parathyroid hormone-related protein (PTHrP) analog is formulated in an ECell composition.

In one embodiment a parathyroid hormone-related protein (PTHrP) analog, including but not limited to abaloparatide, teriparatide or PTH(1-84) is formulated in an ECell composition.

In one embodiment antibiotic agent(s) belonging to the following groups may be formulated in the ECell composition according to the present invention. One of the advantages of incorporating one or more antimicrobial agents in the ECell composition according to the present invention is that it can provide a localized antimicrobial agent(s) release depot with a much higher local concentration of the agent or agents than would be possible by systemic treatment with the same. The flexible and precise thin needle injection technology can further allow surgeon to accurately decorate the infected region(s)/lesion(s) to be treated.

In one embodiment antimicrobial agents such as, without limitation: Aminoglycosides, Amikacin, Gentamicin, Kanamycin, Neomycin, Netilmicin, Tobramycin, Paromomycin, Streptomycin, Spectinomycin(Bs), Ansamycins, Geldanamycin, Herbimycin, Rifaximin, Carbacephem, Loracarbef, Carbapenems, Ertapenem, Doripenem, Meropenem, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefdinir, Cefditoren, Cefotaxime, Cefpodoxime, Ceftibuten, Ceftizoxime, Moxalactam, Ceftriaxone, Cefepime, Ceftaroline fosamil, Ceftobiprole, Glycopeptides, Teicoplanin, Vancomycin, Telavancin, Dalbavancin, Oritavancin, Lincosamides(Bs), Clindamycin, Lincomycin, Lipopeptide, Daptomycin, Macrolides(Bs), Azithromycin, Clarithromycin, Erythromycin, Roxithromycin, Telithromycin, Spiramycin, Fidaxomicin, Monobactams, Aztreonam, Nitrofurans, Furazolidone, Nitrofurantoin(Bs), Oxazolidinones(Bs), Linezolid, Posizolid, Radezolid, Torezolid, Penicillins, Amoxicillin, Ampicillin, Azlocillin, Dicloxacillin, Flucloxacillin, Mezlocillin, Methicillin,Nafcillin,Oxacillin,Penicillin G,Penicillin V,Piperacillin, Temocillin, Ticarcillin, Amoxicillin/clavulanate, Ampicillin/sulbactam, Piperacillin/tazobactam, Ticarcillin/clavulanate, Polypeptides, Bacitracin, Colistin, Polymyxin B, Polymyxin E, Ciprofloxacin, Enoxacin, Gatifloxacin, Gemifloxacin, Levofloxacin, Lomefloxacin, Enrofloxacin, Moxifloxacin, Nadifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide (archaic), Sulfasalazine, Sulfisoxazole, Sulfonamidochrysoidine (archaic), Tetracyclines(Bs), Demeclocycline, Doxycycline, Metacycline, Minocycline, Oxytetracycline,Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/ Dalfopristin, Thiamphenicol, Tigecycline(Bs), Tinidazole, Trimethoprim(Bs), Vancomycin, Doxycyline, Ceftobiprole[, Ceftaroline, Dalbavancin, Fusidic acid, Mupirocin, Omadacycline, Oritavancin, Tedizolid, Telavancin, Tigecycline, Ceftolozane/tazobactam, etambutol, isoniazid, pyrazinamide, aciclovir, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, and the like, but not limited to those are prepared and formulated in an ECell composition.

In some embodiments bioactive agents in an ECell composition may be used for treating or preventing a bacterial, fungal, viral, parasite infection in a subject or an organism. In this context, the microbial infection may be caused by a Gram-negative or a Gram-positive bacterium, fungus, virus or parasite (infestation, e.g., helminths, protozoa). The subject affected by the bacterial infection may be a mammal, such as a human being. The acute and chronic diseases include, but is not limited to endocarditis, abscesses, phlegmons, respiratory and pulmonary infections, central nervous system infections, otitis interna, media and externa, eye infections, bone and joint infections, urinary tract infections, gastrointestinal infections and skin and soft tissue infections including wound infections, pyoderma and dermatitis, chronic and acute wound infections, diabetic ulcers, diabetic foot ulcers, chronic ulcers, implant associated osteomyelitis, and pyoderma.

In one embodiment of the invention any combination of gentamycin, vancomycin, linezolid, clindamycin, levofloxacin, ciprofloxacin, rifampicin, and rifampin is formulated in an ECell composition.

In one embodiment bioactive agents and/or imaging agent in the ECell composition provides an injectable liquid ready for use in the treatment by injection in tissues, including soft tissue, organs, and bones for e.g., as part of a surgical treatment, post-surgical treatment, or radiation therapy, to treat disorders of supportive tissue in a human or animal by regenerating and healing of bone tissue, soft tissue, soft tissue infections and/or treating bone infections, excessive scar or fibrose tissue formation. Such disorders may be, but not limited to bone loss, peritendinous adhesion formation, excessive granulation, chronic wounds, scar tissue, fibrosis, bone fracture, bone non-union, cartilage regeneration, bone trauma, radiation fibrosis, radionecrosis, cartilage regeneration, osteoarthritis, musculoskeletal diseases and disorder, soft tissue infections and/or osteomyelitis and bioactive agents include, but are not limited to antioxidants, vitamins, hormones, antibiotics, cytostatic agents (e.g., 5-FU), bisphosphonates, or growth factors and analogues of these, including but not limited to BMPs, VEGFs (VEGF-A, VEGF-B, VEGF-C, VEGF-D, and placental growth factor (PIGF)), FGFs, TGFb1-3, BMPs, BMP-4, M-CSF, G-CSF, EGFs, EPO, HGFs, IGFs, OGFs, CGRPs,NRGs, PDGF, KGF, ILs, GDF9, GDNF, Calcitonin gene-related peptides (CGRP), Thrombin Peptide 508 (Chrysalin), and β-tricalcium phosphate, Teriparatide, BMPs belonging to the transforming growth factor β(TGFβ) superfamily, including but not limited to TGFβs, activins/inhibins, Mullerian-inhibiting substance (MIS) and glial cell line-derived neurotrophic factor, GFOGER (glycine-phenylalanine-hydroxyproline-glycine-glutamate-arginine), SVVYGLR peptide, amide derivatives of stilbene, Bergenin, P15, RADA16-I, RANKL-binding peptide, core-binding factor A1 (CBFA1) Runt-related transcription factor-2 (RUNX-2), AC-100, mechano growth factor E, Osteoactivin and B2A2-K-NS (B2A) collagen-mimetic formulated in an ECell composition.

In one embodiment a bioactive agent is combined with an antimicrobial agent, or a combination hereof in a ECell composition. In one embodiment of the invention a growth factor for supporting bone formation, including but not limited to TGFb, BMPs (BMP-4), FGF agonists and analogues, and one or a combination of antimicrobial agents in a ECell composition.

In one embodiment solid support material is included in the ECell composition to provide material for tissue regeneration including but not limited to bone regeneration, healing, structure for cosmetic purposes, biomaterial for improved cellular ingrowth. Examples of solid support material include but are not limited to calcium sulphate, calcium phosphate, hydroxyapatite, silver nanoparticles, silver microparticles, organophosphates, whitlockite (WH), octacalcium phosphate, tetracalcium phosphate, beta-TCP, zirconia phosphate, silver nitrate (AgNO₃), chitosan, PLGA, PEG, polycaprolactone (PCL), poly-l-lactic acid, monocalcium phosphate monohydrate, bioglass, polytetrafluoroethylene, polyethylene-oxide-terephtalane, polybuthylene-terephtalate-polymer, titanium, titanium salts, polypropylenefumarate (PPF), poly-methylmethaacrylate (PMMA), tricalcium phosphate, hydroxyapatite.

In one embodiment bioactive agents and/or imaging agent in the ECell composition provides an injectable liquid ready for use in clinical treatment of infections or inflammation or immune reactivity and autoimmunity associated with, but not limited to catheters, stent, grafts, pins, screws, needles, plates, artificial joints, passive and active biomaterials, artificial organs, allografts, orthografts, autografts, xenografts, bioactive devices, and implants. The ECell composition provides a flexible technology to both inject in around the device or material and in the surrounding tissues affect by disease e.g., infections, pain, inflammation, allergic foreign body responses. Possible bioactive agents can further provide local thromboresistance and thereby inhibit: (i) protein and cell adsorption, (ii) thrombin and fibrin formation, and (iii) platelet activation and aggregation to inhibit implant-induced coagulation.

In one embodiment an expanding ECell comprising cell stimulating polymer and or peptides and or lipids has a modified surface that enhances interaction with Cells and with tissues. In one embodiment the expanding ECell expose cell interacting ligands on the surface that support attachment, division, expansion, growth direction, extracellular matrix modelling, phenotypical polarization of the cells in the tissue where the ECell composition is injected. In one embodiment the expanding ECell composition may present natural or recombinant peptide ligands towards the surrounding cells, bioactive peptides, antimicrobial peptides, anti-inflammatory peptides and ligands includes, but are not limited to, RGD, cyclic RGD, RGD, collagen, laminin, fibronectin, collagenases, MMPs, plasmin, glycosaminoglycans, vascular endothelial growth factor, innate defense regulator (IDR) peptides, IDR-1018, KGD, PHSRN. In one embodiment expanding ECell composition include peptides that has therapeutic activity or support tissue and organ repair and or stem cell recruitment including, but not limited to YIGSR, Q11, KGF-ELP, Thymosin-beta4, RGD, RGDfK, GRGDS, QHREDGS, MHSPGAD, VVAGEGDKS, VEGF, FGF, HBPA alone or in combination. Expanding ECell may also include the large extracellular matrix (ECM) molecules, such as collagen and fibronectin, have multiple peptide sequences that are recognized by cells and induce multiple regenerative responses. In one embodiment the expanding ECell composition include but are not limited to natural or recombinant analogues of platelet-derived growth factor (PDGF), RADA class of ionic self-complementary peptides, TGFbeta 1-3, VEGF, FGF, IGF, stromal cell derived factor, GAGs, synthetic GAGs, pro-angiogenic laminin-derived C16, thymosin β4-derived Ac-SDKP. In one embodiment the expanding ECell composition include provide factors for improving bone healing and growth, including but not limited to factors that stimulate osteoconduction, osteoinduction, osteogenesis, and osteointegration. In one embodiment the expanding ECell composition provides or releases bone morphogenic proteins (BMPs, including BMP-2, BMP-4, BMP-7), bone forming peptide-1 (BFP-1).

The excavation of devitalized and infected cortical and cancellous bone is central in the therapy for bone infections, deep tissue infections associated with necrotic tissue and sequester removal, diabetic foot ulcers and may in some cases also involve parts of the joint space and tissues. In one embodiment the expandable ECell composition is injected into the tissue defect formed during excavation and tissue debridement. In one embodiment the expandable ECell composition forms a void filling material that expands to provide optimal filling of the defect. In one embodiment the expandable ECell composition also provide release of one or more antimicrobial agents, including but not limited to Aminoglycosides, Amikacin, Gentamicin, Kanamycin, Neomycin, Netilmicin, Tobramycin, Paromomycin, Streptomycin, Spectinomycin(Bs), Ansamycins, Geldanamycin, Herbimycin, Rifaximin, Carbacephem, Loracarbef, Carbapenems, Ertapenem, Doripenem, Meropenem, Cefadroxil, Cefazolin, Cephradine, Cephapirin, Cephalothin, Cefalexin, Cefaclor, Cefoxitin, Cefotetan, Cefamandole, Cefmetazole, Cefonicid, Loracarbef, Cefprozil, Cefuroxime, Cefdinir, Cefditoren, Cefotaxime, Cefpodoxime, Ceftibuten, Ceftizoxime, Moxalactam, Ceftriaxone, Cefepime, Ceftaroline fosamil, Ceftobiprole, Glycopeptides, Teicoplanin, Vancomycin, Telavancin, Dalbavancin, Oritavancin, Lincosamides(Bs), Clindamycin, Lincomycin, Lipopeptide, Daptomycin, Macrolides(Bs), Azithromycin, Clarithromycin, Erythromycin, Roxithromycin, Telithromycin, Spiramycin, Fidaxomicin, Monobactams, Aztreonam, Nitrofurans, Furazolidone, Nitrofurantoin(Bs), Oxazolidinones(Bs), Linezolid, Posizolid, Radezolid, Torezolid, Penicillins, Amoxicillin, Ampicillin, Azlocillin, Dicloxacillin, Flucloxacillin, Mezlocillin, Methicillin,Nafcillin,Oxacillin,Penicillin G,Penicillin V,Piperacillin, Temocillin, Ticarcillin, Amoxicillin/clavulanate, Ampicillin/sulbactam, Piperacillin/tazobactam, Ticarcillin/clavulanate, Polypeptides, Bacitracin, Colistin, Polymyxin B, Polymyxin E, Ciprofloxacin, Enoxacin, Gatifloxacin, Gemifloxacin, Levofloxacin, Lomefloxacin, Enrofloxacin, Moxifloxacin, Nadifloxacin, Nalidixic acid, Norfloxacin, Ofloxacin, Trovafloxacin, Grepafloxacin, Sparfloxacin, Temafloxacin, Mafenide, Sulfacetamide, Sulfadiazine, Silver sulfadiazine, Sulfadimethoxine, Sulfamethizole, Sulfamethoxazole, Sulfanilimide (archaic), Sulfasalazine, Sulfisoxazole, Sulfonamidochrysoidine (archaic), Tetracyclines(Bs), Demeclocycline, Doxycycline, Metacycline, Minocycline, Oxytetracycline,Tetracycline, Clofazimine, Dapsone, Capreomycin, Cycloserine, Ethambutol(Bs), Ethionamide, Isoniazid, Pyrazinamide, Rifampicin, Rifabutin, Rifapentine, Streptomycin, Arsphenamine, Chloramphenicol(Bs), Fosfomycin, Fusidic acid, Metronidazole, Mupirocin, Platensimycin, Quinupristin/ Dalfopristin, Thiamphenicol, Tigecycline(Bs), Tinidazole, Trimethoprim(Bs), Vancomycin, Doxycyline, Ceftobiprole[, Ceftaroline, Dalbavancin, Fusidic acid, Mupirocin, Omadacycline, Oritavancin, Tedizolid, Telavancin, Tigecycline, Ceftolozane/tazobactam, etambutol, isoniazid, pyrazinamide, aciclovir, Valaciclovir, efavirenz, emtricitabin, tenofovirdisoproxil, Rilpivirine, penicillin, Trimethoprim-sulfamethoxazole, rifampicin, etambutol, isoniazid, pyrazinamide, voriconazole, amphotericin B, caspofungin, flucytosine, itraconazole, and the like. In one embodiment the expandable ECell composition provides void filling and release of gentamycin, vancomycin, clindamycin, rifampicin, rifampin, lexofloxacin as single antibiotics or in any possible combination. In one embodiment the expandable ECell composition contains 1 - 200 mg gentamycin, vancomycin, rifampicin, rifampin, levofloxacin or clindamycin per gram ECell.

In one embodiment the expanding ECell composition provides release of antibiotic and release of surface expression of factors that stimulate osteoconduction, osteoinduction, osteogenesis, and osteointegration.

In one embodiment the ECell composition provides a biomaterial for injection in regions complicated deep wound infections, e.g., diabetic foot ulcers, to serve as a biocompatible scaffold that provides angiogenic growth factors, fibrogenic growth factors, antimicrobial activity to support tissue regeneration, revitalization, epithelization and healing.

In one embodiment the expanding ECell composition provide a scaffold that provides both a physical surface for growth on the surface or into the expanded ECell. In one embodiment the expanding ECell composition provide a physical scaffold that also release pro-survival factors that support tissue regeneration, including stem cell therapies, non-limiting examples include Vascular endothelial growth factor (VEGF),Fibroblast growth factor-2 (FGF-2), Platelet derived growth factor (PDGF), Platelet derived endothelial cell growth factor/thymidine phosphorylase (PD-ECGF/TP), Angiopoietins (Ang), Angiotropins, Hepatocyte growth factor (HGF), Insulin like growth factors (IGFs), Bone morphogenic protein (BMPs), Transforming growth factors (TGFs), Matrix metalloproteinases (MMPs), Tumor necrosis factor (TNF), Epidermal growth factors (EGFs), regulator of G- protein signaling-5 (Rgs5), matrix metalloproteinase-9 (MMP-9), chemokine (C-X-C) ligand-10 (CXCL10), chemokine (C-C) ligand (CCL5)), Adrenomedullin, angiogenin, fibroblast growth factor-2 (FGF2), CXCL12, cistatin C, cysteine-rich angio- genic inducer 61 (Cyr61), Dickkopf-related proteins (Dkk), hepatocyte growth factor (HGF), insulin-like growth factor (IGF), IL-1, IL-6, pigmented epithelium-derived factor (PEDF), placental growth factor (PLGF), SDF1, TSG6, VEGF, MMP-2, tissue inhibitor of metalloproteinase-1 (TIMP-1), TIMP-2, secreted frizzled related protein-2 (SFRP-2), thrombospondin-1, and tenascin C.

In one embodiment the expanding ECell composition provide a physical scaffold that release immunomodulatory factors including but not limited to NOS, IDO, TGF-β and PGE-2, NOTCH, IL-10. In one embodiment the expanding ECell composition provide a physical scaffold that release factors that support stem cell survival including but not limited to EGF, KGF, VEGF-α, TGF, MIP, PGE-2, LIF and metabolites.

### Examples

### Example 1: The effect of excipients on in vitro expansion of expanding ECells

### Aim: To test various compositions of expanding ECells and establish what effect each excipient has on the expansion property of expanding ECells

### Materials & Methods

Various excipients were tested. Each excipient was weighed on an analytical scale at the given w/w fraction, after which they were sonicated at 75 °C for 2 hrs, with vortexing every 30^{th} minute. The formulations were as follows:
*Carbohydrate ester variation:* MeLOIB:CAB:DMSO (60:5:35), LOP:CAB:DMSO (60:5:35), LOIB:CAB:DMSO (60:5:35), TOIB:CAB:DMSO (60:5:35), SuPA:CAB:DMSO (60:5:35), RaBen:CAB:DMSO (60:5:35)
*CAB variation:* LOIB:CAB:DMSO (65:2.5:32.5), LOIB:CAB:DMSO (65:5:30), LOIB:CAB:DMSO (62.5:7.5:30), LOIB:CAB:DMSO (32:18:50)
*Co-solvent variation:* LOIB:CAB:GTO:DMSO (65:5:2.5:27.5), LOIB:CAB:GTH:DMSO (55:5:5:35)
*Solvent type variation,* LOIB:CAB:EtOH (65:5:30), LOIB:CAB:DMSO (65:5:30)

CAB 12kDa was used for these experiments. Expansion was evaluated by injecting ≈100 mg expanding ECells in 4 ml sterile Dulbecco's Phosphate-Buffered Saline (dPBS), in an 8 ml pre-weighed glass vial followed by incubation at 37 °C. At each chosen time point the dPBS was removed, and the vial + expanding ECells weight (with absorbed water) was subtracted the known weight of the vial itself, yielding the weight of the expanding ECells (+ potential absorbed water) at each specific time point. Volumetric measurements were not carried out, solely expansion based on weight. Each expanding ECells formulation was run in triplicates and data analysis was thus based on average weight ± standard deviation.

### Results & Discussion

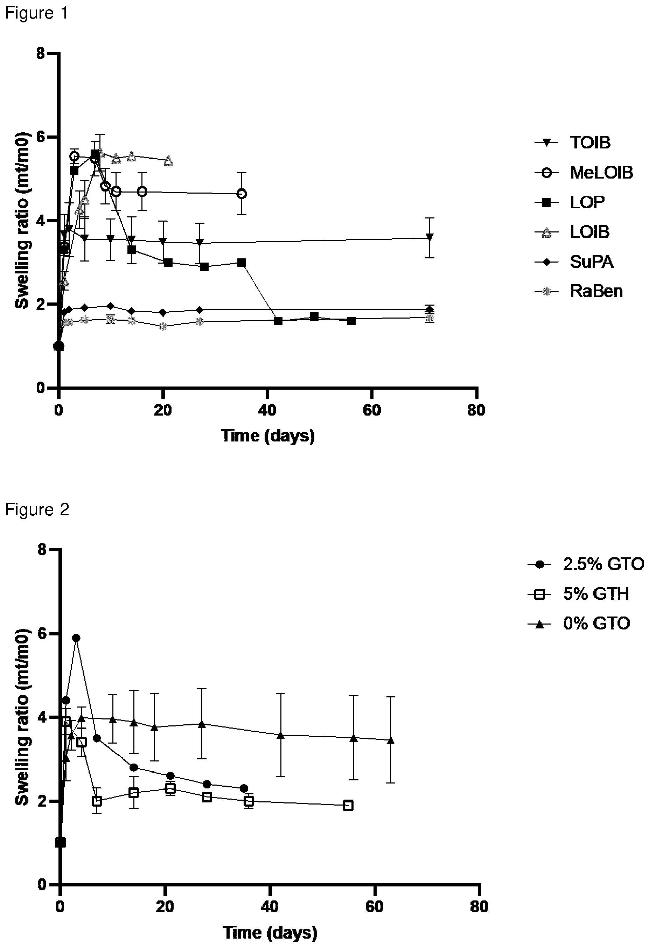

Figure 1 shows the impact of varying the type of carbohydrate ester on the expansion property of expanding ECells. The type of hydrophobic carbohydrate, which is the major constituent of expanding ECells, influences the maximum capacity of expansion. Here, the swelling ratio (on the y-axis) was defined as the weight of the ECell + absorbed water at the specific time point (x-axis), divided by the initial injection mass of the ECell.

The maximum expansion of a LOIB:CAB:DMSO (60:5:35) ECell formulation was ~5.5-fold its initial weight, which peaked after 72 hrs. In general, ECells expand within 2-4 days followed by either steady state or gradual collapse (see Figure 1).

Varying the type of carbohydrate only slightly changed the peak time (2-4 days) but changed the maximum expansion capacity. The order from highest to lowest capacity was: MeLOIB ≥ LOP > LOIB ≥ TOIB > SuPA ≥ RaBen (see Figure 1).

Figure 2 shows the impact of varying the triglyceride co-solvent content on the expansion property of expanding ECells. The w/w content of triglyceride co-solvent changes influences the maximum capacity of expanding and the collapse rate of the formed expanding ECells, tested at 37 °C. Here, the swelling ratio (on the y-axis) was defined as the weight of the ECell + absorbed water at the specific time point (x-axis), divided by the initial injection mass of the ECell.

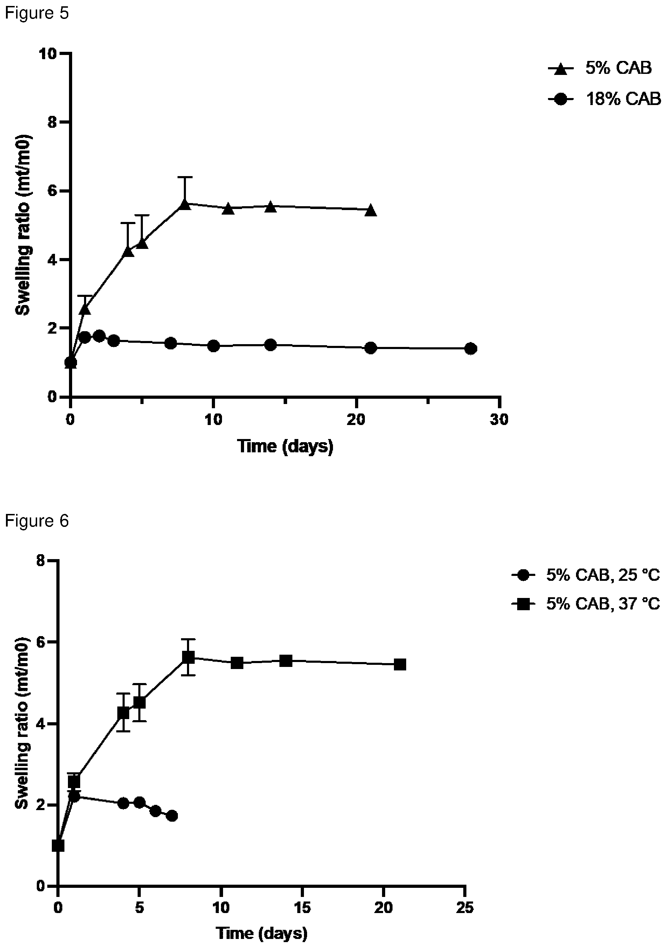

Varying the amount of co-solvent yielded a higher maximum capacity: for LOIB, 4x initial weight without GTO; but with 2.5% GTO, the maximum expansion capacity rose to 5.9-fold. However, the rate of collapse was much faster, meaning the expanding ECells with 2.5% GTO was ~3-fold its initial weight after 10 days, whereas 0% GTO was still ~4-fold its initial weight (see Figure 2). As seen in Figure 2, 5% w/w GTH lowered the maximum expansion capacity, but also dramatically increased the collapsing rate, stabilizing around ~2-fold times the initial weight after up to 55 days.

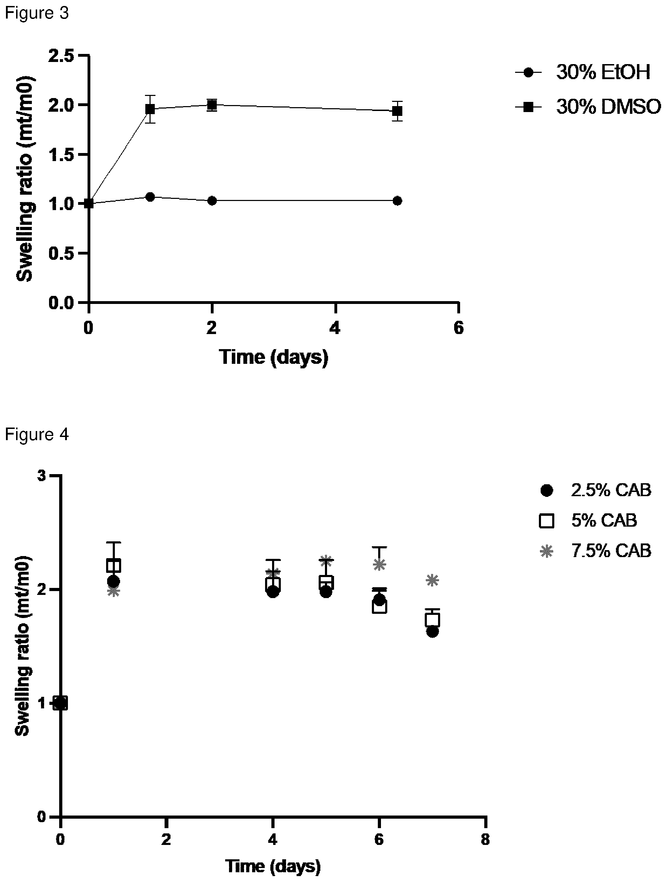

Figure 3 shows the impact of solvent type on the expansion property of expanding ECells, at 25 °C. Two solvent clinical class III solvent types were tested at 25 °C, in two formulations: LOIB:CAB:EtOH (65:5:30) vs. LOIB:CAB:DMSO (65:5:30). EtOH did not seem to cause expansion of expanding ECells, whilst DMSO did. Here, the swelling ratio (on the y-axis) was defined as the weight of the ECell + absorbed water at the specific time point (x-axis), divided by the initial injection mass of the ECell.

Varying the type of solvent from DMSO to EtOH caused a dramatic drop in the maximum expansion capacity as seen in Figure 3, at 25 °C.

Figure 4 shows the impact of CAB content on the expansion property of expanding ECells at 25 °C. Testing 1) LOIB:CAB:DMSO (65:2.5:32.5), 2) LOIB:CAB:DMSO (65:5:30), 3) LOIB:CAB:DMSO (62.5:7.5:30) showed that there was no significant difference in terms of the maximum expansion capacity, but the 7.5 % w/w CAB formulation collapsed significantly slower. Thus, higher CAB fractions lead to slower collapse.

Varying the w/w fraction of CAB(12 kDa) showed that there was no significant difference between expansion of ECells with 2.5, 5 or 7.5% CAB (12kDa) at 25 °C (see Figure 4). The 7.5 % w/w CAB had a longer plateau phase than the other tested compositions.

Figure 5 shows the impact of CAB content on the expansion property of expanding ECells at 37 °C. Formulations of expanding ECells, LOIB:CAB:DMSO (65:5:30) and LOIB:CAB:DMSO (32:18:50) were tested for to compare their expansion property, at 37 °C. There was a significant difference in terms of the maximum expansion capacity. Here, the swelling ratio (on the y-axis) was defined as the weight of the ECell + absorbed water at the specific time point (x-axis), divided by the initial injection mass of the ECell.

Testing 18 % vs. 5 % w/w CAB at 37 °C showed that there is a limit to how much CAB can be contained before reducing the maximum expansion capacity (see Figure 5). Thus, there is a balance between maximum expansion capacity and collapse rate, and these should be optimized for desired tissue-engineering purposes.

### Conclusion

Several factors influence the maximum expansion capacity and subsequent collapse rate of expanding ECells. The major constituent, the carbohydrate ester, dictates the maximum expansion capacity, whereas the co-solvent can influence the expansion capacity and collapse rate. CAB provides mechanical stability and minimize ECell collapse whereas 0 % CAB leads to expansion but collapse as there is no mechanical support. 7.5 % w/w CAB led to the slowest collapse rate, and 2.5 % w/w CAB led to the fastest collapse rate. 7.5 % w/w CAB did not decrease the maximum expansion capacity, but 18 % w/w did.

### Example 2: The effect of temperature on in vitro expansion of expanding ECells

### Aim: To determine whether incubation temperature has an influence on the expanding properties of expanding ECells.

### Materials & Methods

An expanding ECells formulation, LOIB:CAB(12 kDa):DMSO (65:5:30), was made by weighing the w/w fractions of each constituent in a glass vial at the desired mass and sonicated at 75 °C for approximately 2 hours with vortexing every half hour. A depot of 100 µl was injected in 4 ml of Dulbecco's Phosphate-buffered Saline (dPBS) and incubated at 25 or 37 °C, in triplicates, and weighed at various time points.

Each time point was visualized in a graph as the average ratio (mass at time of measurement divided by initial weight, mt/mo) and the standard deviation, seen in Figure 6.

### Results & Discussion

Figure 6 shows the impact of temperature on the expansion property of expanding ECells; 25 *vs.* 37 °C. Varying the temperature under which the expansion of a LOIB:CAB(12 kDa):DMSO (65:5:30) ECell takes place influences the expansion property of the expanding ECell. Here, the swelling ratio (on the y-axis) was defined as the weight of the ECell + absorbed water at the specific time point (x-axis), divided by the initial injection mass of the ECell.

The expansion of ECells at various temperatures is significantly different, as seen in Figure 6. At lower temperatures, expanding ECells have a lower maximum expansion capacity which is reached faster, and the subsequent collapse rate is also faster.

### Conclusion

Lower temperatures confer a lower maximum expansion capacity of expanding ECells and a faster collapse rate of the formed depot, after the peak of expansion has been reached.

### Example 3: The effect of CAB length on in vitro expansion of expanding ECells

### Aim: To investigate the effect of increasing polymer length of cellulose acetate butyrate (CAB) on the expanding property of expanding ECells

### Materials & Methods

CAB at 12- or 30 kDa was formulated as LOIB:CAB:DMSO (60:5:35) w/w fractions. Formulation was done by weighing constituents on a precision scale followed by sonication at 75 °C for 2 hrs, with vortexing every 30^{th} minute.

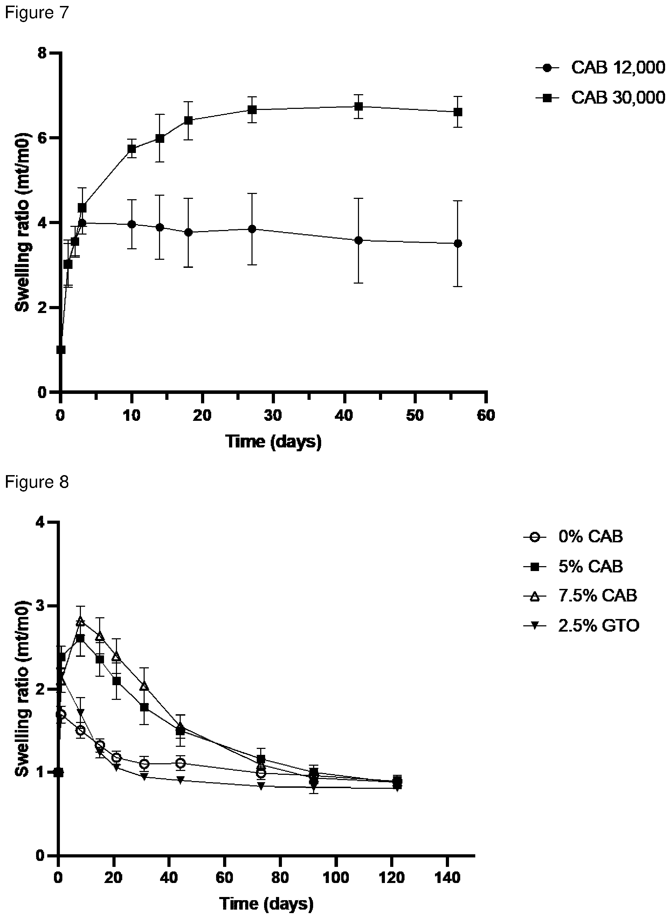

Each formulation was injected in triplicates as 100 µL into 4 mL of Dulbecco's Phosphate-buffered Saline (dPBS) solution, and at each time point (from 0 to 140 days) the dPBS was removed, the expanding ECells was weighed and dPBS was replenished. The weight was noted down and the swelling ratio (mass at time point divided by initial mass, mₜ/m₀) was calculated for each replica. The average expanding ratio ± standard deviation was visualized in Figure 7.

### Results & Discussion

Figure 7 shows the effect of CAB length variation on the expansion property of expanding ECells at 37 °C. Varying the length of CAB polymers in expanding ECells, LOIB:CAB:DMSO (60:5:35), incubated at 37 °C influences the maximum expansion capacity of expanding ECells. Here, the swelling ratio (on the y-axis) was defined as the weight of the ECell + absorbed water at the specific time point (x-axis), divided by the initial injection mass of the ECell.

Expansion of a ECell comprising CAB(12 kDa) showed a lower maximum expansion capacity than a ECell comprising CAB(30 kDa) and reached its maximum capacity faster at 37 °C (see Figure 7).

### Conclusion

Increasing the molecular weight of CAB increases the maximum expansion capacity of expanding ECells at 37 °C.

### Example 4: The effect of CAB and GTO variation on in vivo expansion of expanding ECells

### Aim: To investigate the influence of CAB and GTO variation on expansion of expanding ECells in an in vivo environment

### Materials & Methods

Formulations with varying content of CAB (12kDa) and GTO-containing expanding ECells were made by simply weighing each constituent on a precision scale in a glass vial and sonicating at 75 °C for 2 hours with vortexing every 30^{th} minute. Once the solution clears as a viscous liquid, the formulation is deemed ready for experimental use. For these studies, 10% w/w CLA8 (contrast agent; lactose octa-iodine) replaced 10% w/w LOIB, in comparison to their *in vitro* counterparts, which were described in Examples 1-5. The exact compositions were: LOIB:CLA8:CAB:DMSO (55:10:0:35), LOIB:CLA8:CAB:DMSO (50:10:5:35), LOIB:CLA8:CAB:DMSO (47.5:10:7.5:35), LOIB:CLA8:CAB:GTO:DMSO (47.5:10:5:2.5:35)

Each formulation was injected in 7-weeks old Balb/C mice as 50 µl (left thigh, subcutaneous) and 100 µl (right thigh, subcutaneous) in each respective mouse. Formulations were injected in 4 mice, and at time point t = 14 days, two were euthanized, meaning µCT measurements days 15 and onwards has 6 replicas. At each time point, µCT scans were taken and 3D reconstruction and volume analysis were done using the Inveon Research Workplace software. The average expanding ratio (volume at time point divided by initial volume at day 0, mₜ/m₀) is visualized in Figure 8 with SEM as error bars.

### Results & Discussion

Figure 8 shows the effect of CAB and GTO variation on the *in vivo* expansion property of expanding ECells. Comparing the *in vivo* expansion of four formulations: 1) LOIB:CLA8:CAB:DMSO (55:10:35), 2) LOIB:CLA8:CAB:DMSO (50:10:5:35), 3) LOIB:CLA8:CAB:DMSO (47.5:10:7.5:35), and 4) LOIB:CLA8:CAB:GTO:DMSO (47.5:10:5:2.5:35), it is evident that expansion profiles differ among the groups. Here, the expansion is an average of the computed volume at each time point found through the Inveon Workspace software after µCT imaging, ± SEM. ROIs were manually fitted, and volumes calculated automatically. The swelling ratio is defined as the computed average volume at each time point, divided by the computed volume at t = 0 hrs.

As seen in Figure 8, all formulations expanded in the 37 °C *in vivo* environment and collapsed over time. *In vivo,* there was no significant difference between 5 % or 7.5 % w/w of CAB(12 kDa). 0 % w/w CAB(12 kDa) had a significantly lower maximum expansion capacity than the others, but a collapse rate similar to the formulation with 2.5 % w/w GTO. Both the 2.5 % w/w GTO and 0 % CAB expanding ECells formulations had a significantly faster collapse rate than 5 % and 7.5 % w/w, and their maximum expansion capacity was reached faster. As such, 0 % CAB and 2.5 % GTO should be preferred for applications where a fast collapse rate is important, and *vice versa* for 5 and 7.5 % w/w CAB expanding ECells formulations.

### Conclusion

Addition of 5 or 7.5 % w/w CAB(12 kDa) significantly slows the *in vivo* collapse rate of expanding ECells following the maximum expansion peak. In comparison to 0 % w/w CAB, the peak is also reached slower (t ≈ 1 week, vs. t ≈ 1 day for 0 % w/w CAB). Addition of CAB at 5 or 7.5 % w/w CAB, in comparison to 0 % CAB, also increases the maximum expansion capacity by approximately 100 %, *in vivo.* Addition of 2.5 % w/w GTO in comparison to 0 % GTO (both formulations had 5 % w/w CAB) significantly lowered the maximum expansion capacity, and the time-to-peak expansion of 2.5 % w/w GTO was faster than 0 % GTO. The collapse rate is also significantly faster for expanding ECells formulations containing 2.5 % GTO, *in vivo.*

### Example 5: The effect of varying the carbohydrate ester on in vivo expansion of ECells

### Aim: To investigate the effect of different carbohydrate esters on the in vivo expansion of ECells

### Materials & Methods

Formulations LOIB or LOP-based expanding ECells were made by simply weighing each constituent on a precision scale in a glass vial and sonicating at 75 °C for 2 hours with vortexing every 30^{th} minute. Once the solution clears as a viscous liquid, the formulation is deemed ready for experimental use. For these studies, 10% w/w CLA8 (contrast agent; lactose octa-iodine) replaced 10% w/w LOIB/LOP, in comparison to their *in vitro* counterparts, which were described in Examples 1-5. The final formulations were LOIB- or LOP:CLA8:CAB:DMSO (50:10:5:35).

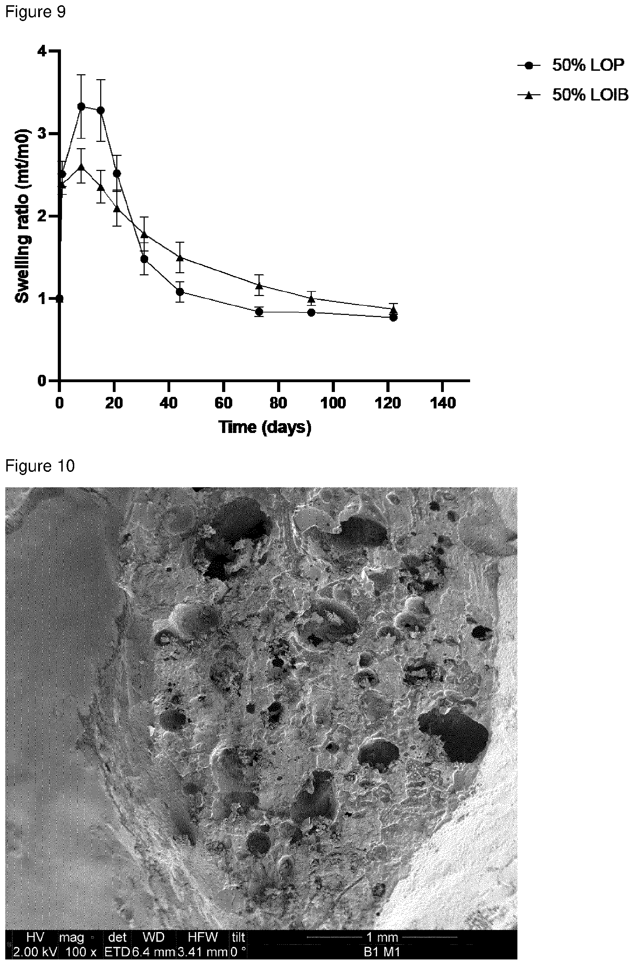

Each formulation was injected in 7-weeks old Balb/C mice as 50 µl (left thigh, subcutaneous) and 100 µl (right thigh, subcutaneous). Each formulation was injected in 4 mice (2 expanding ECells in each mouse = 8 replicas), and at time point t = 14 days, one mouse in the LOIB group and two mice in the LOP group were euthanized, meaning µCT measurements days from day 15 and onward had 6 and 4 replicas, respectively. At each time point, µCT scans were taken and 3D reconstruction and volume analysis were done using the Inveon Research Workplace software. The average expanding ratio (volume at time point divided by initial volume at day 0, mt/mo) is visualized in Figure 9, with SEM as error bars.

### Results & Discussion

Figure 9 shows the effect of LOP- or LOIB-based expanding ECells on its *in vivo* expansion properties. Whether using LOP or LOIB as the main constituent of expanding ECells influences the *in vivo* expansion property. Here, the expansion is an average of the computed volume at each time point found through the Inveon Workspace software after µCT imaging, ± SEM. ROIs were manually fitted, and volumes calculated automatically. The swelling ratio is defined as the computed average volume at each time point, divided by the computed volume at t = 0 hrs.

Using LOP or LOIB as the major backbone of the expanding ECells matrix influences the expansion and collapse properties of expanding ECells. LOP has a higher maximum expansion capacity than LOIB and collapses faster after its peak of expanding. However, there is no significant difference on the time-to-peak when comparing the two formulations.

### Conclusion

Using LOP or LOIB as the main constituent of expanding ECells influences the *in vivo* expansion property. LOP has a higher maximum expansion capacity than LOIB, the time-to-peak is not significantly different but LOP has a significantly faster collapse rate than LOIB.

### Example 6: Microstructures of in vivo-expanded ECells evaluated by cryo-SEM

### Aim: To investigate the microstructures of expanding ECells, following in vivo expansion in mice, by cryogenic scanning electron microscopy (cryo-SEM)

### Materials & Methods

Five formulations of expanding ECells were made for *in vivo* evaluation: 1) LOIB:CLA8:DMSO, (55:10:35), 2) LOIB:CLA8:CAB(12 kDa):DMSO (50:10:5:35), 3) LOIB:CLA8:CAB(12 kDa):DMSO (47.5:10:7.5:35), 4) LOIB:CLA8:CAB(12 kDa):GTO:DMSO (47.5:10:5:2.5:35), 5) LOP:CLA8:CAB(12 kDa):DMSO (50:10:5:35).

Each formulation was injected in 4 Balb/C mice; 50 µl in the left thigh (subcutaneous injection), 100 µl in the right thigh (subcutaneous injection), giving a sample size of n = 8 per formulation. After 14 days, one mouse was euthanized per group for cryo-SEM analysis, and all expanding ECells were taken out after final euthanization at t = 122 days (4 months). Expanding ECells were collected in 5 ml Eppendorf tubes and directly plunged into cold liquid nitrogen to avoid unrelated *ex vivo* collapse.

Cryo-SEM was conducted as described in *Multimodal soft tissue markers for bridging high-resolution diagnostic imaging with therapeutic intervention,* AE Hansen et. Al Science advances 6 (34), 2020.

### Results & Discussion

Figure 10 shows cryo-SEM imaging of an expanding ECells formulation (1): 55:10:35; LOIB, CLA8, DMSO, collected from a mouse after 7 days of *in vivo* expansion and snap-frozen, followed by cryo-SEM imaging. After 7 days of *in vivo* expansion, the expanding ECell formulation of LOIB:CLA8:DMSO (55:10:35) formed a depot as seen in Figure 10. Very large pores were visible in the interior and through to the exterior. The smoother-looking surface on the left-hand side of the picture is an artefact from cryo-SEM imaging (carbon-paste).

From cryo-SEM imaging, it is apparent that expanding ECells contain pores of sizes varying from 10 to 100 µm in diameter. It is also seen that low w/w fractions of CAB confer larger sized pores and more inter-connectedness between pores. *Vice versa,* more CAB reduces the pore sizes and makes less inter-connected pockets throughout the depot matrix.

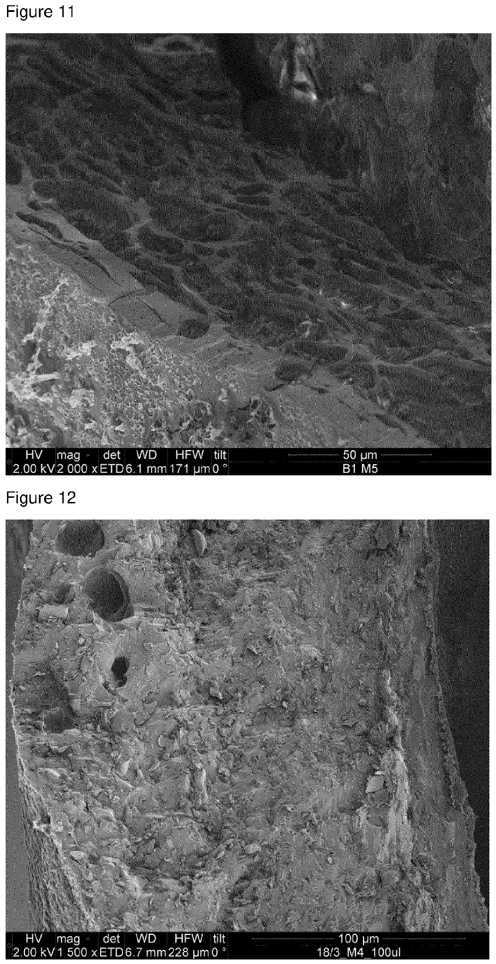

Figure 11 shows cryo-SEM imaging of an expanding ECells formulation (2): LOIB:CLA8:CAB(12 kDa) :DMSO (50:10:5:35), collected from a mouse after 7 days of *in vivo* expansion and snap-frozen, followed by cryo-SEM imaging. After 7 days of *in vivo* expansion, the expanding ECell formulation of LOIB:CLA8:CAB(12 kDa):DMSO (50:10:5:35), formed a depot as seen in Figure 11. Large- and medium-sized pores were visible in the interior and in the exterior. A more densely packed "shell" was apparent on the surface at the width of about 20 µm.

Figure 12 shows cryo-SEM imaging of an expanding ECells formulation (3): LOIB, CLA8, CAB(12 kDa):DMSO (47.5:10:7.5:35), collected from a mouse after 7 days of *in vivo* expansion and snap-frozen, followed by cryo-SEM imaging. After 7 days of *in vivo* expansion, the expanding ECell formulation of LOIB, CLA8, CAB(12 kDa):DMSO (47.5:10:7.5:35), formed a depot as seen in the Figure. Small- and medium-sized pores were visible in the interior and in the exterior. A more densely packed "shell" was apparent on the surface at the width of about 10 µm. Smaller pores were present in this 7.5% CAB formulation relative to the 5 % CAB formulation.

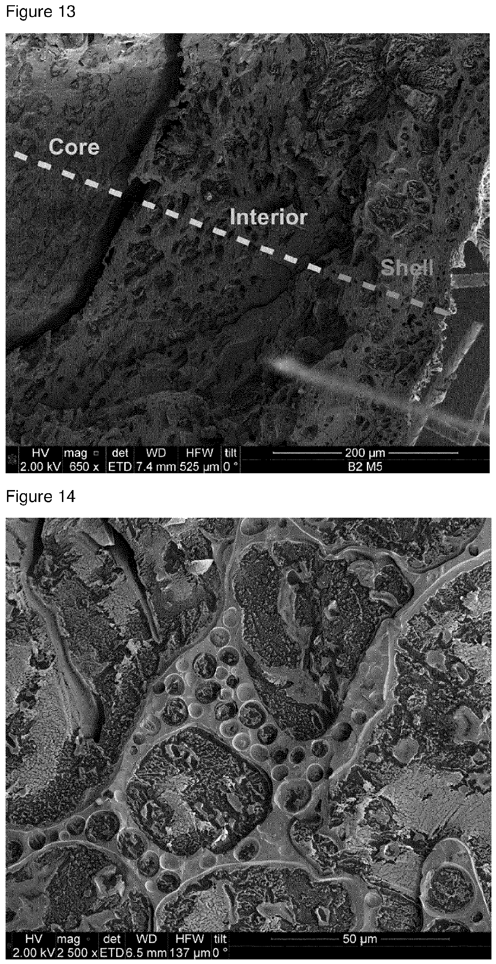

Figure 13 shows cryo-SEM imaging of an expanding ECells formulation (4): LOIB:CLA8:CAB(12 kDa):GTO:DMSO (47.5:10:5:2.5:35), collected from a mouse after 7 days of *in vivo* expansion and snap-frozen, followed by cryo-SEM imaging. After 7 days of *in vivo* expansion, the expanding ECell formulation of LOIB:CLA8:CAB(12 kDa):GTO:DMSO (47.5:10:5:2.5:35), formed a depot in which a cross-section is seen in the Figure. Small- and medium-sized, non-circular pores were visible in the interior and in the exterior. A more densely packed "shell" was apparent on the surface at the width of about 40 µm, and a more densely packed "core". Unlike the previous formulations in this Example, addition of 2.5 % w/w GTO has formed amorphous, rather than circular, pores.

Figure 14 shows cryo-SEM imaging of an expanding ECells formulation (2): LOP:CLA8:CAB(12 kDa):DMSO (50:10:5:35), collected from a mouse after 7 days of *in vivo* expansion and snap-frozen, followed by cryo-SEM imaging. After 7 days of *in vivo* expansion, the expanding ECell formulation of LOP:CLA8:CAB(12 kDa):DMSO (50:10:5:35), formed a depot as seen in the Figure. Large- and medium-sized pores were visible in the interior and in the exterior. A more densely packed "shell" was apparent on the surface at the width of about 20 µm. Unlike the LOIB-based, similar, formulation, medium-sized and smaller pores have formed within the ridges between the larger pores, as seen in the Figure.

### Conclusion

During the process of expansion, ECells form pores. Addition of CAB means relatively smaller pore sizes. At higher CAB fractions (7.5 % CAB rather than 5 %) the pore size generally decreases further. LOP is also shown, in this Example (see Figure 14) to confer the formation of large pores with medium- and small-sized pores in the ridges between the larger pores.

### Example 7: Microstructures of expanding ECells evaluated by Spinning Disc Confocal Microscopy

### Aim: To investigate the microstructures of in vitro-expanded expanding ECells by microscopy, with and without cells

### Materials & Methods

Expanding ECells were formulated as LOIB:CAB(12 kDa):DMSO:Curcumin (65:5:30:0.025), made by simply weighing each excipient as the given w/w ratio on an analytical scale, followed by sonication for 2 hrs at 75 °C, with thorough vortexing every 30^{th} minute. Curcumin was added as a hydrophobic fluorophore specifically for confocal microscopy. ECell injection was made in triplicates, each in 4 ml Dulbecco's Phosphate-buffered Saline and incubated for 7 days at 37 °C in atmospheric air.

C2C12 cancerous, murine myoblast cells were differentiated into osteoblasts by BMP-2 addition, as described by (Rauch et al., 2002), and validated by standard ALP and A-red staining. ECells were transferred by tweezers to Ibidi chambers for confocal microscopy imaging, and 18,000 cells were inoculated per Ibidi chamber for 5 days at 37 °C with atmospheric air containing 5 % CO₂, prior to spinning disc confocal microscopy. At least 5 different sites were imaged and chosen images were selected for presentation in this Example. Immediately prior to imaging, cells were stained with phalloidin-rhodamine, which stains actin, and is excited at 561 nm, whilst 7-AAD was used for staining the nuclei and is excited at 488 nm, and excess dye was washed away before imaging.

### Results & Discussion

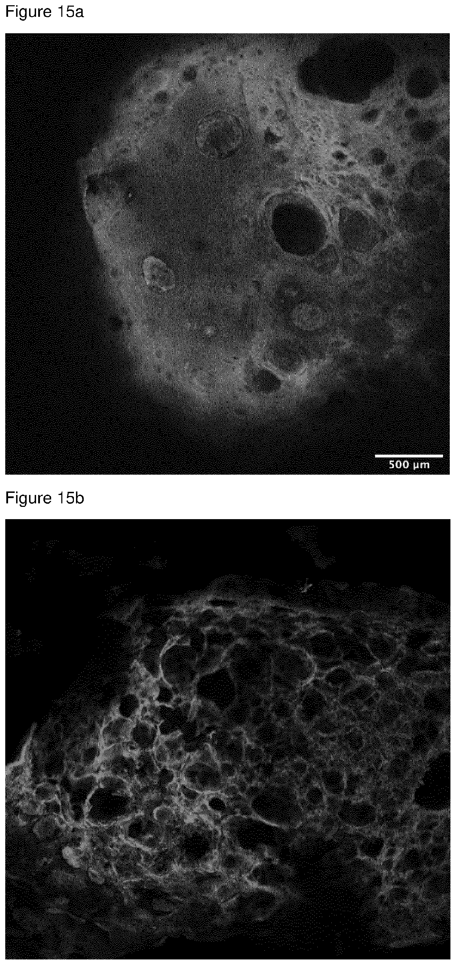

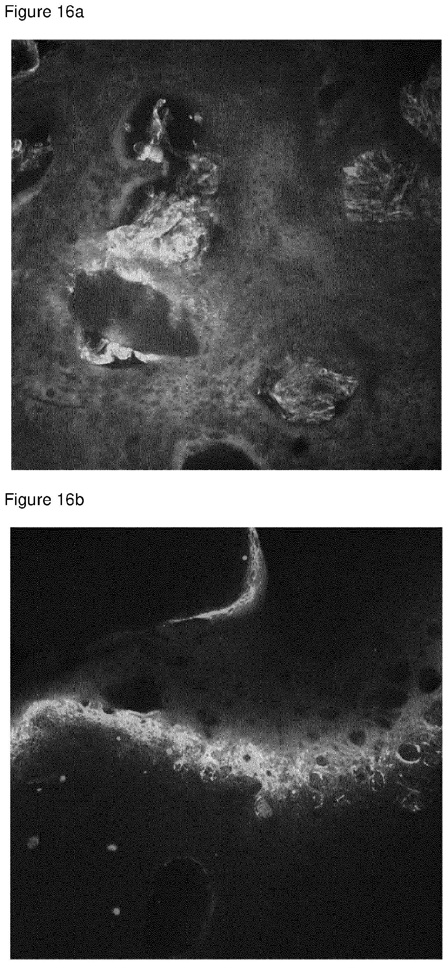

Confocal microscopy allows focal planes of fluorophore excitation, revealing the microstructures of the curcumin-fluorophore-containing expanding ECells (Figure 15-17). With the investigated formulation, pores ranging from 10-650 µm in diameter can be seen on the surface of the expanding ECells going inward, and as seen in Figure 16, osteoblasts can adhere and proliferate on the surface. When cutting open the expanding ECells, the interior also contains a high density of pores also from 10-650 µm, of which only relatively few are large enough for cell migration (Figure 16).

Figures 15a and 15b show *in vitro*-expanded ECells of LOIB:CAB(12 kDa):DMSO (65:5:30) with 0.025% w/w curcumin, imaged by spinning disc confocal microscopy wherein Figure 15a shows the surface and Figure 15b the cross-section. Using confocal microscopy, it is evident that large pores form throughout the exterior into the interior of the expanding ECells (see Figure 15a). Likewise, on the right, large pores have formed throughout the interior of the expanding ECells. Pore sizes ranging, with this formulation, from 10 to 650 µm (0.65 mm) in diameter are visible. These pore sizes are large enough to accommodate ingrowth of human cells.

Figures 16a and 16b *in vitro*-expanded ECells of LOIB:CAB(12 kDa):DMSO (65:5:30) with 0.025% curcumin, incubated for 5 days with osteoblast-differentiated C2C12 cells, imaged by spinning disc confocal microscopy. Staining cells with phalloidin-rhodamine and 7-AAD stain, and by using confocal microscopy, it is evident that the surface of the expanding ECells is teeming with osteoblast cells after 5 days of incubation (see the bright, hair-like structures in Figure 16b), and likewise for the first layer beneath the expanding ECells shell (see the bright structures in Figure 16a). Curcumin is grey, visualizing the expanding ECells matrix itself; the phalloidin-rhodamine causes a brightness in greyscale, and the bright clumps (left and right) is nuclear stain by 7-AAD.

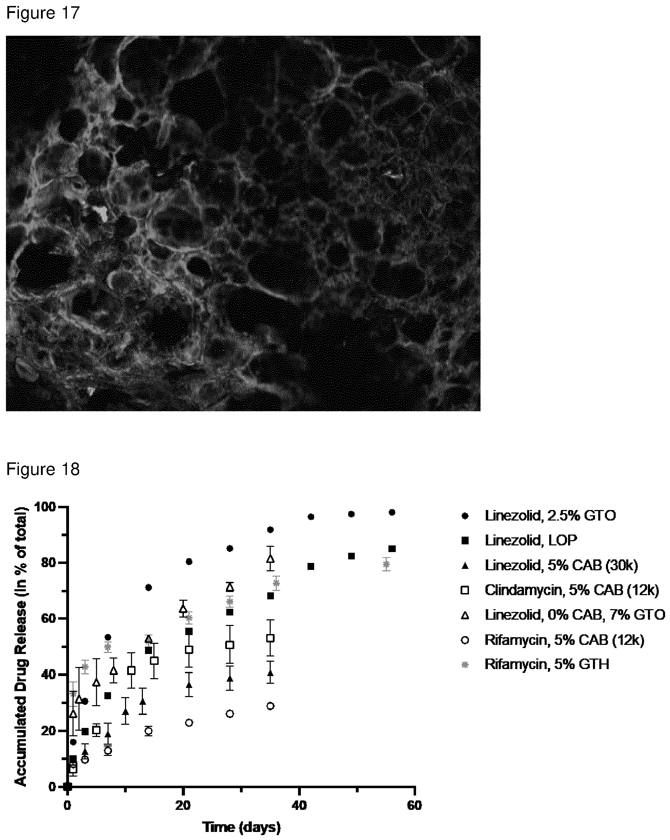

Figure 17 shows a cross-section of *in vitro*-expanded ECell of LOIB:CAB(12 kDa):DMSO, 65:5:30, with 0.025 % w/w curcumin and incubated for 5 days with osteoblast-differentiated C2C12 cells, imaged by spinning disc confocal microscopy. Staining cells with phalloidin-rhodamine, and by using confocal microscopy, it is revealed that a few pores from the exterior allow cell migration to the interior of the expanding ECells, as seen in the cross-section in Figure 17. Curcumin is colored grey, visualizing the expanding ECells matrix itself and the phalloidin-rhodamine causes the bright clumps seen in Figure 17.

### Conclusion

1-week old expanding ECells form pores throughout its exterior and interior matrix. These pores and its surface can accommodate cells which can adhere and proliferate. As such, expanding ECells can function as biocompatible, injectable, cell scaffolds.

### References

Rauch, C., Brunet, A.-C., Deleule, J., Farge, E., 2002. C 2C 12 myoblast/osteoblast transdifferentiation steps enhanced by epigenetic inhibition of BMP2 endocytosis. American Journal of Physiology-Cell Physiology 283, C235-C243. https://doi.org/10.1152/ajpcell.00234.2001

### Example 8: In vitro release of antimicrobials from expanding ECells

### Aim: To characterize the in vitro release profile of antimicrobial compounds from expanding ECells

### Materials & Methods

Three different antimicrobials were tested with varying logP values: Linezolid (logP = 0.64), Clindamycin (logP = 1.04) and Rifamycin SV (logP = 4.17). These were tested in 5 different formulations, in total: LOIB:CAB:DMSO (65:5:30), LOIB:CAB:GTO:DMSO (65:5:2.5:27.5), LOP:CAB:DMSO (65:5:30), LOIB:CAB(30 kDa):DMSO (60:5:35), LOIB:CAB(12 kDa):GTH:DMSO (55:5:5:35)

All formulations were prepared by weighing each excipient on a precision scale to the chosen w/w fraction and sonicating the solution at 75 °C for 2 hrs, with vortexing every 30^{th} minute.

Each formulation was loaded with antimicrobial in varying concentrations (see Table 1) and was injected as ≈100 µl into 4 ml Dulbecco's Phosphate-buffered Saline (dPBS), in a glass vial, and incubated at 37 °C in atmospheric air, only taken out when samples were collected and new dPBS was added. Sample collection proceeded by removing all dPBS possible with a pipette, weighing the expanding ECells, then saving 1 ml for analysis, returning the rest, and adding new dPBS until 4,000 mg of water was present.

At each chosen time point, 1 ml of the release medium was collected and RHPLC analysis was carried out. For this purpose, a C18 column with a 5-100% solution B gradient (99.99% MeCN w. 0.01% TFA) over 7 minutes, followed by 1 minute 100 % B, then 1 minute 0 % B, was utilized. Solution A was an aqueous mixture of 5 % v/v MeCN and 0.01% TFA. The AUC of Linezolid was measured at 250 nm, 280 nm for Rifamycin and 202 nm for Clindamycin. Rifamycin splits into several peaks over time, and the total AUC of all peaks were used for quantification and standard curve generation.

### Results & Discussion

Figure 18 shows *in vitro* release of antimicrobial compounds from expanding ECells over time (in days). Testing the *in vitro* release rate of linezolid, clindamycin or rifamycin from various expanding ECells formulation shows that addition of triglycerides (GTO, GTH) significantly increases release rate, and that more hydrophobic compounds (rifamycin > clindamycin) have a relatively slower release rate.

Addition of GTO or GTH significantly increased the release rate, and as seen in Example 1, the collapse rate increases when adding triglycerides. Comparing Rifamycin and Clindamycin release from 5% CAB (12k) in Figure 18 shows that compounds with a higher LogP value (i.e., more hydrophobic) has a slower release rate.

**Table 1. A list of tested antibiotics, their concentrations, and expanding ECells formulations used for in vitro release. Various expanding ECells formulations loaded with antibiotics; linezolid, clindamycin and rifamycin, were tested in vitro, as shown in Figure 18.**

| Formulation | Antibiotic concentration |
|---|---|
| 65:5:2.5:27.5 | 15 mg/g, Linezolid |
| LOIB:CAB(12 kDa):GTO:DMSO | |
| 65:5:30 | 15 mg/g, Linezolid |
| LOP: CAB(12 kDa):DMSO | |
| 60:5:35 | 30 mg/g, Linezolid |
| LOIB: CAB(30 kDa):DMSO | |
| 65:5:30 | 10 mg/g, Clindamycin |
| LOIB:CAB(12 kDa):DMSO | |
| 65:5:30 | 35 mg/g, Rifamycin |
| LOIB: CAB(12 kDa):DMSO | |
| 55:5:5:35 | 20 mg/g, Rifamycin |
| LOIB: CAB(12 kDa):GTH:DMSO | |

### Conclusion

Addition of triglycerides to expanding ECells increases the release rate, and more hydrophobic compounds have a slower release rate.

### Example 9: In vitro release of MMP-inhibitors from expanding ECells

### Aim: To characterize the in vitro release profile of small molecule inhibitors from expanding ECells

### Materials & Methods

The *in vitro* release profile of three inhibitor compounds were elucidated from a LOIB:CAB(12 kDa):DMSO (65:5:30) formulation. Formulations were made by weighing each excipient for the expanding ECells at their w/w fraction followed by sonication at 75 °C for 2 hrs, with thorough vortexing every 30^{th} minute. Loading of inhibitors were done by adding the desired concentration in mg per g of expanding ECells and stirring on a magnetic stirrer at room temperature until the solution became clear.

The three tested compounds were MMP-1 inhibitor Batimastat (logP = 3.27), MMP-2 and MMP-9 inhibitor, SB-3CT (logP = 2.75), and lastly, inhibitor of gp-130, which lowers IL-6 production, LMT-28 (logP = 3.71).

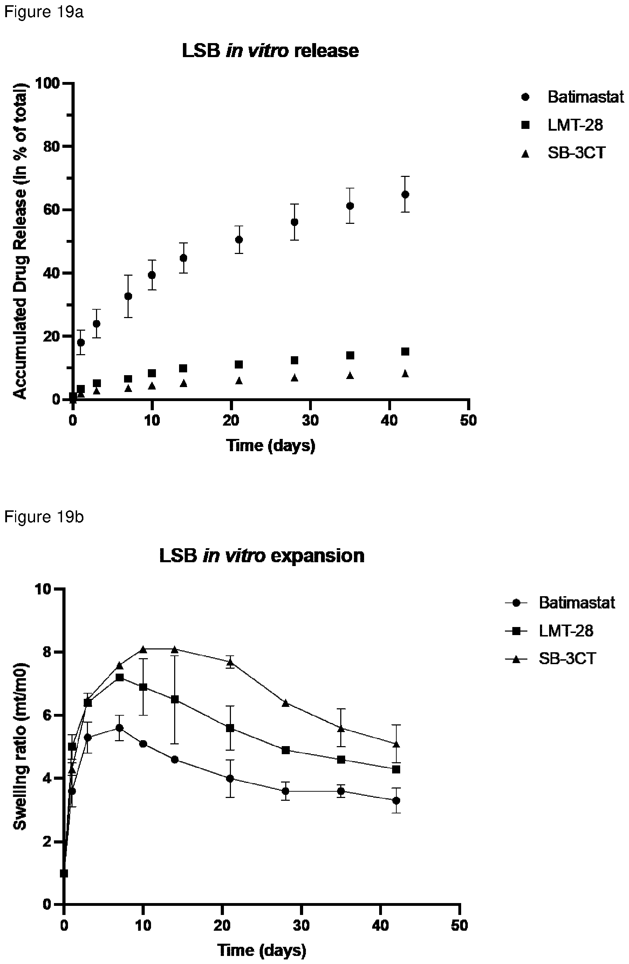

Each compound was loaded as 5 mg/g in the expanding ECell formulation and injected in 4 ml Dulbecco's Phosphate-buffered Saline (dPBS) as ≈ 100 µl, in triplicates, and incubated at 37 °C. At chosen time points (see Figure 19a), all dPBS was removed by pipetting, the expanding ECells + potentially absorbed water was weighed, and its weight noted down, 1 ml dPBS was collected for release quantification, and the remaining dPBS + fresh dPBS to a total of 4 ml (4 g) was added.

Weight data was used to generate a graph of the relative expanding ratio as a function of time (Figure 19b), and from the 1 ml collected, RHPLC analysis was carried out. For this purpose, a C18 column with a 5-100% solution B gradient (99.99% MeCN w. 0.01% TFA) over 7 minutes, followed by 1 minute 100 % B, then 1 minute 0 % B, was utilized. Solution A was an aqueous mixture of 5 % v/v MeCN and 0.01% TFA. The AUC of Batimastat and LMT-28 was measured at 202 nm, while SB-3CT had its maximum absorption at 201 nm, which wavelength was also used for its AUC measurements.

### Results & Discussion

The tested MMP inhibitors and gp-130 inhibitor was released *in vitro* in varying degrees. After 42 days, the Batimastat-containing expanding ECells had released about 60 % of its cargo, 10 % for LMT-28 and about 5% for SB-3CT. Interestingly, as the same expanding ECells formulation was used for all three drugs and seeing as the maximum expansion capacity of expanding ECells is lowest for the drug with the highest release rate, evidently, the dissolved drug's properties influence the ECells' expansion profile.

Figure 19a shows the *in vitro* release of Batimastat, LMT-28 and SB-3CT from an expanding ECell formulation at 37 °C, in dPBS and Figure 19b shows the expanding of each expanding ECells over time during the course of the study. *In vitro* release showed that Batimastat had a significantly higher release from an identical formulation relative to LMT-28 or SB-3CT. Even though SB-3CT had the lowest logP value. It also observed that the formulation with the lowest release had the highest maximum expansion capacity (Figure 19a and 19b).

### Conclusion

Dissolved drug content influences the expansion profile of expanding ECells, and it is possible to sustainably release the tested compounds *in vitro.*

### Example 10: Solubility of CAB, CP and PEG in Expanding ECells

### Aim: To investigate the solubility of cellulose-acetate-butyrate (CAB), cellulose propionate (CP) and pegylated lipids in expanding ECells

### Materials & Methods

Cellulose-acetate-butyrate (CAB) with a molecular weight of 12 kDa (12,000 g/mol; unless stated otherwise) was weighed on a precision scale and added LOIB and DMSO at varying w/w fractions, as follows: LOIB:CAB:DMSO (64.5:0.5:35), LOIB:CAB:DMSO (64:1:35), LOIB:CAB:DMSO (65:2.5:32.5), LOIB:CAB:DMSO (65:5:30), LOIB:CAB:DMSO (62.5:7.5:30), LOIB:CAB:DMSO (60:10:30), LOIB:CAB:DMSO (32:18:50), LOIB:CAB(30 kDa):DMSO (60:5:35), LOIB:CAB(70 kDa):DMSO (60:5:35), LOIB:CP(70 kDa):DMSO (60:5:35), LOIB:CAB:DSPE-PEG2k:DMSO (64.5:5:0.5:30), LOIB:CAB:DPPE-PEG2k:DMSO (64.5:5:0.5:30), LOIB:CAB:DPPE-PEG2k:DMSO (64:5:1:30)

After addition of LOIB and DMSO, each ECell formulation was sonicated at 75 °C for 2 hrs and vortexed every 30^{th} minute.

### Results & Discussion

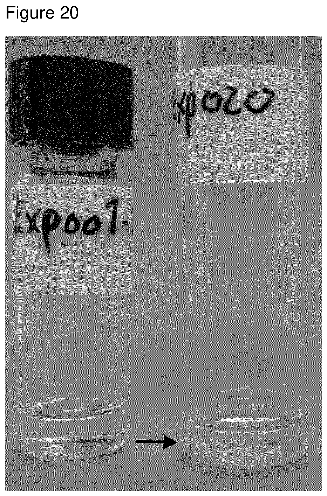

All tested expanding ECells were a nice homogenous, translucent, stable solution after dissolution of CAB (see Figure 20), CP, DPPE-PEG2k or DSPE-PEG2k, and remained so after staying at room temperature for at least 24 hrs. Formulations composed of CAB (12 kDa) and DSPE/DPPE-PEG have also been tested in formulations containing 5 or 10 % w/w lactose-octa-iodine (CLA8) contrast agent, which did not affect the solubility at the listed w/w fractions of the mentioned polymers.

Figure 20 shows dissolution of 5 % w/w CAB in a LOIB:DMSO ECell (vial labeled Exp001) 5 % w/w CAB is soluble in 65 % w/w LOIB and 30 % w/w DMSO. On the right (vial labeled Exp020) Cellulose Acetate (Mₙ = 30,000) at 5 % w/w in the same formulation of LOIB:DMSO was insoluble, as the solution did not become translucent after sonication, but rather, appeared opaque.

### Conclusion

CAB at 12 kDa can easily be dissolved in expanding ECells at the listed formulations of 0.5, 1, 2.5, 5, 7.5, 10 and 18 % w/w concentrations; and 30- or 70 kDa CAB can be dissolved at 5 % w/w. DSPE- and DPPE-PEG2k was dissolvable at 0.5 and 1 % w/w. CP (70 kDa) was soluble at ≤5 % w/w.

### Example 11: Solvents governs the expansivity of ECell

### Aim: Display differences in solvent impact on the expansivity of ECell.

### Materials & Methods

LOIB:CAB ECell formulations with Ethanol (EtOH), Dimethyl sulphoxide (DMSO), Dimethyl formamide (DMF), N-methyl pyrrolidone (NMP), Propylene carbonate (PC), Benzyl alcohol (BnOH) or Butyl acetate (BuAc) as solvent (LOIB:CAB (Mn 12000):Solvent 67.5:2.5:30) were formulated. These ECells were prepared to study eventual surface and expansion effects of the specific solvent type on LOIB:CAB compositions. ECell formulations were prepared in 900 mg aliquots. 12 h stability at room temperature (RT) was investigated and documented in images. Only soluble formulations of appropriate viscosity were injected as gel depots into PBS (100 µL, for visual inspection).

Duplicates consisting of 100 µL ECell depots of each clear formulation were injected into 2 mL PBS buffer for inspection. Samples were kept at 37°C, and images of each injected gel depot composition were recorded at 1.5 h and 2 days and 7 days post injection (pi) to investigate and document effects of solvent type on the depots.

### Results and Discussion

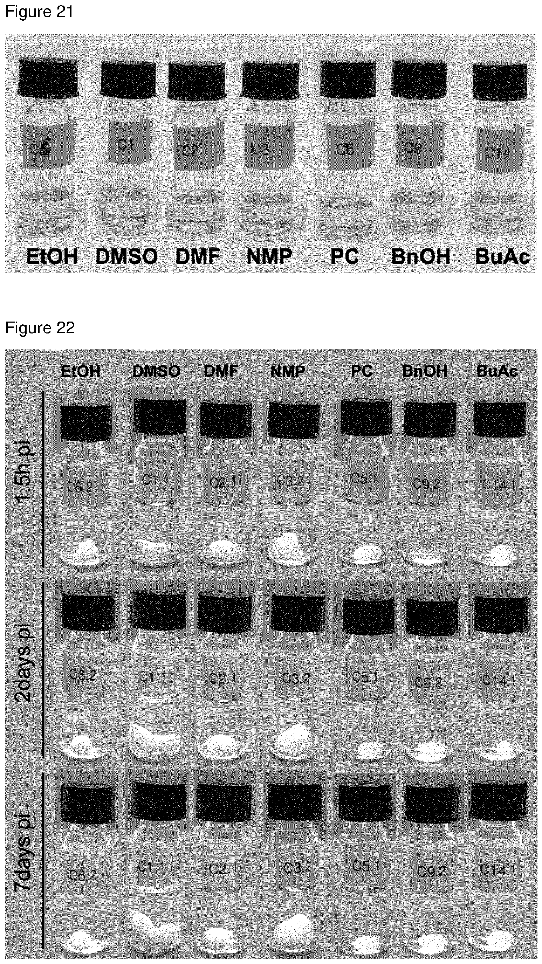

Figure 21 shows images of ECells compositions based on different types of solvent. Homogeneous LOIB:CAB:solvent (67.5:2.5:30) formulations were produced for testing of expansion.

ECell compositions were prepared as homogeneous transparent solutions (Figure 21) that were injected into PBS buffer. Images were recorded as function of time documenting the expansion of the ECells. Here NMP, DMSO and DMF (polar, aprotic solvents) were found to cause expansion of the ECell, i.e. the ECell size expands in size from 1.5h to 2days and 7days and is generally larger than non-expanding ECells at 1.5h pi. Compositions comprising EtOH, PC, BnOH and BuAc did not expand, as judged by the eye (Figure 22).

Figure 22 shows images of expanding ECells comprising different types of solvent. Homogeneous LOIB:CAB:solvent (67.5:2.5:30) formulations were produced for testing of expansion. Each composition was injected into buffer and the expansion was recorded 1.5h, 2days and 7 days post injection (pi). The samples were stored at 37°C for the duration of the experiment.

### Conclusion

The type of solvent governs the expansion properties of ECell, and the polar aprotic solvents DMSO, NMP and DMF were found to promote expansion.

### Example 12: Solubility of polymers and their impact on ECell expansion

### Aim: Investigate the impact of polymer type on the expansion of ECells.

### Materials and Methods:

2.5% of different polymers were formulated in base compositions LOIB:Polymer:DMSO 67.5:2.5:30. As control, a formulation without polymer ("no polymer control") was made and injected for comparison, in order to study eventual surface and expansion effects of the specific polymer component on the injected composition. A non-expanding control was further included with the composition LOIB:CAB(Mn 12000):PC (67.5:2.5:30). Gel formulations containing each polymer type were prepared in 600 mg aliquots. Overnight stability at r.t. was investigated and documented in images. Only soluble formulations were injected as gel depots into PBS.

The following polymers were tested (Molecular weights listed are generally average number molecular weights, Mn. Abbreviations used in figures are given below in parantheses after each name, if different from the ones written): Poly D,L lactide-co-glycolide 75:25, 75-115 KDa, Ester terminated (PLGA ester). Poly D,L lactide-co-glycolide 75:25, 4-15 KDa, acid terminated (PLGA-COOH short). Poly D,L lactide-co-glycolide 75:25, 10-18 KDa, acid terminated (PLGA-COOH medium). Poly-L-lactide (PLA), 10-18 KDa, ester terminated (PLA ester medium). PLA, 18-28 KDa, ether terminated (PLA ether medium). PLA, 2 KDa. PLA, 50 KDa. Alpha -tocopherol PEG-1000 succinate (acid terminated) (VitE-PEG1K). Polypropylene glycol) monobutyl ether (PPG), 2.5 KDa. PCL-PEG-PCL (1:1:1 KDa). Polycaprolactone diol (PCL Diol), 2 KDa. Polyethylene glycol methyl ether (PEG-OMe), 1KDa. PEG, 1.5 KDa. PEG(2000)-C18, 2 KDa. Polyethylene imine, branched (bPEI), 600 Da. bPEI, 1.2KDa. Cellulose acetate butyrate (CAB), 12KDa. CAB, 30 KDa. CAB, 70 KDa. Cellulose propionate (CP), 70 KDa. Celluose, acetate propionate (CAP), 75 KDa.

Duplicates consisting of 100 µL gel depots of each clear formulation were injected into 2 mL PBS buffer. Samples were kept at 37°C, and images of the LOIB:DMSO and LOIB:CAB:PC controls vs LOIB:Polymer:DMSO gels side-by-side were taken at 1.5 h and 2 days and 7 days post injection to investigate and document effects of polymer on eventual swelling of the markers

### Results and Discussion

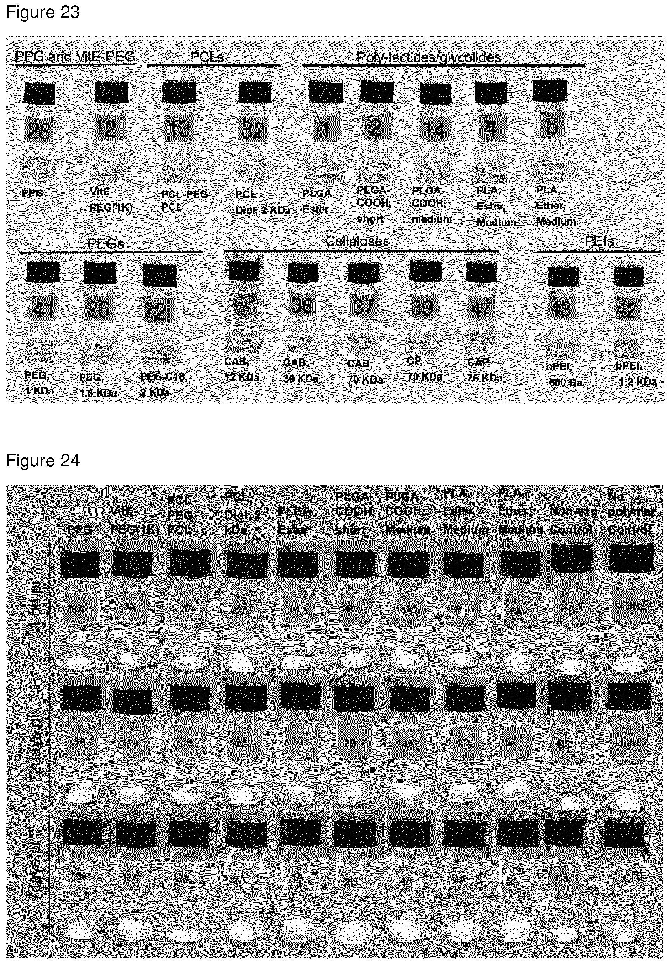

Figure 23 shows images of homogeneous ECell compositions comprising 2.5%w/w of various polymers, such as PPG, VitE-PEG, PCL-PEG-PCL, PCL Diol 2 kDa, PLGA Ester, PLGA-COOH short, PLGA-COOH medium, PLA Ester medium, PLA Ether medium, PEG 1kDa, PEG 1.5kDa and PEG-C18 2kDa, CAB 12 kDa, CAB 30 kDa, CAB 70 kDa, CP 70 kDa, CAP 75 kDa, bPEI 600 Da, and bPEI 1.2 kDa. Base composition of the ECell formulation was LOIB:Polymer:DMSO 67.5:2.5:30.

Homogeneous and transparent ECell compositions comprising 2.5% w/w of various polymers were prepared successfully as displayed in Figure 23.

The ECell formulations were injected into PBS and images of the ECells were recorded at 1.5h, 2 days and 7 days post injection (pi) upon incubation at 37°C (Figure 24-25).

The ECell composition containing no polymer (LOIB:DMSO formulation) was found to expand and form a semi-transparent structure comprising visibly appreciable water bubbles at day 7 (Figure 24-25). This structure was partially preserved for compositions including the PPG, PEG 1kDa, PEG 1.5kDa and PEG-C18 2kDa polymers. The ECells containing the remaining polymers, except PEG-PCL-PEG, formed opaque depots that all expanded compared to the non-expanding control. The expansion of ECells containing CAP 75kDa expanded less than CAB 70kDa, which may be attributed to difference in both polarity (acetate:propionate instead of acetate:butyrate) and % ester functionalization between the two polymers. Differences in global shape was apparent for the different depots, where some appeared spherical, and others had an irregular in shape.

Figure 24 shows images displaying expanding ECells comprising different polymers, such as PPG, VitE-PEG, PCL-PEG-PCL, PCL Diol2 kDa, PLGA Ester, PLGA-COOH short, PLGA-COOH medium, PLA Ester medium, and PLA Ether medium. Base composition of the ECell formulation was LOIB:Polymer:DMSO 67.5:2.5:30 injected into PBS. Expansion was conducted at 37°C and images were recorded 1.5h, 2 days and 7 days post injection (pi).

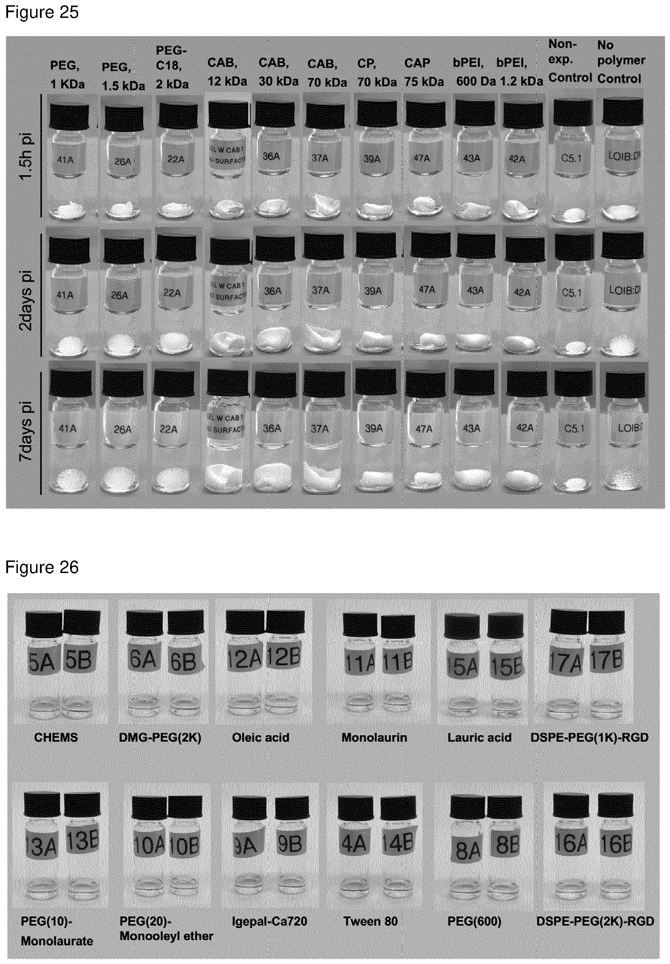

Figure 25 shows images displaying expanding ECells comprising different polymers, such as PEG 1kDa, PEG 1.5kDa and PEG-C18 2kDa, CAB 12 kDa, CAB 30 kDa, CAB 70 kDa, CP 70 kDa, CAP 75 kDa, bPEI 600 Da, and bPEI 1.2 kDa. Base composition of the ECell formulation was LOIB:Polymer:DMSO 67.5:2.5:30 injected into PBS. Expansion was conducted at 37°C and images were recorded 1.5h, 2 days and 7 days post injection (pi).

### Conclusion

ECell containing no polymer (LOIB:DMSO formulation) expanded and formed large water bubble structures. Incorporation of polymers did in most cases not prevent expansion of the ECell but instead affected the global shape of the formed depot, mostly leading to expanded depots compared to the control.

### Example 13: Solubility of lipids and their impact on ECell expansion

### Aim: Demonstrate solubility of lipids and surfactants in ECell and evaluate their impact on the ECell expansion.

### Materials & Methods

Different surfactants were formulated in expandable (w CAB, Mn 12000) vs non-expandable (w GTH) ECell formulations in a concentration of 1% w/w. Base formulations used: LOIB:CAB:DMSO:Surfactant 66.5:2.5:30:1 (expandable) and LOIB:GTH:DMSO:Surfactant (69:10:20:1) (non-expandable), except formulations with RGD lipids where 0.1% surfactant and 0.9% extra DMSO was added. All formulations were designed to have approximately same solvent vs carbohydrate content, hereby making studied effects on the formulations comparable. Gel formulations containing each surfactant were prepared in 600 mg aliquots. Overnight stability at r.t. was investigated and documented in images presented in Figure 26. Only soluble formulations were injected as gel depots into PBS.

Duplicates consisting of 100 µL gel depots of each ECell formulation were injected into 2 mL PBS buffer. Samples were incubated at 37°C, and images of a non-expandable vs an expandable ECell containing the same surfactant side-by-side were taken at 1.5 h and 2 days and 7 days post injection to investigate and document surfactant effects on ECell expansion.

### Results & Discussions

Homogeneous ECell compositions, with and without 2.5% CAB polymer, were prepared containing 1% w/w of either CHEMS, DMG-PEG2K, Igepal Ca-720, PEG(20)-monooleyl ether, Monolaurin, Oleic acid, Lauric acid, PEG(10)-monolaurate, Tween 80 or 0.1% of DSPE-PEG(2000)-RGD, DSPE-PEG(1000)-RGD and formulations are shown in Figure 26

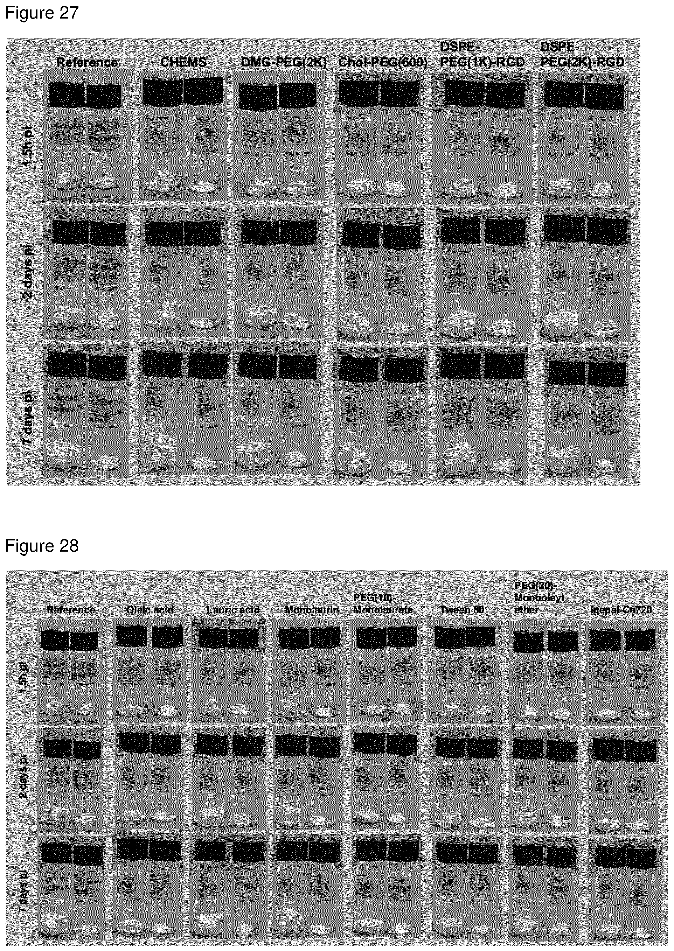

The in vitro expansivity of the ECells were tested by injection of the formulations into PBS buffer followed by incubation at 37°C. Images of the expanding ECells were recorded 1.5hour, 2 days and 7 days post injection (pi), which are presented in Figure 27-28.

Figure 26 shows solubility of lipids/surfactant in ECell compositions. Transparent and homogeneous LOIB:CAB:DMSO:Surfactant 66.5:2.5:30:1 (left vials labelled A in the figure), and LOIB:GTH:DMSO:Surfactant 69:10:20:1 (right vials labelled B in the figure) ECell fomulations comprising varying surfactants. RGD functionalized lipids were added in 0.1%w/w as an exception.

Figure 27 shows expanding ECell compositions comprising lipids/surfactants. Expansion of LOIB:CAB:DMSO:Surfactant 66.5:2.5:30:1 (left vials labelled A.1 in the figure), and LOIB:GTH:DMSO:Surfactant 69:10:20:1 (right vials labelled B.1 in the figure) ECell fomulations, comprising varying surfactants, as a function of time (1.5 hours, 2 days and 7 days). The Reference composition was LOIB:CAB:DMSO 67.5:2.5:30 (left vial), and LOIB:GTH:DMSO 70:10:20 (right vial).

The ECell expansion was relatively similar to the reference for compositions containing Chol-PEG(600), CHEMS, DSPE-PEG(1k)-RGD, DSPE-PEG(2k)-RGD, Lauric acid, monolaurin and slightly reduced for DMG-PEG(2K), oleic acid, PEG(10)-monolaurate, Tween80 or Igepal-Ca720. Some ECells without CAB but containing CHEMS, Tween80 or Igepal-Ca720 did show a slight flattening of the depot compared to reference, which may indicate reduced interfacial tension.

Figure 28 shows expansion of ECell compositions comprising lipids/surfactants. Expansion of LOIB:CAB:DMSO:Surfactant 66.5:2.5:30:1 (left vials labelled A.1 in the figure), and LOIB:GTH:DMSO:Surfactant 69:10:20:1 (right vials labelled B.1 in the figure) ECell fomulations comprising varying surfactants as a function of time (1.5 hours, 2 days and 7 days). The Reference composition was LOIB:CAB:DMSO 67.5:2.5:30 (left vial), and LOIB:GTH:DMSO 70:10:20 (right vial).

### Conclusion

A range of lipids and surfactant were successfully embedded in ECell compositions with and without CAB polymer. The expansion of ECell was similar to slightly reduced for all compositions containing lipids/surfactants.

### Example 14: CT and MRI contrast of expanding ECells

### Aim: Evaluation of radiographic contrast and magnetic resonance signal of expanding ECell formulations LOIB:CLA-8:DMSO (55:10:35) and LOIB:CLA-8:CAB:DMSO (47.45:10:7.5:35)

### Materials & Methods

ECell compositions LOIB:CLA-8:DMSO (55:10:35) and LOIB:CLA-8:CAB:DMSO (47.45:10:7.5:35) were injected subcutaneously in both sides of the flank of balb/CJrj female mice. Injection was performed using a 23 Ga. needle, and a 1 mL syringe. At 15 min, 4 hours, 24 hours, 1 week and 2 weeks after injection CT scans were performed (Inveon, Siemens Medical, 65kV 400mAs) and XX days a MR scan was performed (Bruker, Pharma scan 7T, T1 and T2 turbo scans). For all scans mice under general anesthesia (sevoflurane 3-5% in 60%O₂/40%air). Images were evaluated in commercial software (Inveon, Siemens medical and Horos 3.3.6).

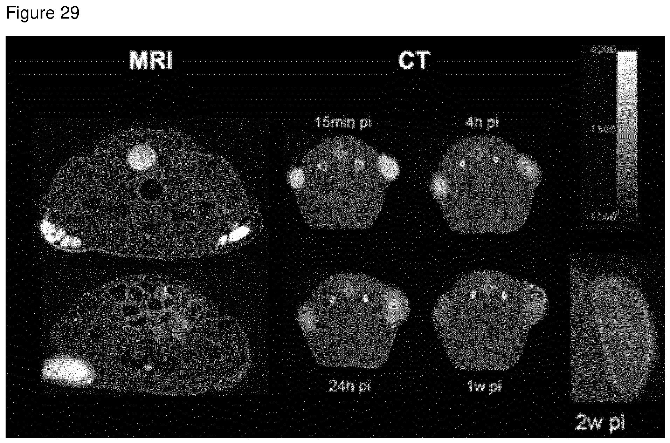

Figure 29 shows MRI and CT of ECell injected subcutaneously in mice. Left column; T2 weighted MRI images (axial slices) acquired 14 days after injection of ECell compositions LOIB:CLA-8:DMSO (55:10:35) (top) and LOIB:CLA-8:CAB:DMSO (47.45:10:7.5:35) (bottom). Right image serie; CT scan images (axial slices) of the LOIB:CLA-8:CAB:DMSO (47.45:10:7.5:35) composition at indicated time points after injection.

### Results

The ECells increased in volume and decreased in radiodensity over the period investigated. On MRI scans the ECells display a high level of contrast on T2 weighted images. The LOIB:CLA-8:CAB:DMSO (47.45:10:7.5:35) displayed the highest increase in volume whereas the LOIB:CLA-8:DMSO (55:10:35) displayed the most well circumscribed and highest MRI contrast on T2 weighted images.

### Conclusion

ECell enlarge in volume in vivo as observed in laboratory evaluations. The observed enlargement is associated with a reduced radiographic contrast. The MRI contrast levels demonstrate that ECell can serve as a marker technology for MR imaging purposes.

## Claims

1. A composition comprising:
- at least one gel-forming hydrophobic carbohydrate ester or an analogue thereof based on mono-, di-, and tri-saccharides, or mixtures thereof;
- at least one polymer; and
- at least one solvent being mixable with water;
wherein the composition is a ECell being a hydrophobic gel-depot having the ability to take up water by means of said at least one solvent being mixable with water and having the ability to self-expand by formation of water pores, channels, and/or cavities.

2. The composition according to claim 1, wherein the gel-forming carbohydrate ester is a carbohydrate ester analogue based on glucose, fructose, galactose, sibose, xylose, sucrose, lactulose, maltose, trehalose, cellobiose, chitobiose, isomaltise, nigerotriose, maltotrios, melezitose, maltotriulose, raffinose and kestose, or a combination thereof.

3. The composition according to claim 1 or 2, wherein the gel-forming carbohydrate ester is selected from sucrose acetate isobutyrate (SAIB), sucrose octapropionate (SOP), sucrose octaisobutyrate (SOIB) sucrose octabenzoate (SuBen), lactose octapropionate (LOP), lactose octaisobutyrate (LOIB), methoxy- lactose octaisobutyrate (MeOLOIB), methyl hepta-O-isobutyryl-α,β-lactoside (MeLOIB), lactose octabenzoate (LacBen), trehalose octapropionate (TOP), trehalose octaisobutyrate (TOIB), trehalose octabenzoate (TreBen), rafinose undecaisobutyrate (RUIB), and rafinose undecabenzoate (RaBen), or a combination thereof;
preferably the gel-forming carbohydrate ester is selected from LOP, LOIB, MeLOIB, SOP, SAIB, SOIB, TOP and TOIB, or a combination thereof.

4. The composition according to any of claims 1-3, wherein the polymer is selected from polyethyleneimine (PEI), polylysine (PLL), polyarginine (PAA), Chitosan, Cellulose, Poly(2-ethyl-2-oxazoline) (ULTROXA), Diethylaminoethyl-dextran (DEAE-dextran), dendritic polyamidoamine (PAMAM) and [poly(N,N-dimethylaminoethyl methacrylate] (PDMAEMA), polylactic acid (PLA), poly(lactic-co-glycolic acid) (PLGA) and Poly(N-isopropylacrylamide) (PNIPAM), Poly D,L lactide-co-glycolide, PLGA-PEG-PLGA, Poly lactic acid (PLA), poly(lactic-co-glycolic acid) (PLGA), Polyethylene glycol (PEG), Polycaprolactone, Polycaprolactone diol, Poly(methyl methacrylate) (PMMA), polyvinyl pyrrolidone (PVP), Poly-allylamine, b-PEI (branched polyethylene imine), I-PEI (linear polyethylene imine),Poly(L-lactide) amine terminated, Trimethyl chitosan, Chitin Potassium hyaluronate (HA), Alpha tocopherol, Polypropylene glycol) monobutyl ether, PCL-PEG-PCL, Polycaprolactone diol, PEG-OMe, Carrageenan, 2-Diethylamino-etyl cellulose (DEAE cellulose), Cellulose acetate (CA), Ethyl Cellulose (EC), Methyl Cellulose (ME), Cyanoethyl cellulose, Cellulose acetate phthalate (CaPh), Cellulose acetate butyrate (CAB), Cellulose propionate (CP), Cellulose butyrate (CB), Celluose acetate propionate (CAP), 2-Hydroxyethyl cellulose, Hydroxypropyl cellulose, (Hydroxypropyl)methyl cellulose, and Sodium carboxymethyl cellulose, or a combination thereof;
preferably the polymer is selected from polyethyleneimine (PEI), Ethyl Cellulose (EC), Cyanoethyl cellulose, Cellulose acetate propionate (CAP), Cellulose acetate phthalate (CaPh), Cellulose acetate butyrate (CAB), Cellulose propionate (CP), Cellulose butyrate (CB), Celluose acetate propionate (CAP), 2-Hydroxyethyl cellulose, Hydroxypropyl cellulose, (Hydroxypropyl)methyl cellulose, and Sodium carboxymethyl cellulose), or a combination thereof;
preferably the polymer is selected from Ethyl Cellulose (EC), Cellulose acetate butyrate (CAB), Cellulose propionate (CP), Cellulose butyrate (CB), and Cellulose acetate propionate (CAP), or a combination thereof;
preferably CAB.

5. The composition according to any of claims 1-4, wherein the solvent is selected from glycerol, diglycerol, polyglycerol, propylene glycol, polypropylene glycol, ethylene glycol, diethylene glycol, triethylene glycol, polyethylene glycol, benzyl benzoate, triglycerides, acetone, benzyl alcohol, ethanol, ethyl lactate, propylene carbonate and dimethyl sulfoxide, or a combination thereof;
preferably the solvent is selected from ethanol, propylene carbonate, dimethyl sulphoxide, and benzyl alcohol, or a combination thereof;
preferably dimethyl sulphoxide.

6. The composition according to any of claims 1-5, wherein it further comprises at least one co-solvent being an oil selected from of tripropionin, tributyrin, glycerol trivalerate, glycerol trihexanoate (GTH), glycerol trioctanoate (GTO), glycerol tridecanoate (GTD), glycerol tridodecanoate, ethyl hexanoate, ethyl octanoate, ethyl decanoate, ethyl laurate, ethyl myristate, ethyl palmitate, ethyl stearate, ethyl oleate, corn oil, peanut oil, coconut oil, sesame oil, cinnamon oil, soybean oil, and poppyseed oil, or a combination thereof;
preferably glycerol trihexanoate (GTH), glycerol trioctanoate (GTO), glycerol tridecanoate (GTD), or Ethyl-palmitate, or a combination thereof.

7. The composition according to any of claims 1-6, wherein it further comprises one or more lipids selected from cholesterol, lanosterol, ergosterol, DLPC, DLPE, DLPG, DMPC, DMPE, DMPG, DPPC, DPPE, DPPG, DSPC, DSPE, DSPG, DOPC, DOPE, DOPG, phospholipids (DLPE, DMPE, DPPE, DSPE, or DOPE) PEGylated with either PEG350, PEG500, PEG750, PEG1k, PEG2k, PEG3k or PEG5k; cholesterol PEGylated with either PEG350, PEG500, PEG600, PEG750, PEG1k, PEG2k, PEG3k or PEG5k; 1,2-Dimyristoyl-rac-glycero-3-methoxypolyethylene glycol-2000 (DMG-PEG2k), Distearoyl-rac-glycerol-PEG2K (DSG-PEG2k), DC-chol, DMTAP, DPTAP, DSTAP, DOTAP, DOTMA, DOSPA, DDAB, DMDAP, DPDAP, DSDAP, DODAP, DODMA, DOBAQ, DLin-DMA, DLin-KC2-DMA, DLin-MC3-DMA, C12-200, A6, OF-02, A18-Iso5-2DC18, YSK05, 7C1, G0-C14, L319, OF-Deg-Lin, 306-O12B, 3060110, FTT5, 9A1P9, 98N12-5, 304013, cKK-E12, Spermine-chol, and MVL5;
preferably one or more lipids selected from zwitterionic lipids, cationic lipids, anionic lipids, or pegylated lipids, or a combination thereof.

8. The composition according to claim 4 or 7, wherein the polymers of claim 4 or the lipids of claim 7 are functionalized with groups selected from:
the ligands RGD, ceruloplasmin, prothrombin, alpha-2-HS-glycoprotein, alpha-1-antichymotrypsin,proline-rich peptides, arginine-rich peptides, lysine-rich peptides, Pep-1, L-oligomers, and Calcitonin peptide(s),
cell penetrating peptides (CPP), such as TAT, KALA;
ligands of a receptor, such as cytokines, hormones, or growth factors;
small molecules (e.g. carbohydrates like mannose or galactose or synthetic ligands),
small molecule agonists, inhibitors or antagonists of receptors (e.g. RGD peptidomimetic analogues) or any such molecule. Particularly preferred are cell penetrating peptides (CPPs), which include, without being limited thereto protamine, nucleoline, spermine or spermidine, poly-L-lysine (PLL), basic polypeptides, poly-arginine, chimeric CPPs, such as Transportan, or MPG peptides, HIV-binding peptides, Tat, HIV-1 Tat (HIV), Tat-derived peptides, oligoarginines, members of the penetratin family, e.g. Penetratin, Antennapedia-derived peptides (particularly from Drosophila antennapedia), pAntp, plsl, etc., antimicrobial-derived CPPs e.g. Buforin-2, Bac715-24, SynB, SynB(1), pVEC, hCT-derived peptides, SAP, MAP, PpTG20, proline-rich peptides, Loligomers, arginine-rich peptides, Calcitonin-peptides, FGF, Lactoferrin, poly-L-lysine, poly-arginine, histones, VP22 derived or analog peptides, Pestivirus Erns, HSV, VP22 (Herpes simplex), MAP, KALA or protein transduction domains (PTDs, PpT620, proline-rich peptides, arginine-rich peptides, lysine-rich peptides, Pep-1, L-oligomers, Calcitonin peptide(s).

9. The composition according to any of claims 1-8, wherein it further comprises at least one excipient selected from CT contrast agents, radiographic contrast agents, fluorophores, chromophores, MRI contrast agents, radiotracers, and contrast carrying nanoparticles, or a combination thereof.

10. The composition according to claim 9, wherein the CT (and radiographic) contrast agents are selected from lipiodol, α-, β-Lactose octa para-iodobenzoate (CLA-8), 6,6'-(2,4,6-triiodophenoxy)acetoxy-isobutyric-sucrose (xSAIB), lactose, sucrose octa para-iodobenzoate, and octa meta-iodobenzoate.

11. The composition according to any of claims 1-10, wherein it further comprises at least one therapeutic agent selected from anticancer agents, immunotherapeutics, chemotherapeutics, antimicrobials, antibiotics, antivirals, antifungals, antiprotozoals, anthelminthics, anti-inflammatory agents, anticoagulant, antidepressant, antiepileptic, antipsychotic, sedatives, antidiabetic, cardiovascular, analgesic agents, therapeutic peptides, diagnostic peptides, agonistic receptor ligands, antagonistic receptor ligands, peptides with regulatory or enzymatic activity, hormones, peptides with special targeting, vaccines, antigens, steroids, immunotherapeutic agonists, immunotherapeutic antagonists, growth factors, cytokines, chemokines, hormones, glycoprotein hormones, antibodies, affibodies, and nanobodies, or a combination thereof.

12. The composition according to any of claims 1-11, wherein the composition comprises the carbohydrate ester in a range of 50-90 w/w%, preferably 60-80w/w% preferably 70 w/w%, of the ECell; or
wherein the composition comprises the polymer in a range of 1-20 w/w%, preferably 2.5-15 w/w%, preferably 5 w/w%, of the ECell; or
wherein the composition comprises the co-solvent in a range of 1-20 w/w%, preferably 2.5-15 w/w%, preferably 5 w/w%, of the ECell; or
wherein the composition comprises the lipid in a range of 0.01-10 w/w%, preferably 0.1-5 w/w%, preferably 1 w/w%, of the ECell.

13. The composition according to any of claims 1-12, for use in therapy, preferably for use in therapy selected from cancer therapy, such as immunotherapy, chemotherapy, cryotherapy, ablation therapy, adoptive cell transfer; adjuvant therapy for radiation therapy or cancer surgery; pre-, intra-, and post-surgical therapy for improved healing, bone loss, peritendinous adhesions, or bone non-union; infections associated with, soft tissue, bones, abscesses, phlegmons, fistulas; infections associated with, or in, catheters, drains, stents, tissue grafts, transplantations, transplanted organs, artificial organs, hearing aids, implanted devices, bone implants, metal or ceramic internal or external fixation devices, artificial organs, protheses, reconstructive surgery, plastic surgery, tissue loss, tissue defects, or pain relief; local or systemic analgesia or anaesthesia; and local or systemic immune suppression; or
the composition according to any of claims 1-12 for use in the treatment of diseases selected from cancers, such as myelodysplastic syndrome; autoimmune disorders; rheumatoid disease; degenerative disorders; anaemia; endocrine disorders, such as endocrine gland hyposecretion, endocrine gland hypersecretion, tumours (benign or malignant) of endocrine glands, hormone replacements, hormone supplementation, hormone imbalances, hormone inactivation, adoptive cell therapies; and stem cells, such as chimeric antigen receptor cell products, or myeloid and lymphoid cell products.

14. The composition according to any of claims 1-12 for use as:
i) fiducial markers with positive MRI contrast for guiding external beam radiotherapy (EBRT), image guided interventions, such as surgery or biopsy procedures;
ii) surgical void filler in debrided bone infections, bone trauma, tissue defects after surgical interventions, trauma, plastic surgery or reconstructive surgery;
iii) for expanding void filler or tissue spacer for minimizing normal tissue damage associated with radiation therapy, thermal ablation, or cryotherapy;
iv) surgical spacing for sparing fragile structures in radiation therapy;
v) cell adaptive regenerative tissue scaffolds;
vi) scaffolds that supports cell engraftment and polarizations for cell therapies such as stem cell therapy;
vii) expanding drug eluding depot with tailored drug release profiles; or
vii) prostate rectum spacing.

15. Method comprising using a composition according to any of claims 1-12, in imaging, wherein the method comprises the steps of:
- injecting the composition according to any of claims 1-12 into water, aqueous media or tissue, wherein the composition is a ECell being a hydrophobic gel-depot is expanding by uptake of the water by means of said at least one solvent being mixable with water and having the ability to self-expand by formation of water pores, channels, and/or cavities; and
- locating the expanded ECell by means of imaging;
or
using a composition according to any of claims 1-12, in controlled release of at least one therapeutic agent, wherein the method comprises the steps of:
- injecting the composition according to any of claims 1-12 into water, aqueous media or tissue, wherein the composition is a ECell being a hydrophobic gel-depot is expanding by uptake of the water by means of said at least one solvent being mixable with water and having the ability to self-expand by formation of water pores, channels, and/or cavities, and
- discharging of the at least one therapeutic agent from the ECell by controlled release.
